# EUROPEAN PATENT APPLICATION

(11) **EP 2 039 695 A1**
(43) Date of publication of application: **25.03.2009**
(21) Application number: 07790546.1
(22) Date of filing: 10.07.2007
(51) Int. Cl.: C07D 487/04, A61K 31/4985, A61P 3/04, A61P 13/02, A61P 43/00

(54) **BICYCLIC HETEROCYCLIC COMPOUND AND USE THEREOF**

(30) Priority: 11.07.2006 JP 2006190924
(71) Applicant: Takeda Pharmaceutical Company Limited, Osaka-shi, Osaka 541-0045 (JP)
(72) Inventor: MATSUMOTO, Takahiro, Osaka-shi Osaka 532-8686 (JP); KAMO, Izumi, Osaka-shi Osaka 532-8686 (JP)
(74) Representative: Weber, Thomas
(86) International application number: PCT/JP2007/063728
(87) International publication number: WO 2008/007664

(57) **Abstract**

The present invention provides a bicyclic heterocyclic compound having a serotonin 5-HT_{2c} receptor activation action and the like.

A compound represented by the formula (I): 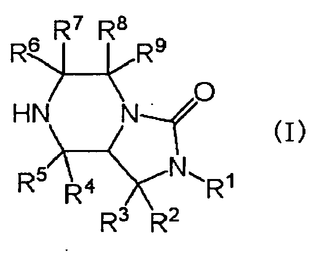 wherein each symbol is as defined in the specification, or a salt thereof.

## Description

### Technical Field

The present invention relates to a bicyclic heterocyclic compound having excellent serotonin 5-HT_{2C} receptor activating action, and useful as a drug for the prophylaxis or treatment of stress urinary incontinence, obesity, pelvic organ prolapse and the like, and the like.

### Background Art

The serotonin 5-HT_{2C} receptor, one of the receptors of the biological transmitter serotonin, is distributed mainly in the central nervous system and controls many physiological functions in vivo. A representative example is the control of appetite; it has been demonstrated in a study with rodents that when the central serotonin 5-HT_{2C} receptor is stimulated, eating behavior lessons and body weight is lost. In humans as well, it has been reported that when a serotonin 5-HT_{2C} receptor activator is administered, appetite is suppressed and body weight is lost (see non-patent document 1). In addition, stimulation of the central serotonin 5-HT_{2C} receptor has been shown to suppress depression-related behavior in a rat study using a serotonin 5-HT_{2C} receptor activator (see non-patent document 2), and has also been reported to be effective on many central nervous diseases such as anxiety (see non-patent document 3). The serotonin 5-HT_{2C} receptor is also highly expressed in the parasympathetic nucleus and motorial nerve cell bodies in the sacral spinal cord, and is thought to control peripheral nervous functions (see non-patent document 4). It has been reported that when a serotonin 5-HT_{2C} receptor activator is administered to rats, penile erection is induced (see non-patent document 5), and urethral resistance is increased (see patent document 1); all these actions are attributed to stimulation of the serotonin 5-HT_{2C} receptor in the sacral spinal cord. For serotonin 5-HT_{2C} receptor activators, many clinical applications are likely, with particular expectations for anti-obesity drugs, antidepressants, anti-anxiety drugs, therapeutic drugs for male erectile dysfunction, and therapeutic drugs for stress urinary incontinence and the like.

Bicyclic and tricyclic heterocyclic compounds are reported in non-patent documents 6 to 13 and patent documents 2 to 17.
However, no reference is made to a serotonin 5-HT_{2C} receptor activating action of these compounds.
non-patent document 1: Expert Opinion on Investigational Drugs, 2006, vol. 15, p. 257-266
non-patent document 2: J. Pharmacol. Exp. Ther., 1998, vol. 286, p. 913-924
non-patent document 3: Pharmacology Biochemistry Behavior, 2002, vol. 71, p. 533-554
non-patent document 4: Neuroscience, 1999, vol. 92, p. 1523-1537
non-patent document 5: Eur. J. Pharmacol., 2004, vol. 483, p. 37-43
non-patent document 6: Journal of Medicinal Chemistry, 2005, vol. 48, p. 5104-5107
non-patent document 7: Bioorganic & Medicinal Chemistry Letters, 2004, vol. 14, p. 6107-6111
non-patent document 8: European Journal of Medicinal Chemistry, 2001, vol. 36, p. 203-209
non-patent document 9: Biochemistry, 2000, vol. 39, p. 6325-6335
non-patent document 10: Journal of Pharmacology and Experimental Therapeutics, 1998, vol. 287, p. 315-321
non-patent document 11: Journal of Medicinal Chemistry, 1982, vol. 25, p. 161-166
non-patent document 12: Biochemical Pharmacology, 1979, vol. 28, p. 2633-2637
non-patent document 13: Acta Univ. Carolinae, Med., Suppl. 1965, vol. 21, p. 155-163
patent document 1: WO2004/096196
patent document 2: JP-A-2003-12672
patent document 3: US-A-2002/173445
patent document 4: US Patent No. 6657063
patent document 5: JP-A-2005-524610
patent document 6: WO2003/011824
patent document 7: JP-A-2003-002834
patent document 8: WO2002/044181
patent document 9: JP-A-2004-501917
patent document 10: JP-A-2004-515462
patent document 11: JP-A-2004-509072
patent document 12: JP-A-2003-512468
patent document 13: WO2000/035885
patent document 14: JP-A-2000-159770
patent document 15: JP-A-2000-072771
patent document 16: JP-A-2000-516639
patent document 17: JP-A-63-166880

### Disclosure of the Invention

### Problems to be Solved by the Invention

There is a demand for the development of a compound having a serotonin 5-HT_{2C} receptor activating action, useful as an agent for the prophylaxis or treatment of stress urinary incontinence, obesity, pelvic organ prolapse and the like, and superior in the receptor selectivity, pharmacological efficacy, duration of action, specificity, low toxicity and the like.

The present invention aims to provide an agent for the prophylaxis or treatment of disease such as stress urinary incontinence, obesity, pelvic organ prolapse and the like, which comprises a bicyclic heterocyclic compound having a serotonin 5-HT_{2C} receptor activating action and the like, and having a chemical structure different from those of known compounds including the above-mentioned compounds.

### Means of Solving the Problems

The present inventors have conducted intensive studies and succeeded for the first time in the creation of a serotonin 5-HT_{2C} receptor activator comprising a compound represented by the formula:

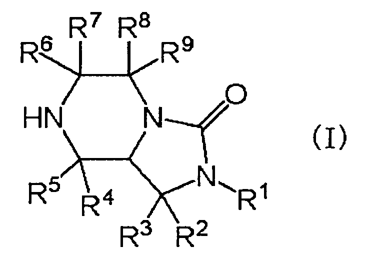

wherein
R¹ is a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s) or a heterocyclic group optionally having substituent(s);
R², R³, R⁴, R⁵, R⁶, R⁷, R⁸ and R⁹ are the same or different and each is a hydrogen atom, a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s), a hydroxyl group optionally having a substituent, mercapto optionally having a substituent, amino optionally having substituent(s) or a heterocyclic group optionally having substituent(s), or each of R⁴ and R⁵, R⁶ and
R⁷, and R⁸ and R⁹ optionally form, together with the adjacent carbon atom, carbonyl, thiocarbonyl or imino optionally having a substituent,
or a salt thereof (hereinafter to be sometimes abbreviated as compound (I)) or a prodrug thereof, and further found that compound (I) unexpectedly has superior properties as a serotonin 5-HT_{2C} receptor activator and is sufficiently satisfactory as a pharmaceutical agent. Based on these findings, they have completed the present invention.

Accordingly, the present invention relates to
[1] compound (I),
[2] compound (I) wherein R¹ is aryl optionally having substituent(s), aralkyl optionally having substituent(s) or a heterocyclic group optionally having substituent(s),
[3] compound (I) wherein R¹ is
   (1) C₆₋₁₄ aryl optionally having 1 to 3 substituents selected from
      (a) a halogen atom,
      (b) cyano,
      (c) a hydroxyl group,
      (d) amino,
      (e) C₁₋₆ alkyl optionally having 1 to 3 halogen atoms,
      (f) C₆₋₁₄ aryl,
      (g) C₇₋₁₆ aralkyl,
      (h) di-C₁₋₆ alkylamino,
      (i) C₁₋₆ alkoxy,
      (j) C₆₋₁₄ aryloxy,
      (k) C₁₋₆ alkylthio optionally having 1 to 3 halogen atoms,
      (l) C₁₋₆ alkyl-carbonyl,
      (m) C₁₋₆ alkoxy-carbonyl,
      (n) C₁₋₆ alkylsulfonyl, and
      (o) a 5- to 8-membered aromatic heterocyclic group containing, besides carbon atom, 1 to 4 hetero atoms selected from nitrogen atom, sulfur atom and oxygen atom, and optionally condensed with C₃₋₈ cycloalkane,
   (2) C₇₋₁₆ aralkyl,
   (3) a 5- to 8-membered non-aromatic heterocyclic group containing, besides carbon atom, 1 to 4 hetero atoms selected from nitrogen atom, sulfur atom and oxygen atom, and optionally condensed with C₆₋₁₄ arene, or
   (4) a 5- to 8-membered aromatic heterocyclic group containing, besides carbon atom, 1 to 4 hetero atoms selected from nitrogen atom, sulfur atom and oxygen atom, and optionally having 1 to 3 C₁₋₆ alkyl optionally having 1 to 3 halogen atoms, and optionally condensed with C₆₋₁₄ arene,
[4] compound (I) wherein R², R³, R⁴, R⁵, R⁶, R⁷, R⁸ and R⁹ are each a hydrogen atom,
[5] 2-(2-thienyl)hexahydroimidazo[1,5-a]pyrazin-3(2H)-one, 2-(3-thienyl)hexahydroimidazo[1,5-a]pyrazin-3(2H)-one, 2-[5-methyl-2-(trifluoromethyl)-3-furyl]hexahydroimidazo[1,5-a]pyrazin-3(2H)-one, or 2-(3-ethylphenyl)hexahydroimidazo[1,5-a]pyrazin-3(2H)-one, or a salt thereof,
[6] a prodrug of compound (I),
[7] a pharmaceutical agent comprising compound (I) or a prodrug thereof,
[8] the pharmaceutical agent of [7], which is a serotonin 5-HT_{2c} receptor activator,
[9] the pharmaceutical agent of [7], which is an agent for the prophylaxis or treatment of stress urinary incontinence, obesity and/or pelvic organ prolapse,
[10] a method for the prophylaxis or treatment of stress urinary incontinence, obesity and/or pelvic organ prolapse in a mammal, comprising administering an effective amount of compound (I) or a prodrug thereof to the mammal,
[11] use of compound (I) or a prodrug thereof for the production of an agent for the prophylaxis or treatment of stress urinary incontinence, obesity and/or pelvic organ prolapse,
   and the like.
The present invention is explained in detail in the following.

### Effect of the Invention

Since compound (I) and a prodrug thereof of the present invention have a superior serotonin 5-HT_{2C} receptor activating action, they are useful as safe drugs for the prophylaxis or treatment of all serotonin 5-HT_{2C}-related diseases, for example, stress urinary incontinence, obesity, pelvic organ prolapse and the like.

### Detailed Description of the Invention

The present invention is explained in detail in the following.
First, the definition of each symbol in compound (I) is explained in the following.

In the present specification, examples of the "hydrocarbon group optionally having substituent(s)" include "alkyl optionally having substituent(s)", "alkenyl optionally having substituent(s)", "alkynyl optionally having substituent(s)", "aralkyl optionally having substituent(s)", "aryl optionally having substituent(s)", "cycloalkyl optionally having substituent(s)" and the like.

In the present specification, examples of the "alkyl optionally having substituent(s)" include C₁₋₆ alkyl (e.g., methyl, ethyl, propyl, isopropyl, butyl, isobutyl, sec-butyl, tert-butyl, pentyl, hexyl etc.) optionally having substituent(s) selected from
(i) a halogen atom (e.g., fluorine atom, chlorine atom, bromine atom, iodine atom),
(ii) cyano,
(iii) a hydroxyl group,
(iv) nitro,
(v) formyl,
(vi) amino,
(vii) mono- or di-C₁₋₆ alkylamino (e.g., methylamino, ethylamino, propylamino, dimethylamino, diethylamino, dipropylamino etc.),
(viii) C₁₋₆ alkyl-carbonylamino (e.g., acetylamino, ethylcarbonylamino etc.),
(ix) C₁₋₆ alkoxy-carbonylamino (e.g., methoxycarbonylamino, ethoxycarbonylamino, propoxycarbonylamino etc.),
(x) C₃₋₈ cycloalkyl (e.g., cyclopropyl, cyclobutyl, cyclopentyl, cyclohexyl etc.),
(xi) C₆₋₁₄ aryl (e.g., phenyl, 1-naphthyl, 2-naphthyl etc.) optionally substituted by substituent(s) selected from a halogen atom (e.g., fluorine atom, chlorine atom, bromine atom, iodine atom) and C₁₋₆ alkoxy (e.g., methoxy, ethoxy, propoxy etc.),
(xii) C₁₋₆ alkoxy (e.g., methoxy, ethoxy, propoxy, isopropoxy, butoxy, isobutoxy, sec-butoxy, tert-butoxy etc.) optionally substituted by halogen atom(s) (e.g., fluorine atom, chlorine atom, bromine atom, iodine atom),
(xiii) C₇₋₁₆ aralkyloxy (e.g., benzyloxy etc.),
(xiv) C₆₋₁₄ aryloxy (e.g., phenoxy etc.),
(xv) carboxyl,
(xvi) C₁₋₆ alkoxy-carbonyl (e.g., methoxycarbonyl, ethoxycarbonyl, propoxycarbonyl, isopropoxycarbonyl, tert-butoxycarbonyl etc.),
(xvii) C₇₋₁₆ aralkyloxy-carbonyl (e.g., benzyloxycarbonyl etc.),
(xviii) C₆₋₁₄ aryloxy-carbonyl (e.g., phenoxycarbonyl etc.),
(xix) C₁₋₆ alkyl-carbonyl (e.g., acetyl, ethylcarbonyl, propylcarbonyl, isopropylcarbonyl, 2,2-dimethylpropylcarbonyl etc.),
(xx) C₃₋₈ cycloalkyl-carbonyl (e.g., cyclopropylcarbonyl, cyclobutylcarbonyl, cyclopentylcarbonyl, cyclohexylcarbonyl etc.),
(xxi) C₇₋₁₆ aralkyl-carbonyl (e.g., benzylcarbonyl etc.),
(xxii) carbamoyl,
(xxiii) thiocarbamoyl,
(xxiv) mono- or di-C₁₋₆ alkyl-carbamoyl (e.g., methylcarbamoyl, ethylcarbamoyl, propylcarbamoyl, isopropylcarbamoyl, dimethylcarbamoyl, diethylcarbamoyl, dipropylcarbamoyl etc.),
(xxv) mono- or di-C₇₋₁₆ aralkyl-carbamoyl (e.g., benzylcarbamoyl, dibenzylcarbamoyl etc.),
(xxvi) mercapto,
(xxvii) C₁₋₆ alkylthio (e.g., methylthio, ethylthio, propylthio etc.) optionally substituted by halogen atom(s) (e.g., fluorine atom, chlorine atom, bromine atom, iodine atom),
(xxviii) C₇₋₁₆ aralkylthio (e.g., benzylthio etc.),
(xxix) C₁₋₆ alkylsulfonyl (e.g., methylsulfonyl, ethylsulfonyl, propylsulfonyl, isopropylsulfonyl etc.),
(xxx) C₃₋₈ cycloalkylsulfonyl (e.g., cyclopropylsulfonyl, cyclobutylsulfonyl, cyclopentylsulfonyl etc.),
(xxxi) C₆₋₁₄ arylsulfonyl (e.g., phenylsulfonyl, 1-naphthylsulfonyl, 2-naphthylsulfonyl etc.),
(xxxii) C₇₋₁₆ aralkylsulfonyl (e.g., benzylsulfonyl etc.),
(xxxiii) a 5- to 8-membered non-aromatic heterocyclic group containing, besides carbon atom, 1 to 4 hetero atoms selected from nitrogen atom, sulfur atom and oxygen atom (e.g., pyrrolidinyl, tetrahydrofuryl, tetrahydrothienyl, piperidyl, tetrahydropyranyl, morpholinyl, thiomorpholinyl, piperazinyl etc.),
(xxxiv) a 5- to 8-membered aromatic heterocyclic group containing, besides carbon atom, 1 to 4 hetero atoms selected from nitrogen atom, sulfur atom and oxygen atom (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.),
(xxxv) 5- to 8-membered non-aromatic heterocyclyl-carbonyl containing, besides carbon atom, 1 to 4 hetero atoms selected from nitrogen atom, sulfur atom and oxygen atom (e.g., pyrrolidinylcarbonyl, tetrahydrofurylcarbonyl, tetrahydrothienylcarbonyl, piperidylcarbonyl, tetrahydropyranylcarbonyl, morpholinylcarbonyl, thiomorpholinylcarbonyl, piperazinylcarbonyl etc.),
(xxxvi) 5- to 8-membered aromatic heterocyclyl-carbonyl containing, besides carbon atom, 1 to 4 hetero atoms selected from nitrogen atom, sulfur atom and oxygen atom (e.g., furylcarbonyl, thienylcarbonyl, pyrrolylcarbonyl, oxazolylcarbonyl, isoxazolylcarbonyl, thiazolylcarbonyl, isothiazolylcarbonyl, imidazolylcarbonyl, pyrazolylcarbonyl, 1,2,3-oxadiazolylcarbonyl, 1,2,4-oxadiazolylcarbonyl, 1,3,4-oxadiazolylcarbonyl, furazanylcarbonyl, 1,2,3-thiadiazolylcarbonyl, 1,2,4-thiadiazolylcarbonyl, 1,3,4-thiadiazolylcarbonyl, 1,2,3-triazolylcarbonyl, 1,2,4-triazolylcarbonyl, tetrazolylcarbonyl, pyridylcarbonyl, pyridazinylcarbonyl, pyrimidinylcarbonyl, pyrazinylcarbonyl, triazinylcarbonyl etc.),
(xxxvii) ureido,
(xxxviii) C₁₋₆ alkyl-ureido (e.g., methylureido, ethylureido, propylureido etc.),
(xxxix) C₆₋₁₄ aryl-ureido (e.g., phenylureido, 1-naphthylureido, 2-naphthylureido etc.),
(xxxx) C₁₋₄ alkylenedioxy (e.g., methylenedioxy, ethylenedioxy, propylenedioxy etc.)
and the like. The number of the substituents is 1 to 4, preferably 1 to 3.

In the present specification, examples of the "alkenyl optionally having substituent(s)" include C₂₋₆ alkenyl (e.g., vinyl, 1-propenyl, allyl, isopropenyl, butenyl, isobutenyl etc.) optionally having 1 to 4, preferably 1 to 3, substituents, which the above-mentioned "alkyl optionally having substituent(s)" optionally has.
In the present specification, examples of the "alkynyl optionally having substituent(s)" include C₂₋₆ alkynyl (e.g., ethynyl, propargyl, butynyl, 1-hexynyl etc.) optionally having 1 to 4, preferably 1 to 3, substituents, which the above-mentioned "alkyl optionally having substituent(s)" optionally has.

In the present specification, examples of the "aralkyl optionally having substituent(s)" include C₇₋₁₆ aralkyl (e.g., benzyl, 2-phenylethyl, 1-phenylethyl, 3-phenylpropyl etc.) optionally having 1 to 4, preferably 1 to 3, substituents selected from
(i) a substituent which the above-mentioned "alkyl optionally having substituent(s)" optionally has,
(ii) C₁₋₆ alkyl (e.g., methyl, ethyl, propyl, isopropyl, butyl, isobutyl, sec-butyl, tert-butyl, pentyl, hexyl etc.) optionally substituted by substituent(s) selected from a halogen atom (e.g., fluorine atom, chlorine atom, bromine atom, iodine atom), C₁₋₆ alkoxy (e.g., methoxy, ethoxy, propoxy etc.), C₆₋₁₄ arylsulfonyl and a heterocyclic group (e.g., morpholinyl, pyridyl, imidazopyridyl, benzimidazolyl etc.),
(iii) C₇₋₁₆ aralkyl (e.g., benzyl, 2-phenylethyl, 1-phenylethyl, 3-phenylpropyl, 4-phenylbutyl etc.),
   and the like.
In the present specification, examples of the "aryl optionally having substituent(s)" include C₆₋₁₄ aryl (e.g., phenyl, naphthyl etc.) optionally having 1 to 4, preferably 1 to 3, substituents, which the above-mentioned "aralkyl optionally having substituent(s)" optionally has.
In the present specification, "C₆₋₁₄ aryl optionally condensed with C₃₋₈ cycloalkane" is C₃₋₈ cycloalkane (e.g., cyclopropane, cyclobutane, cyclopentane, cyclohexane etc.) optionally condensed with C₆₋₁₄ aryl (e.g., phenyl, naphthyl etc.), and examples thereof include indanyl, tetrahydronaphthyl and the like.
In the present specification, examples of the "cycloalkyl optionally having substituent(s)" include C₃₋₈ cycloalkyl (e.g., cyclopropyl, cyclobutyl, cyclopentyl, cyclohexyl etc.) optionally having 1 to 4, preferably 1 to 3, substituents, which the above-mentioned "aralkyl optionally having substituent(s)" optionally has.

In the present specification, examples of the "acyl" include "alkylcarbonyl optionally having substituent(s)", "alkenylcarbonyl optionally having substituent(s)", "alkynylcarbonyl optionally having substituent(s)", "aralkylcarbonyl optionally having substituent(s)", "arylcarbonyl optionally having substituent(s)", "cycloalkylcarbonyl optionally having substituent(s)" and the like.
In the present specification, examples of the "alkylcarbonyl optionally having substituent(s)" include C₁₋₆ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl, pentylcarbonyl, hexylcarbonyl etc.) optionally having 1 to 4, preferably 1 to 3, substituents, which the above-mentioned "alkyl optionally having substituent(s)" optionally has.
In the present specification, examples of the "alkenylcarbonyl optionally having substituent(s)" include C₂₋₆ alkenyl-carbonyl (e.g., vinylcarbonyl, 1-propenylcarbonyl, allylcarbonyl, isopropenylcarbonyl, butenylcarbonyl, isobutenylcarbonyl etc.) optionally having 1 to 4, preferably 1 to 3, substituents, which the above-mentioned "alkyl optionally having substituent(s)" optionally has.
In the present specification, examples of the "alkynylcarbonyl optionally having substituent(s)" include C₂₋₆ alkynyl-carbonyl (e.g., ethynylcarbonyl, propargylcarbonyl, butynylcarbonyl, 1-hexynylcarbonyl etc.) optionally having 1 to 4, preferably 1 to 3, substituents, which the above-mentioned "alkyl optionally having substituent(s)" optionally has.

In the present specification, examples of the "aralkylcarbonyl optionally having substituent(s)" include C₇₋₁₆ aralkyl-carbonyl (e.g., benzylcarbonyl, 2-phenylethylcarbonyl, 1-phenylethylcarbonyl, 3-phenylpropylcarbonyl etc.) optionally having 1 to 4, preferably 1 to 3, substituents, which the above-mentioned "aralkyl optionally having substituent(s)" optionally has.
In the present specification, examples of the "arylcarbonyl optionally having substituent(s)" include C₆₋₁₄ aryl-carbonyl (e.g., benzoyl, naphthylcarbonyl etc.) optionally having 1 to 4, preferably 1 to 3, substituents, which the above-mentioned "aralkyl optionally having substituent(s)" optionally has.
In the present specification, examples of the "cycloalkylcarbonyl optionally having substituent(s)" include C₃₋₈ cycloalkyl-carbonyl (e.g., cyclopropylcarbonyl, cyclobutylcarbonyl, cyclopentylcarbonyl, cyclohexylcarbonyl etc.) optionally having 1 to 4, preferably 1 to 3, substituents, which the above-mentioned "aralkyl optionally having substituent(s)" optionally has.

In the present specification, examples of the "heterocyclic group optionally having substituent(s)" include
(1) a 5- to 8-membered non-aromatic heterocyclic group (e.g., pyrrolidinyl, tetrahydrofuryl, tetrahydrothienyl, piperidyl, tetrahydropyranyl, morpholinyl, thiomorpholinyl, piperazinyl, azepanyl, 1,4-diazepanyl etc.) containing, besides carbon atom, 1 to 4 hetero atoms selected from nitrogen atom, sulfur atom and oxygen atom, and optionally having 1 to 3 substituents, which the above-mentioned "aralkyl optionally having substituent(s)" optionally has, and optionally condensed with C₆₋₁₄ arene (e.g., benzene), and
(2) a 5- to 8-membered aromatic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) containing, besides carbon atom, 1 to 4 hetero atoms selected from nitrogen atom, sulfur atom and oxygen atom, and optionally having 1 to 3 substituents, which the above-mentioned "aralkyl optionally having substituent(s)" optionally has, and optionally condensed with C₆₋₁₄ arene (e.g., benzene).

In the present specification, examples of the "hydroxyl group optionally having a substituent" include a hydroxyl group and a hydroxyl group having a substituent.
In the present specification, examples of the "hydroxyl group having a substituent" include a hydroxyl group having the above-mentioned "hydrocarbon group optionally having substituent(s)", "acyl" or "heterocyclic group optionally having substituent(s)".

In the present specification, examples of the "mercapto optionally having a substituent" include mercapto and mercapto having a substituent.
In the present specification, examples of the "mercapto having a substituent" include mercapto having the above-mentioned "hydrocarbon group optionally having substituent(s)", "acyl" or "heterocyclic group optionally having substituent(s)".

In the present specification, examples of the "amino optionally having substituent(s)" include amino and amino having substituent(s).
In the present specification, examples of the "amino having substituent(s)" include amino having 1 or 2 substituents selected from the above-mentioned "hydrocarbon group optionally having substituent(s)", "acyl" and "heterocyclic group optionally having substituent(s)".

In the present specification, example of the "imino optionally having a substituent" include imino and imino having a substituent.
In the present specification, example of the "imino having a substituent" include imino having the above-mentioned "hydrocarbon group optionally having substituent(s)" or "heterocyclic group optionally having substituent(s)".

In compound (I), R¹ is a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s) or a heterocyclic group optionally having substituent(s).
R¹ is preferably a hydrocarbon group optionally having substituent(s) (particularly aryl optionally having substituent(s), aralkyl optionally having substituent(s)) or a heterocyclic group optionally having substituent(s).
R¹ is particularly preferably
   (1) C₆₋₁₄ aryl (e.g., phenyl) optionally having 1 to 3 substituents selected from
      (a) a halogen atom (e.g., fluorine atom, chlorine atom, bromine atom),
      (b) cyano,
      (c) a hydroxyl group,
      (d) amino,
      (e) C₁₋₆ alkyl (e.g., methyl, ethyl) optionally having 1 to 3 halogen atoms (e.g., fluorine atom) (e.g., methyl, trifluoromethyl, ethyl),
      (f) C₆₋₁₄ aryl (e.g., phenyl),
      (g) C₇₋₁₆ aralkyl (e.g., benzyl),
      (h) di-C₁₋₆ alkylamino (e.g., diethylamino),
      (i) C₁₋₆ alkoxy (e.g., methoxy),
      (j) Cue-14 aryloxy (e.g., phenoxy),
      (k) C₁₋₆ alkylthio (e.g., methylthio) optionally having 1 to 3 halogen atoms (e.g., fluorine atom) (e.g., methylthio, trifluoromethylthio),
      (l) C₁₋₆ alkyl-carbonyl (e.g., acetyl),
      (m) C₁₋₆ alkoxy-carbonyl (e.g., ethoxycarbonyl),
      (n) C₁₋₆ alkylsulfonyl (e.g., methylsulfonyl), and
      (o) a 5- to 8-membered aromatic heterocyclic group (e.g., pyrrolyl) containing, besides carbon atom, 1 to 4 hetero atoms selected from nitrogen atom, sulfur atom and oxygen atom,
      and optionally condensed with C₃₋₈ cycloalkane (e.g., cyclopentane),
      (e.g., phenyl, indanyl),
   (2) C₇₋₁₆ aralkyl (e.g., benzyl),
   (3) a 5- to 8-membered non-aromatic heterocyclic group (e.g., dihydrofuryl) containing, besides carbon atom, 1 to 4 hetero atoms selected from nitrogen atom, sulfur atom and oxygen atom, and optionally condensed with C₆₋₁₄ arene (e.g., benzene), (e.g., dihydrobenzofuryl), or
   (4) a 5- to 8-membered aromatic heterocyclic group (e.g., pyridyl, furyl, thienyl, isoxazolyl) containing, besides carbon atom, 1 to 4 hetero atoms selected from nitrogen atom, sulfur atom and oxygen atom, and optionally having 1 to 3 C₁₋₆ alkyl (e.g., methyl) optionally having 1 to 3 halogen atoms (e.g., fluorine atom) (e.g., methyl, trifluoromethyl), and optionally condensed with C₁₅₋₁₄ arene (e.g., benzene), (e.g., pyridyl, furyl, thienyl, isoxazolyl, benzothienyl).

In compound (I), R², R³, R⁴, R⁵, R⁶, R⁷, R⁸ and R⁹ are the same or different and each is a hydrogen atom, a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s), a hydroxyl group optionally having a substituent, mercapto optionally having a substituent, amino optionally having substituent(s) or a heterocyclic group optionally having substituent(s), or each of R⁹ and R⁵, R⁶ and R⁷, and R⁸ and R⁹ optionally form, together with the adjacent carbon atom, carbonyl, thiocarbonyl or imino optionally having a substituent.
R² is preferably a hydrogen atom.
R³ is preferably a hydrogen atom.
R⁴ is preferably a hydrogen atom.
R⁵ is preferably a hydrogen atom.
R⁶ is preferably a hydrogen atom.
R⁷ is preferably a hydrogen atom.
R⁸ is preferably a hydrogen atom.
R⁹ is preferably a hydrogen atom.

Compound (I) is particularly preferably the following compound.

### [Compound A]

### Compound (I) wherein

R¹ is
(1) C₆₋₁₄ aryl (e.g., phenyl) optionally having 1 to 3 substituents selected from
   (a) a halogen atom (e.g., fluorine atom, chlorine atom, bromine atom),
   (b) cyano,
   (c) a hydroxyl group,
   (d) amino,
   (e) C₁₋₆ alkyl (e.g., methyl, ethyl) optionally having 1 to 3 halogen atoms (e.g., fluorine atom) (e.g., methyl, trifluoromethyl, ethyl),
   (f) C₆₋₁₄ aryl (e.g., phenyl),
   (g) C₇₋₁₆ aralkyl (e.g., benzyl),
   (h) di-C₁₋₆ alkylamino (e.g., diethylamino),
   (i) C₁₋₆ alkoxy (e.g., methoxy),
   (j) C₆₋₁₄ aryloxy (e.g., phenoxy),
   (k) C₁₋₆ alkylthio (e.g., methylthio) optionally having 1 to 3 halogen atoms (e.g., fluorine atom) (e.g., methylthio, trifluoromethylthio),
   (l) C₁₋₆ alkyl-carbonyl (e.g., acetyl),
   (m) C₁₋₆ alkoxy-carbonyl (e.g., ethoxycarbonyl),
   (n) C₁₋₆ alkylsulfonyl (e.g., methylsulfonyl), and
   (o) a 5- to 8-membered aromatic heterocyclic group (e.g., pyrrolyl) containing, besides carbon atom, 1 to 4 hetero atoms selected from nitrogen atom, sulfur atom and oxygen atom,
   and optionally condensed with C₃₋₈ cycloalkane (e.g., cyclopentane),
   (e.g., phenyl, indanyl),
(2) C₇₋₁₆ aralkyl (e.g., benzyl),
(3) a 5- to 8-membered non-aromatic heterocyclic group (e.g., dihydrofuryl) containing, besides carbon atom, 1 to 4 hetero atoms selected from nitrogen atom, sulfur atom and oxygen atom, and optionally condensed with C₆₋₁₄ arene (e.g., benzene), (e.g., dihydrobenzofuryl), or
(4) a 5- to 8-membered aromatic heterocyclic group (e.g., pyridyl, furyl, thienyl, isoxazolyl) containing, besides carbon atom, 1 to 4 hetero atoms selected from nitrogen atom, sulfur atom and oxygen atom, and optionally having 1 to 3 C₁₋₆ alkyl (e.g., methyl) optionally having 1 to 3 halogen atoms (e.g., fluorine atom) (e.g., methyl, trifluoromethyl), and optionally condensed with C₆₋₁₄ arene (e.g., benzene), (e.g., pyridyl, furyl, thienyl, isoxazolyl, benzothienyl); and R², R³, R⁴, R⁵, R⁶, R⁷, R⁸ and R⁹ are each a hydrogen atom.

### [Compound B]

2-(3-methylphenyl)hexahydroimidazo[1,5-a]pyrazin-3(2H)-one or a salt thereof,
2-(3-ethylphenyl)hexahydroimidazo[1,5-a]pyrazin-3(2H)-one or a salt thereof,
2-(2-thienyl)hexahydroimidazo[1,5-a]pyrazin-3(2H)-one or a salt thereof,
2-(3-thienyl)hexahydroimidazo[1,5-a]pyrazin-3(2H)-one or a salt thereof, or
2-[5-methyl-2-(trifluoromethyl)-3-furyl]hexahydroimidazo[1,5-a]pyrazin-3(2H)-one or a salt thereof, (specifically,
2-(3-methylphenyl)hexahydroimidazo[1,5-a]pyrazin-3(2H)-one hydrochloride,
2-(3-ethylphenyl)hexahydroimidazo[1,5-a]pyrazin-3(2H)-one hydrochloride,
2-(2-thienyl)hexahydroimidazo[1,5-a]pyrazin-3(2H)-one trifluoroacetate,
2-(3-thienyl)hexahydroimidazo[1,5-a]pyrazin-3(2H)-one hydrochloride, or
2-[5-methyl-2-(trifluoromethyl)-3-furyl]hexahydroimidazo[1,5-a]pyrazin-3(2H)-one hydrochloride).

### [Compound C]

2-(2-thienyl)hexahydroimidazo[1,5-a]pyrazin-3(2H)-one or a salt thereof,
2-(3-thienyl)hexahydroimidazo[1,5-a]pyrazin-3(2H)-one or a salt thereof,
2-[5-methyl-2-(trifluoromethyl)-3-furyl]hexahydroimidazo[1,5-a]pyrazin-3(2H)-one or a salt thereof, or
2-(3-ethylphenyl)hexahydroimidazo[1,5-a]pyrazin-3(2H)-one or a salt thereof,
   (specifically,
2-(2-thienyl)hexahydroimidazo[1,5-a]pyrazin-3(2H)-one trifluoroacetate,
2-(3-thienyl)hexahydroimidazo[1,5-a]pyrazin-3(2H)-one hydrochloride,
2-[5-methyl-2-(trifluoromethyl)-3-furyl]hexahydroimidazo[1,5-a]pyrazin-3(2H)-one hydrochloride, or
2-(3-ethylphenyl)hexahydroimidazo[1,5-a]pyrazin-3(2H)-one hydrochloride).

When compound (I) is a salt, examples of the salt include salt with inorganic base, ammonium salt, salt with organic base, salt with inorganic acid, salt with organic acid, salt with basic or acidic amino acid and the like.
Preferable examples of the salt with inorganic base include alkali metal salts such as sodium salt, potassium salt and the like; alkaline earth metal salts such as calcium salt, magnesium salt, barium salt and the like; aluminum salt and the like.
Preferable examples of the salt with organic base include salts with trimethylamine, triethylamine, pyridine, picoline, ethanolamine, diethanolamine, triethanolamine, dicyclohexylamine, N,N'-dibenzylethylenediamine and the like.
Preferable examples of the salt with inorganic acid include salts with hydrochloric acid, hydrobromic acid, nitric acid, sulfuric acid, phosphoric acid and the like.
Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salt with basic amino acid include salts with arginine, lysine, ornithine and the like.
Preferable examples of the salt with acidic amino acid include salts with aspartic acid, glutamic acid and the like.
Of these salts, pharmaceutically acceptable salts are preferable.

Compound (I) encompasses a solvate, for example, hydrate. In addition, compound (I) may be labeled with an isotope (e.g., ³H, ¹⁴C, ³⁵S, ¹²⁵I etc.) and the like.
When compound (I) of the present invention has an asymmetric center, isomers such as enantiomer, diastereomer and the like may be present. Such isomers and a mixture thereof are all encompassed in the scope of the present invention. When an isomer due to conformation is present, such isomer and a mixture thereof are also encompassed in compound (I) of the present invention.

The production methods of compound (I) of the present invention are explained in the following.
Compound (I) and a starting compound thereof can be produced by a means known *per se,* for example, a method shown by the following scheme and the like. In the following, the "room temperature" generally means 10-30°C and, unless otherwise specified, each symbol in the chemical structural formulas in the scheme is as defined above. The compounds in the schemes encompass salts thereof, and examples of such salt include those similar to the salt of compound (I) and the like.
While the compound obtained in each step can be used for the next reaction in the form of a reaction mixture or a crude product. Alternatively, it can also be isolated from a reaction mixture according to a conventional method, and easily purified by a separation means such as recrystallization, distillation, chromatography and the like.

Compound (I) of the present invention can be produced by, for example, the following methods.

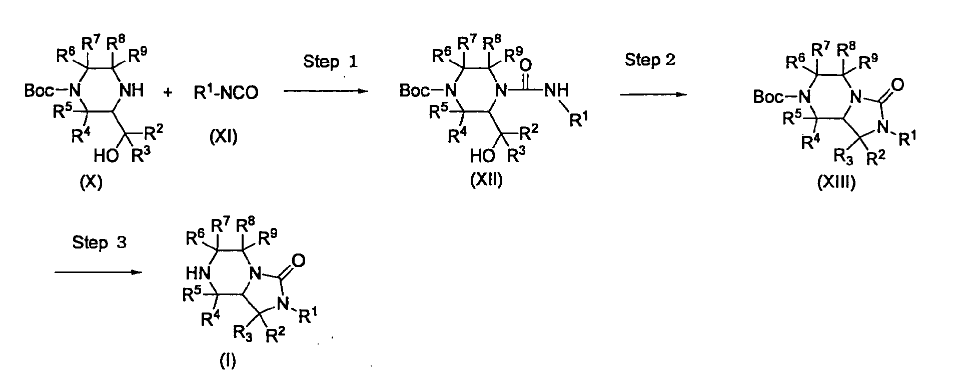

wherein each symbol is as defined above.

### (Step 1)

In this step, a compound represented by the formula (X) or a salt thereof (hereinafter to be referred to as compound (X)) is subjected to a ureation reaction to produce a compound represented by the formula (XII) or a salt thereof (hereinafter to be referred to as compound (XII)).
The ureation reaction is carried out by a conventional method in the presence of an isocyanate represented by the formula (XI) (hereinafter to be referred to as isocyanate (XI)) in a solvent that does not adversely influence the reaction. The amount of isocyanate (XI) to be used is preferably about 1 to about 5 molar equivalents relative to compound (X).
Examples of the solvent that does not adversely influence the reaction include ethers such as tetrahydrofuran and the like; halogenated hydrocarbons such as chloroform and the like; aromatic hydrocarbons such as toluene and the like; amides such as N,N-dimethylformamide and the like; sulfoxides such as dimethyl sulfoxide and the like, and the like. These solvents may be used in a mixture of two or more kinds at an appropriate ratio. The amount of the solvent to be used is generally 1- to 100-fold volume relative to compound (X).
The reaction temperature is generally about -50°C to about 250°C, preferably 0°C to 120°C.
The reaction time is generally about 0.5 to about 24 hr. The thus-obtained compound (XII) can be isolated and purified by a known separation and purification means, for example, concentration, concentration under reduced pressure, solvent extraction, crystallization, recrystallization, phase transfer, chromatography and the like.

### (Step 2)

In this step, compound (XII) is subjected to cyclization using the Mitsunobu reaction and the like to produce a compound represented by the formula (XIII) or a salt thereof (hereinafter to be referred to as compound (XIII)).
This reaction can be carried out according to a method known per se, for example, the method described in Synthesis, page 1, 1981, and the like, or a method analogous thereto.
That is, this reaction is carried out in the presence of an organic phosphoric compound and an electrophile in a solvent that does not adversely influence the reaction. Examples of the organic phosphoric compound include triphenylphosphine, tributylphosphine and the like. Examples of the electrophile include diethyl azodicarboxylate, diisopropyl azodicarboxylate, dipiperazine azodicarboxylate and the like.
The amount of each of the organic phosphoric compound and electrophile to be used is preferably about 1 to about 5 molar equivalents relative to compound (XII). Examples of the solvent that does not adversely influence the reaction include ethers such as tetrahydrofuran and the like; halogenated hydrocarbons such as chloroform and the like; aromatic hydrocarbons such as toluene and the like; amides such as N,N-dimethylformamide and the like; sulfoxides such as dimethyl sulfoxide and the like, and the like. These solvents may be used in a mixture of two or more kinds at an appropriate ratio. The amount of the solvent to be used is generally 1- to 100-fold volume relative to compound (XII).
The reaction temperature is generally about -50°C to about 250°C, preferably 0°C to 120°C.
The reaction time is generally about 0.5 to about 24 hr. The thus-obtained compound (XIII) can be isolated and purified by a known separation and purification means, for example, concentration, concentration under reduced pressure, solvent extraction, crystallization, recrystallization, phase transfer, chromatography and the like.

### (Step 3)

In this step, the tert-butoxycarbonyl of compound (XIII) is removed to convert compound (XIII) to compound (I). This reaction can be carried out according to a method known per se, generally by reacting compound (XIII) with an acid in a solvent that does not adversely influence the reaction.
Examples of the acid include hydrochloric acid, hydrobromic acid, sulfuric acid, trifluoroacetic acid, trifluoromethanesulfonic acid, hydrogen chloride and the like. The amount of the acid to be used is preferably about 1 to about 100 molar equivalents, relative to compound (XIII).
Examples of the solvent that does not adversely influence the reaction include alcohols such as methanol and the like; ethers such as tetrahydrofuran and the like; halogenated hydrocarbons such as chloroform and the like; aromatic hydrocarbons such as toluene and the like; amides such as N,N-dimethylformamide and the like; sulfoxides such as dimethyl sulfoxide and the like; esters such as ethyl acetate and the like, and the like. These solvents may be used in a mixture of two or more kinds at an appropriate ratio. The amount of the solvent to be used is generally 1- to 100-fold volume relative to compound (XIII).
The reaction temperature is generally about -50°C to about 250°C, preferably 0°C - 120°C. The reaction time is generally about 0.5 to about 24 hr.
The thus-obtained compound (I) can be isolated and purified by known separation and purification means, for example, concentration, concentration under reduced pressure, solvent extraction, crystallization, recrystallization, phase transfer, chromatography, distillation and the like.

When compound (I) contains an optical isomer, a stereoisomer, a regioisomer or a rotamer, these are also encompassed in compound (I), and can be obtained as a single product according to synthesis and separation methods known per se (e.g., concentration, solvent extraction, column chromatography, recrystallization, etc.). For example, when compound (I) has an optical isomer, an optical isomer resolved from this compound is also encompassed in compound (I).
The optical isomer can be produced by a method known per se. To be specific, an optically active synthetic intermediate is used, or the final racemate product is subjected to optical resolution according to a conventional method to give an optical isomer.
The method of optical resolution may be a method known per se, such as a fractional recrystallization method, a chiral column method, a diastereomer method, etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.) is formed, which is separated by a fractional recrystallization method, and if desired, a free optical isomer is obtained by a neutralization step.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, isopropanol; acetonitrile, trifluoroacetic acid, diethylamine, etc.) solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method, etc.) and the like, and is subjected to a chemical treatment such as hydrolysis and the like to separate an optically active reagent moiety, whereby an optical isomer is obtained. For example, when compound (I) contains a hydroxyl group, or a primary or secondary amino group in a molecule, the compound and an optically active organic acid (e.g., MTPA [α-methoxy-α-(trifluoromethyl)phenylacetic acid], (-)-menthoxyacetic acid, etc.) and the like are subjected to condensation reaction to give diastereomers in the ester form or in the amide form, respectively. When compound (I) has carboxyl, this compound and an optically active amine or optically active alcohol are subjected to condensation reaction to give diastereomers in the amide form or in the ester form, respectively. The separated diastereomer is converted to an optical isomer of the original compound by acid hydrolysis or base hydrolysis.

The compound (I) may be a crystal.
The crystal of the compound (I) can be produced by crystallization of compound (I) according to crystallization methods known *per se.*
Examples of the crystallization method include a method of crystallization from a solution, a method of crystallization from vapor, a method of crystallization from the melts and the like.

The "crystallization from a solution" is typically a method of shifting a non-saturated state to supersaturated state by varying factors involved in solubility of compounds (solvent composition, pH, temperature, ionic strength, redox state, etc.) or the amount of solvent. To be specific, for example, a concentration method, a slow cooling method, a reaction method (a diffusion method, an electrolysis method), a hydrothermal growth method, a flux method and the like. Examples of the solvent to be used include aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, dioxane, etc.), nitriles (e.g., acetonitrile, etc.), ketones (e.g., acetone, etc.), sulfoxides (e.g., dimethyl sulfoxide, etc.), acid amides (e.g., N,N-dimethylformamide, etc.), esters (e.g., ethyl acetate, etc.), alcohols (e.g., methanol, ethanol, isopropyl alcohol, etc.), water and the like. These solvents are used alone or in a combination of two or more at a suitable ratio (e.g., 1:1 to 1:100 (a volume ratio)). Where necessary, a seed crystal can be used.
The "crystallization from vapor" is, for example, a vaporization method (a sealed tube method, a gas stream method), a gas phase reaction method, a chemical transportation method and the like.
The "crystallization from the melts" is, for example, a normal freezing method (a Czockralski method, a temperature gradient method and a Bridgman method, etc.), a zone melting method (a zone leveling method and a floating zone method, etc.), a special growth method (a VLS method and a liquid phase epitaxy method, etc.) and the like.

Preferable examples of the crystallization method include a method of dissolving compound (I) in a suitable solvent (e.g., alcohols such as methanol, ethanol, etc., and the like) at a temperature of 20 to 120°C, and cooling the resulting solution to a temperature not higher than the temperature of dissolution (e.g., 0 to 50°C, preferably 0 to 20°C) and the like.
The thus obtained crystals of the present invention can be isolated, for example, by filtration and the like.
As an analysis method of the obtained crystal is generally a method of crystal analysis by powder X-ray diffraction. As a method of determining crystal orientation, a mechanical method or an optical method and the like can also be used.
The crystal of compound (I) obtained by the above-mentioned production method (hereinafter to be abbreviated as "the crystal of the present invention") has high purity, high quality, and low hygroscopicity, is not denatured even after a long-term preservation under general conditions, and is extremely superior in the stability. In addition, it is also superior in the biological properties (e.g., pharmacokinetics (absorption, distribution, metabolism, excretion), efficacy expression etc.) and is extremely useful as a pharmaceutical agent.
In the present specification, the melting point means a melting point measured using, for example, a micro melting point apparatus (YANACO, MP-500D), a DSC (differential scanning calorimetry) apparatus (SEIKO, EXSTAR6000) and the like.

A prodrug of the compound (I) means a compound which is converted to the compound (I) with a reaction due to an enzyme, an gastric acid, etc. under the physiological condition in the living body, that is, a compound which is converted to the compound (I) with oxidation, reduction, hydrolysis, etc. according to an enzyme; a compound which is converted to the compound (I) by hydrolysis etc. due to gastric acid, etc. A prodrug for compound (I) may be a compound obtained by subjecting amino in compound (I) to an acylation, alkylation or phosphorylation [e.g., a compound obtained by subjecting amino in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.]; a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation, dimethylaminomethylcarbonylation, etc.); a compound obtained by subjecting carboxy in compound (I) to an esterification or amidation [e.g., a compound obtained by subjecting carboxy in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification, methylamidation, etc.] and the like. Any of these compounds can be produced from compound (I) by a method known per se.
A prodrug for compound (I) may also be one which is converted into compound (I) under a physiological condition, such as those described in IYAKUHIN no KAIHATSU (Development of Pharmaceuticals), Vol. 7, Design of Molecules, p.163-198, Published by HIROKAWA SHOTEN (1990).
Compound (I) of the present invention or a prodrug thereof (hereinafter to be simply abbreviated as compound (I)) has a superior serotonin 5-HT_{2C} receptor activating action.
In addition, compound (I) of the present invention has low toxicity and is safe.

Accordingly, compound (I) of the present invention having a superior serotonin 5-HT_{2C} receptor activating action is useful as a prophylaxis or therapeutic drug for all serotonin 5-HT_{2C} associated diseases in mammals (e.g., mouse, rat, hamster, rabbit, cat, dog, bovine, sheep, monkey, human and the like), for example,
(1) lower urinary tract symptoms [for example, abnormal urination such as overactive bladder, stress urinary incontinence, mixed urinary incontinence, lower urinary tract symptoms associated with benign prostatic hyperplasia, pelvic visceral pain, lower urinary tract symptoms associated with chronic prostatitis, lower urinary tract symptoms associated with interstitial cystitis etc. and the like]
(2) metabolic diseases [for example, diabetes (insulin dependent diabetes, diabetic complications, diabetic retinopathy, diabetic microangiopathy, diabetic neuropathy etc.), impaired glucose tolerance, obesity [e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity], benign prostatic hyperplasia, sexual dysfunction and the like]
(3) central nervous system diseases [for example, neurodegenerative diseases (e.g., Alzheimer's disease, Down's disease, Parkinson's disease, Creutzfeldt-Jakob disease, amyotrophic lateral sclerosis (ALS), Huntington chorea, diabetic neuropathy, multiple sclerosis etc.), mental diseases (e.g., schizophrenia, depression, mania, anxiety neurosis, obsessive-compulsive neurosis, panic disorder, epilepsy, alcohol dependence, drug dependence, anxiety, anxious mental state, emotional abnormality, cyclothymia, nervous erethism, autism, faint, addiction, low sex drive etc.), disorders such as central nervous system and peripheral nerve disorders (e.g., head trauma, spinal trauma, brain edema, disorders of sensory function, abnormality of sensory function, disorders of autonomic nervous function, abnormality of autonomic nervous function, whiplash injury etc.), memory disorders (e.g., senile dementia, amnesia, cerebrovascular dementia etc.), cerebrovascular disorder (e.g., cerebral hemorrhage, cerebral infarction and the like and sequelae or complication thereof, asymptomatic cerebrovascular accident, transient cerebral ischemic attack, hypertensive encephalopathia, blood-brain barrier disorder, etc.), recurrence and sequelae of cerebrovascular disorders (e.g., neural symptoms, mental symptoms, subjective symptoms, disorders of daily living activities etc.), central nervous system hypofunction after brain blood vessel occlusion, disorder or abnormality of autoregulation ability of brain circulation or renal circulation etc.], sleep disorder
(4) genital insufficiency diseases [for example, male erectile dysfunction, dysspermia, premature ejaculation, female genital insufficiency etc.]
(5) digestive organ diseases [for example, an irritable bowel syndrome, inflammatory bowel disease, ulcerative colitis, Crohn's disease, diseases caused by a spiral urease-positive gram-negative bacterium (e.g., Helicobacter pylori, etc.) (e.g., gastritis, gastric ulcer, etc.), gastric cancer, postgastrostomy disorder, indigestion, esophageal ulcer, pancreatitis, polyp of the colon, cholelithiasis, hemorrhoids, peptic ulcer, situational ileitis, gluttony, constipation, diarrhea, borborygmus, etc.]
(6) inflammatory or allergic diseases [for example, allergic rhinitis, conjunctivitis, gastrointestinal allergy, pollinosis, anaphylaxis, dermatitis, herpes, psoriasis, bronchitis, expectoration, retinopathy, postoperative and posttraumatic inflammation, regression of puffiness, pharyngitis, cystitis, meningitidis, inflammatory ophthalmic diseases, etc.]
(7) osteoarthropathy diseases [for example, rheumatoid arthritis (chronic rheumatoid arthritis), arthritis deformans, rheumatoid myelitis, osteoporosis, abnormal growth of cells, bone fracture, bone refracture, osteomalacia, osteopenia, Paget's disease of bone, rigid myelitis, articular tissue destruction by gonarthrosis deformans and similar diseases thereto, etc.]
(8) respiratory diseases [for example, cold syndrome, pneumonia, asthma, pulmonary hypertension, pulmonary thrombi/pulmonary obliteration, pulmonary sarcoidosis, pulmonary tuberculosis, interstitial pneumonia, silicosis, adult respiratory distress syndrome, chronic obstructive pulmonary disease, cough, etc.]
(9) infectious diseases [HIV infectious diseases, virus infectious diseases due to cytomegalo virus, influenza virus, herpes virus and the like, rickettsia infectious diseases, bacterial infectious diseases, sexually-transmitted diseases, carinii pneumonia, Helicobacter pylori infectious disease, systemic fungal infectious diseases, tuberculosis, invasive staphylococcal infectious diseases, acute viral encephalitis, acute bacterial meningitidis, AIDS encephalitis, septicemia, sepsis, sepsis gravis, septic shock, endotoxin shock, toxic shock syndromes, etc.]
(10) cancers [for example, primary, metastatic or recurrent breast cancer, prostatic cancer, pancreatic cancer, gastric cancer, lung cancer, colorectal cancer (colon cancer, rectal cancer, anal cancer), esophagus cancer, duodenal cancer, head and neck cancer (cancer of the tongue, pharynx cancer, laryngeal cancer), brain tumor, schwannoma, non-small cell lung cancer, small cell lung cancer, liver cancer, kidney cancer, biliary tract cancer, uterine cancer (endometrial cancer, cancer of the uterine cervix), ovarian cancer, urinary bladder cancer, skin cancer, Hemangioma, malignant lymphoma, malignant melanoma, thyroid cancer, bone tumor, Hemangioma, vascular fibroma, retinosarcoma, penile cancer, solid cancer in childhood, Kaposi's sarcoma, Kaposi's sarcoma caused by AIDS, maxillary tumor, fibrous histiocytoma, leiomyosarcoma, rhabdomyosarcoma, liposarcoma, fibroid tumors of the uterus, osteoblastoma, osteosarcoma, chondrosarcoma, cancerous mesothelioma, tumors such as leukemia, Hodgkin's disease, etc.]
(11) circulatory diseases [for example, acute coronary artery syndromes (e.g., acute myocardial infarction, unstable angina, etc.), peripheral arterial occlusion, Raynaud's disease, Buerger's disease, restenosis after coronary-artery intervention (percutaneous transluminal coronary angioplasty (PTCA), directional coronary atherectomy (DCA), stenting, etc.), restenosis after coronary-artery bypass operation, restenosis after intervention (angioplasty, atherectomy, stenting, etc.) or bypass operation in other peripheral artery, ischemic cardiac diseases (e.g., myocardial infarction, angina, etc.), myocarditis, intermittent claudication, lacunar infarction, arteriosclerosis (e.g., atherosclerosis, etc.), cardiac failure (acute cardiac failure, chronic cardiac failure including congestive cardiac failure), arrhythmia, progress of atherosclerotic plaque, thrombosis, hypertension, hypertensive tinnitus, hypotension, etc.]
(12) pains [e.g., headache, migraine, neuralgia, pelvic visceral pain (including cystalgia), etc.]
(13) autoimmune diseases [for example, collagen disease, systemic lupus erythematosus, scleroderma, polyarteritis, myasthenia gravis, multiple sclerosis, Sjogren's syndrome, Behcet's disease, etc.]
(14) hepatic diseases [e.g., hepatitis (including chronic hepatitis), cirrhosis, interstitial hepatic diseases, etc.]
(15) pancreatic diseases [e.g., pancreatitis (including chronic pancreatitis), etc.]
(16) renal diseases [e.g., nephritis, glomerulonephritis, glomerulosclerosis, renal failure, thrombotic microangiopathy, dialysis complications, organ disorders including nephropathia by radiation, diabetic nephropathy, etc.]
(17) endocrine diseases [e.g., Addison's disease, Cushing's syndrome, melanocytoma, primary aldosteronism, etc.]
(18) other diseases
   (a) transplant rejection [e.g., posttransplantational rejection, posttransplantational polycythemia, hypertension, organ disorder and/or vascular hypertrophy, graft-versus-host disease, etc.]
   (b) abnormality in characteristic of blood and/or blood components [e.g., enhancement in platelet aggregation, abnormality of erythrocyte deformability, enhancement in leukocyte adhesiveness, increase in blood viscosity, polycythemia, vascular peliosis, autoimmune hemolytic anemia, disseminated intravascular coagulation syndrome (DIC), multiple myelopathy, etc.]
   (c) gynecologic diseases [e.g., climacteric disorder, gestational toxicosis, endometriosis, hysteromyoma, ovarian disease, mammary disease, premenstrual syndrome, pelvic organ prolapse, (e.g., prolapse of anterior wall of the vagina, prolapse of vaginal apex, prolapse of posterior wall of vagina, prolapse of uterus etc.), other diseases where organ is prolapsed from the normal position due to weakened pelvic floor muscle (e.g., rectal prolapse etc.) and the like]
   (d) dermatic diseases [e.g., keloid, Hemangioma, psoriasis, pruritus, etc.]
   (e) ophthalmic diseases [e.g., glaucoma, ocular hypertension disease, etc.]
   (f) otolaryngological diseases [e.g., Menuel syndrome, tinnitus, gustation disorder, dizziness, disequilibrium, dysphagia, etc.]
   (g) diseases due to environmental and/or occupational factors (e.g., radiation disorder, disorders by ultraviolet ray/infrared ray/laser ray, altitude sickness, etc.)
   (h) ataxia, stiffness, tremor, motion impairment, akinesia
   (i) chronic fatigue syndrome
   (j) sudden infant death syndrome
   (k) hiccup
   (l) diseases causing palpitation, vertigo, heartburn and the like.
In these diseases, the compound of the present invention is particularly useful as a serotonin 5-HT_{2C} receptor activator, as an ameliorator for lower urinary tract symptoms such as overactive bladder and/or stress urinary incontinence, as a prophylactic or therapeutic agent for these lower urinary tract symptoms, a prophylactic or therapeutic drug for obesity or a prophylactic or therapeutic drug for pelvic organ prolapse.
Preparations comprising compound (I) of the present invention may be in any solid forms of powders, granules, tablets, capsules, etc., and in any liquid forms of syrups, emulsions, injections, etc.
The preparations of the present invention for prophylaxis or treatment can be produced by any conventional methods, for example, blending, kneading, granulation, tableting, coating, sterilization, emulsification, etc., in accordance with the forms of the preparations to be produced. For the production of such pharmaceutical preparations, for example, each of the items in General Rules for Preparations in the Japanese Pharmacopoeia, can be made reference to. In addition, the preparations of the present invention may be formulated into a sustained release preparation containing active ingredients and biodegradable polymer compounds. The sustained release preparation can be produced according to the method described in JP-A-9-263545.

In the preparations of the present invention, the content of the compound (I) varies depending on the forms of the preparations, but is generally in the order of 0.01 to 100 % by weight, preferably 0.1 to 50 % by weight, more preferably 0.5 to 20 % by weight, relative to the total weight of each preparation.
When the compound (I) of the present invention is used in the above-mentioned pharmaceutical products, it may be used alone, or in admixture with a suitable, pharmacologically acceptable carrier, for example, excipients (e.g., starch, lactose, sucrose, calcium carbonate, calcium phosphate, etc.), binders (e.g., starch, arabic gum, carboxymethyl cellulose, hydroxypropyl cellulose, crystalline cellulose, alginic acid, gelatin, polyvinylpyrrolidone, etc.), lubricants (e.g., stearic acid, magnesium stearate, calcium stearate, talc, etc.), disintegrants (e.g., calcium carboxymethylcellulose, talc, etc.), diluents (e.g., water for injection, physiological saline, etc.) and if desired, with the additives (e.g., a stabilizer, a preservative, a colorant, a fragrance, a solubilizing agent, an emulsifier, a buffer, an isotonic agent, etc.) and the like, by ordinary methods. It can be formulated into the solid preparations such as powders, fine granules, granules, tablets, capsules, etc., or into the liquid preparations such as injections, etc., and can be administered orally or parenterally. When compound (I) is formed as a preparation for topical administration and administered, it can also be directly administered to the affected part of an articular disease. In this case, an injection is preferable. It can also be administered as a parenteral agent for topical administration (e.g., intramuscular injection, subcutaneous injection, organ injection, injection to the vicinity of a joint and the like, solid preparation such as implant, granule, powder and the like, liquid such as suspension and the like, ointment etc.) and the like.

For formulation into an injection, for example, compound (I) is formulated into an aqueous suspension with a dispersing agent (e.g., surfactant such as Tween 80, HCO-60 and the like, polysaccharides such as carboxymethylcellulose, sodium alginate, hyaluronic acid and the like, polysorbate etc.), preservative (e.g., methylparaben, propylparaben etc.), isotonic agent (e.g., sodium chloride, mannitol, sorbitol, glucose etc.), buffer (e.g., calcium carbonate etc.), pH adjuster (e.g., sodium phosphate, potassium phosphate etc.) and the like to give a preparation for practical injection. In addition, an oily suspension can be obtained by dispersing compound (I) together with vegetable oil such as sesame oil, corn oil and the like or a mixture thereof with a phospholipid such as lecithin and the like, or medium-chain triglyceride (e.g., miglyol 812 etc.) to give an injection to be actually used.

An agent for the prophylaxis or treatment of the present invention can be used along with other pharmaceutical agent.
As the drug that can be mixed with or concomitantly used with compound (I) of the present invention (hereinafter to be abbreviated as concomitant drug), for example, the following drugs can be used.

### (1) Other drugs for treating stress urinary incontinence

Adrenaline α1 receptor agonists (e.g., ephedrine hydrochloride, midodrine hydrochloride), adrenaline β2 receptor agonists (e.g., Clenbuterol), noradrenaline uptake inhibitory substances, noradrenaline and serotonin uptake inhibitory substances (e.g., duloxetine), tricyclic antidepressants (e.g., imipramine hydrochloride), anticholinergic agents or smooth muscle stimulants (e.g., oxybutynin hydrochloride, propiverine hydrochloride, celimeverine hydrochloride), female hormone drugs (e.g., conjugated estrogen (premarin), estriol) and the like.

### (2) Agent for treating diabetes

Insulin preparations [e.g., animal insulin preparations extracted from the bovine or swine pancreas; human insulin preparations synthesized by a genetic engineering technique using Escherichia coli or a yeast; insulin zinc; protamine zinc insulin; a fragment or a derivative of insulin (e.g., INS-1, etc.)], insulin sensitizers (e.g., pioglitazone hydrochloride, troglitazone, rosiglitazone or its maleate, JTT-501, MCC-555, YM-440, GI-262570, KRP-297, FK-614, CS-011, etc.), α-glucosidase inhibitors (e.g., voglibose, acarbose, miglitol, emiglitate, etc.), biguanides (e.g., phenformin, metformin, buformin, etc.), sulfonylureas (e.g., tolbutamide, glibenclamide, gliclazide, chlorpropamide, tolazamide, acetohexamide, glyclopyramide, glimepiride, etc.) and other insulin secretagogues (e.g., repaglinide, senaglinide, mitiglinide or its calcium salt hydrate, GLP-1, nateglinide, etc.), dipeptidylpeptidase IV inhibitors (e.g., vildagliptin, sitagliptin, saxagliptin, alogliptin, NVP-DPP-728, PT-100, P32/98, etc.), β3 agonists (e.g., CL-316243, SR-58611-A, UL-TG-307, AJ-9677, AZ40140, etc.), amylin agonists (e.g., pramlintide, etc.), phosphotyrosine phosphatase inhibitors (e.g., vanadic acid, etc.), gluconeogenesis inhibitors (e.g., glycogen phosphorylase inhibitors, glucose-6-phosphatase inhibitors, glucagon antagonists, etc.), SGLT (sodium-glucose cotransporter) inhibitors (e.g., T-1095, etc.) and the like.

### (3) Agent for treating diabetic complications

Aldose reductase inhibitors (e.g., tolrestat, epalrestat, zenarestat, zopolrestat, fidarestat (SNK-860), minalrestat (ARI-509), CT-112, etc.), neurotrophic factors (e.g., NGF, NT-3, etc.), AGE inhibitors (e.g., ALT-945, pimagedine, pyratoxathine, N-phenacylthiazolium bromide (ALT-766), EXO-226, etc.), active oxygen scavengers (e.g., thioctic acid, etc.), cerebral vasodilators (e.g., tiapride, etc.) and the like.

### (4) Antihyperlipidemic agent

Satin compounds inhibiting cholesterol synthesis (e.g., pravastatin, simvastatin, lovastatin, atorvastatin, fluvastatin, cerivastatin or their salt (e.g., sodium salt, etc.), etc.), squalene synthase inhibitors, fibrate compounds having triglyceride lowering action (e.g., bezafibrate, clofibrate, simfibrate, clinofibrate, etc.) and the like.

### (5) Hypotensive agent

Angiotensin converting enzyme inhibitors (e.g., captopril, enalapril, delapril, etc.), angiotensin II antagonists (e.g., losartan, candesartan cilexetil, etc.), calcium antagonists (e.g., manidipine, nifedipine, amlodipine, efonidipine, nicardipine, etc.), clonidine, and the like.

### (6) Antiobesity agent

Antiobesity drugs acting on the central nervous system (e.g. dexfenfluramine, fenfluramine, phentermine, sibutramine, anfepramone, dexamphetamine, mazindol, phenylpropanolamine, clobenzorex, etc.), pancreatic lipase inhibitors (e.g. orlistat, etc.), β3 agonists (e.g. CL-316243, SR-58611-A, UL-TG-307, AJ-9677, AZ40140, etc.), anorectic peptides (e.g. leptin, CNTF (Ciliary Neurotrophic Factor), etc.), cholecystokinin agonists (e.g. lintitript, FPL-15849, etc.).

### (7) Diuretic agent

Xanthine derivatives (e.g., theobromine sodium salicylate, theobromine calcium salicylate, etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide, etc.), antialdosterone preparations (e.g., spironolactone, triamterene, etc.), carbonic anhydrase inhibitors (e.g., acetazolamide, etc.), chlorobenzenesulfonamide preparations (e.g., chlorthalidone, mefruside, indapamide, etc.), azosemide, isosorbide, ethacrynic acid, piretanide, bumetanide, furosemide, etc.

### (8) Chemotherapeutic agent

Alkylating agents (e.g., cyclophosphamide, ifosamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, taxol, etc.), cisplatin, carboplatin, etoposide, etc. Among these, 5-fluorouracil derivatives such as Furtulon and Neo-Furtulon are preferred.

### (9) Immunotherapeutic agent

Microorganism- or bacterium-derived components (e.g., muramyl dipeptide derivatives, Picibanil, etc.), immunopotentiator polysaccharides (e.g., lentinan, schizophyllan, krestin, etc.), genetically engineered cytokines (e.g., interferons, interleukins (IL), etc.), colony stimulating factors (e.g., granulocyte colony stimulating factor, erythropoietin, etc.) and the like. Among these, IL-1, IL-2, IL-12, etc. are preferred.

### (10) Therapeutic agent recognized to ameliorate cachexia in animal models or clinical practice

Progesterone derivatives (e.g., megestrol acetate) [Journal of Clinical Oncology, vol. 12, pp. 213-225, 1994], metoclopramide pharmaceuticals, tetrahydrocannabinol pharmaceuticals (the above references are applied to both), fat metabolism ameliorating agents (e.g., eicosapentaenoic acid) [British Journal of Cancer, vol. 68, pp. 314-318, 1993], growth hormones, IGF-1, and antibodies to the cachexia-inducing factors such as TNF-α, LIF, IL-6 and oncostatin M.

### (11) Antiinflammatory agent

Steroids (e.g., dexamethasone, etc.), sodium hyaluronate, cyclooxygenase inhibitors (e.g., indomethacin, ketoprofen, loxoprofen, meloxicam, ampiroxicam, celecoxib, rofecoxib, etc.) and the like.

### (12) Miscellaneous

Glycosylation inhibitors (e.g., ALT-711, etc.), nerve regeneration promoting drugs (e.g., Y-128, VX853, prosaptide, etc.), drugs acting on the central nervous system (e.g., antidepressants such as desipramine, amitriptyline, imipramine, fluoxetine, paroxetine, doxepin, etc.), anticonvulsants (e.g., lamotrigine, carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine, paroxetine), narcotic analgesics (e.g., morphine), GABA receptor agonists (e.g., gabapentin), GABA uptake inhibitors (e.g., tiagabine), α₂ receptor agonists (e.g., clonidine), local analgesics (e.g., capsaicin), protein kinase C inhibitors (e.g., LY-333531), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatryptan), serotonin receptor antagonists (e.g., cyproheptadine hydrochloride, ondansetron), serotonin uptake inhibitors (e.g., fluvoxamine maleate, fluoxetine, paroxetine), hypnotics (e.g., triazolam, zolpidem), anticholinergic agents, α₁ receptor blocking agents (e.g., tamsulosin, silodosin, naftopidil), muscle relaxants (e.g., baclofen), potassium channel openers (e.g., nicorandil), calcium channel blocking agents (e.g., nifedipine), agents for preventing and/or treating Alzheimer's disease (e.g., donepezil, rivastigmine, galanthamine), agents for treating Parkinson's disease (e.g., L-dopa), agents for preventing and/or treating multiple sclerosis (e.g., interferon β-1a), histamine H₁ receptor inhibitors (e.g., promethazine hydrochloride), proton pump inhibitors (e.g., lansoprazole, omeprazole), antithrombotic agents (e.g., aspirin, cilostazol), NK-2 receptor antagonists, agents of treating HIV infection (saquinavir, zidovudine, lamivudine, nevirapine), agents of treating chronic obstructive pulmonary diseases (salmeterol, thiotropium bromide, cilomilast), etc.
Anticholinergic agents include, for example, atropine, scopolamine, homatropine, tropicamide, cyclopentolate, butylscopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrogen bromide, homatropine hydrogen bromide, cyclopentolate hydrochloride, flavoxate hydrochloride, pirenzepine hydrochloride, trihexyphenidyl hydrochloride, oxybutynin hydrochloride, tolterodine tartrate, etc.), preferably, oxybutynin, propiverine, darifenacin, tolterodine, temiverine, trospium chloride or a salt thereof (e.g., oxybutynin hydrochloride, tolterodine tartrate, etc.). In addition, acetylcholinesterase inhibitors (e.g., distigmine, etc.) and the like can be used.
NK-2 receptor antagonists include, for example, a piperidine derivative such as GR159897, GR149861, SR48968 (saredutant), SR144190, YM35375, YM38336, ZD7944, L-743986, MDL105212A, ZD6021, MDL105172A, SCH205528, SCH62373, R-113281, etc., a perhydroisoindole derivative such as RPR-106145, etc., a quinoline derivative such as SB-414240, etc., a pyrrolopyrimidine derivative such as ZM-253270, etc., a pseudopeptide derivative such as MEN11420 (nepadutant), SCH217048, L-659877, PD-147714 (CAM-2291), MEN10376, S16474, etc., and others such as GR100679, DNK333, GR94800, UK-224671, MEN10376, MEN10627, or a salt thereof, and the like.

In combination of the compound of the present invention and the concomitant drug, the administration time of the compound (I) and the concomitant drug is not restricted, and the compound (I) or a pharmaceutical composition thereof and the concomitant drug or a pharmaceutical composition thereof can be administered to the administration subject simultaneously, or may be administered at different times. The dosage of the concomitant drug may be determined according to the dose clinically used, and can be appropriately selected depending on the administration subject, administration route, disease, combination and the like.
The concomitant administration mode is not particularly restricted, and it is sufficient that the compound (I) and the concomitant drug are combined in administration. Examples of such administration mode include the following methods: (1) The compound (I) or a pharmaceutical composition thereof and the concomitant drug are simultaneously produced to give a single preparation which is administered. (2) The compound (I) or a pharmaceutical composition thereof and the concomitant drug or a pharmaceutical composition thereof are separately produced to give two kinds of preparations which are administered simultaneously by the same administration route. (3) The compound (I) or a pharmaceutical composition thereof and the concomitant drug or a pharmaceutical composition thereof are separately produced to give two kinds of preparations which are administered by the same administration route only at the different times. (4) The compound (I) or a pharmaceutical composition thereof and the concomitant drug or a pharmaceutical composition thereof are separately produced to give two kinds of preparations which are administered simultaneously by different administration routes. (5) The compound (I) or a pharmaceutical composition thereof and the concomitant drug or a pharmaceutical composition thereof are separately produced to give two kinds of preparations which are administered by different administration routes at different times (e.g., the compound (I) or a pharmaceutical composition thereof; the concomitant drug or a pharmaceutical composition thereof are administered in this order, or in the reverse order).
The mixing ratio of compound (I) and a concomitant drug in the combination drug of the present invention can be appropriately determined according to the subject of administration, administration route, disease and the like.
For example, while the content of compound (I) in the combination drug of the present invention varies depending on the form of the preparation, it is generally about 0.01 to about 100 wt%, preferably about 0.1 to about 50 wt%, more preferably about 0.5 to about 20 wt%, relative to the whole preparation.

While the content of the concomitant drug in the combination drug of the present invention varies depending on the form of the preparation, it is generally about 0.01 to about 100 wt%, preferably about 0.1 to about 50 wt%, more preferably about 0.5 to about 20 wt%, relative to the whole preparation.
While the content of the additive such as a carrier and the like in the combination drug of the present invention varies depending on the form of the preparation, it is generally about 1 to about 99.99 wt%, preferably about 10 to about 90 wt%, relative to the whole preparation.
Similar contents can be employed when compound (I) and the concomitant drug are independently formulated.
While the dose varies depending on the kind of compound (I) or a pharmaceutically acceptable a salt thereof, administration route, symptom, age of patients and the like, for example, for oral administration to an adult patient with stress urinary incontinence, obesity and/or pelvic organ prolapse, it is about 0.005 - 50 mg, preferably about 0.05 - 10 mg, more preferably about 0.2 - 4 mg/kg body weight/day as compound (I), which can be administered in 1 to about 3 portions.
When the pharmaceutical composition of the present invention is a sustained-release preparation, the dose varies depending on the kind and content of compound (I), dosage form, period of sustained drug release, subject animal of administration (e.g., mammals such as human, rat, mouse, cat, dog, rabbit, bovine, swine and the like) and administration object. For parenteral administration, for example, about 0.1 to about 100 mg of compound (I) only needs to be released in one week from the administered preparation.
The dose of the concomitant drug may be set within the range such that it causes no problems of side effects. The daily dose as the concomitant drug varies depending on severity of symptoms, age, sex, weight and sensitivity of the subject to be administered, time and interval of administration, property, formulation and kinds of pharmaceutical preparation, kinds of active ingredients, etc., and is not particularly limited. In the case of oral administration, a daily dosage in terms of drugs is usually in the order of about 0.001 to 2000 mg, preferably about 0.01 to 500 mg, and more preferably about 0.1 to 100 mg, per 1 kg body weight of mammals, which may be administered once a day or in two to four divided portions a day.

In administering the combination drug of the present invention, it may be administered at the same time or, the concomitant drug may be administered before administering the compound (I), and vice versa. In case of staggered administration, the time interval varies depending on the active ingredients to be administered, a formulation and an administration route. For example, if the concomitant drug is administered first, the compound (I) may be administered 1 minute to 3 days, preferably 10 minutes to 1 day, more preferably 15 minutes to 1 hour after administering the concomitant drug. If the compound (I) is administered first, the concomitant drug may be administered 1 minute to 1 day, preferably 10 minutes to 6 hours, more preferably 15 minutes to 1 hour after administering the compound (I).
The pharmaceutical composition of the present invention shows low toxicity and can be used safely. Particularly, since the Example compounds shown below are superior in the absorption by oral administration, they can be advantageously used for oral preparations.

### Examples

The present invention is further described in detail with reference to Examples, Formulation Examples and Experimental Examples which are not intended to restrict the invention and may be modified without departing from the scope of the invention.
Elution in the column chromatography in the following Examples was conducted under observation by TLC (thin layer chromatography), unless otherwise specifically indicated. In the TLC observation, 60F254, TLC plates, produced by Merck & Co., Inc. was used, and the solvent employed as an elution solvent in the column chromatography was used as an eluent. For the detection, a UV detector was used. As silica gel for the column chromatography, Silica Gel 60 (70 to 230 mesh) produced by Merck & Co., Inc. was used. The room temperature referred herein means temperature generally from about 10°C to 30°C. For drying extract, sodium sulfate or magnesium sulfate was used.
The abbreviations in the Examples mean the following.
NMR: nuclear magnetic resonance spectrum
Hz: hertz
J: coupling constant
m: multiplet
q: quartet
t: triplet
d: doublet
dd: double doublet
s: singlet
br: broad
dt: double triplet
brs: broad singlet
N: normal concentration
DMSO: dimethyl sulfoxide
TFA: trifluoroacetic acid
5-HT: serotonin (or 5-hydroxytryptamine)

### Example 1

### 2-phenylhexahydroimidazo[1,5-a]pyrazin-3(2H)-one hydrochloride

### (1) tert-butyl 4-(anilinocarbonyl)-3-(hydroxymethyl)piperazine-1-carboxylate

To a solution of tert-butyl 3-(hydroxymethyl)piperazine-1-carboxylate (1.00 g, 4.62 mmol) in tetrahydrofuran (20 ml) was added phenyl isocyanate (0.603 ml, 5.54 mmol) under ice-cooling, and the mixture was stirred at room temperature for 1 hr. The solvent was evaporated under reduced pressure. The residue was recrystallized from a mixed solvent of hexane and ethyl acetate to give the object product (1.47 g, 94.8%) as a solid.
¹H-NMR (CDCl₃) δ; 1.40 (9H, s), 3.03 - 3.21 (4H, m), 3.67 - 4.25 (6H, m), 6.99 - 7.04 (1H, m), 7.26 - 7.28 (4H, m), 7.50 (1H, br s).

### (2) tert-butyl 3-oxo-2-phenylhexahydroimidazo[1,5-a]pyrazine-7(1H)-carboxylate

To a solution of tert-butyl 4-(anilinocarbonyl)-3-(hydroxymethyl)piperazine-1-carboxylate (1.00 g, 2.98 mmol) and triphenylphosphine (1.56 g, 5.96 mmol) in N,N-dimethylformamide (20 ml) was added 40% diethyl azodicarboxylate-toluene solution (2.59 g, 5.96 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (920 mg, 97.2%) as a solid.
¹H-NMR (CDCl₃) δ; 1.49 (9H, s), 2.70 - 2.96 (3H, m), 3.42 - 3.47 (1H, m), 3.71 - 3.75 (1H, m), 3.90 - 3.96 (2H, m), 4.10 - 4.34 (2H, m), 7.03 - 7.08 (1H, m), 7.31 - 7.37 (2H, m), 7.53 - 7.57 (2H, m).

### (3) 2-phenylhexahydroimidazo[1,5-a]pyrazin-3(2H)-one hydrochloride

A solution of tert-butyl 3-oxo-2-phenylhexahydroimidazo[1,5-a]pyrazine-7(1H)-carboxylate (600 mg, 1.89 mmol) and 2N hydrogen chloride-methanol solution (20 ml) was stirred at room temperature for 4 hr, and the solvent was evaporated under reduced pressure. The residue was recrystallized from a mixed solvent of methanol and ether to give the object product (340 mg, 71.1%) as a solid.
¹H-NMR (CDCl₃) δ; 2.84 - 2.97 (2H, m), 3.23 - 3.40 (3H, m), 3.57 - 3.61 (1H, m), 3.85 - 4.00 (2H, m), 4.08 - 4.15 (1H, m), 7.00 - 7.05 (1H, m), 7.30 - 7.36 (2H, m), 7.54 - 7.57 (2H, m), 9.53 (2H, br s).

### Example 2

### 2-(3-bromophenyl)hexahydroimidazo[1,5-a]pyrazin-3(2H)-one hydrochloride

### (1) tert-butyl 4-[(3-bromophenyl)carbamoyl]-3-(hydroxymethyl)piperazine-1-carboxylate

To a solution of tert-butyl 3-(hydroxymethyl)piperazine-1-carboxylate (1.00 g, 4.62 mmol) in tetrahydrofuran (20 ml) was added 3-bromophenyl isocyanate (0.692 ml, 5.54 mmol) under ice-cooling, and the mixture was stirred at room temperature for 4 hr. The solvent was evaporated under reduced pressure. The residue was recrystallized from a mixed solvent of hexane and ethyl acetate to give the object product (1.67 g, 87.4%) as a solid.
¹H-NMR (CDCl₃) δ; 1.46 (9H, s), 1.65 (1H, br s), 3.05 - 3.23 (3H, m), 3.52 - 3.85 (4H, m), 3.98 - 4.08 (2H, m), 7.11 - 7.21 (3H, m), 7.52 (1H, s), 7.87 (1H, br s).

### (2) tert-butyl 2-(3-bromophenyl)-3-oxohexahydroimidazo[1,5-a]pyrazine-7(1H)-carboxylate

To a solution of tert-butyl 4-[(3-bromophenyl)carbamoyl]-3-(hydroxymethyl)piperazine-1-carboxylate (1.60 g, 3.87 mmol) and triphenylphosphine (2.64 g, 10.1 mmol) in N,N-dimethylformamide (32 ml) was added 40% diethyl azodicarboxylate-toluene solution (4.35 g, 10.1 mmol) at room temperature, and the mixture was stirred under a nitrogen atmosphere at room temperature for 5 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (1.50 g, 98.0%) as a solid.
¹H-NMR (CDCl₃) δ; 1.49 (9H, s), 2.67 - 3.00 (3H, m), 3.38 - 3.43 (1H, m), 3.69 - 3.77 (1H, m), 3.87 - 3.93 (2H, m), 4.11 - 4.24 (2H, m), 7.14 - 7.21 (2H, m), 7.51 - 7.55 (1H, m), 7.70 (1H, br s).

### (3) 2-(3-bromophenyl)hexahydroimidazo[1,5-a]pyrazin-3(2H)-one hydrochloride

A mixture of tert-butyl 2-(3-bromophenyl)-3-oxohexahydroimidazo[1,5-a]pyrazine-7(1H)-carboxylate (220 mg, 0.555 mmol) and 4N hydrogen chloride-ethyl acetate (10 ml) solution was stirred at room temperature for 30 min, and the solvent was evaporated under reduced pressure. The residue was recrystallized from a mixed solvent of methanol and ether to give the object product (162 mg, 87.6%) as a solid.
¹H-NMR (DMSO-d₆) δ; 2.92 (2H, br s), 3.16 - 3.47 (3H, m), 3.58 - 3.62 (1H, m), 3.85 - 4.15 (3H, m), 7.19 - 7.48 (3H, m), 7.89 (1H, s), 9.49 (2H, br s).

### Example 3

### 2-(3-chlorophenyl)hexahydroimidazo[1,5-a]pyrazin-3(2H)-one hydrochloride

### (1) tert-butyl 4-[(3-chlorophenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 3-chlorophenyl isocyanate (0.254 ml, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (400 mg, 77.8%) as a solid.
¹H-NMR (CDCl₃) δ; 1.47 (9H, s), 3.08 - 3.23 (4H, m), 3.70 - 4.13 (6H, m), 6.95- 6.99 (1H, m), 7.11 - 7.20 (2H, m), 7.37 (1H, s), 7.80 (1H, br s).

### (2) tert-butyl 2-(3-chlorophenyl)-3-oxohexahydroimidazo[1,5-a]pyrazine-7(1H)-carboxylate

To a solution of tert-butyl 4-[(3-chlorophenyl)carbamoyl]-3-(hydroxymethyl)piperazine-1-carboxylate (370 mg, 1.00 mmol) and triphenylphosphine (526 mg, 2.00 mmol) in N,N-dimethylformamide (7 ml) was added 40% diethyl azodicarboxylate-toluene solution (870 mg, 2.00 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (350 mg, 99.7%) as a solid.
¹H-NMR (CDCl₃) δ; 1.49 (9H, s), 2.67 - 3.00 (3H, m), 3.39 - 3.44 (1H, m), 3.70 - 3.78 (1H, m), 3.88 - 3.94 (2H, m), 4.08 - 4.28 (2H, m), 6.99 - 7.02 (1H, m), 7.21 - 7.27 (1H, m), 7.45 - 7.48 (1H, m), 7.57 (1H, br s).

### (3) 2-(3-chlorophenyl)hexahydroimidazo[1,5-a]pyrazin-3(2H)-one hydrochloride

A mixture of tert-butyl 2-(3-chlorophenyl)-3-oxohexahydroimidazo[1,5-a]pyrazine-7(1H)-carboxylate (330 mg, 0.938 mmol) and 4N hydrogen chloride-ethyl acetate (10 ml) solution was stirred at room temperature for 12 hr, and the solvent was evaporated under reduced pressure. The residue was recrystallized from a mixed solvent of methanol and ether to give the object product (208 mg, 77.0%) as a solid.
¹H-NMR (DMSO-d₆) δ; 2.50 (2H, br s), 3.22 - 3.63 (4H, m), 3.85 - 4.16 (3H, m), 7.07 (1H, d, J = 6.9 Hz), 7.33 - 7.44 (2H, m), 7.75 (1H, s), 9.48 (2H, br s).

### Example 4

### 2-(2-chlorophenyl)hexahydroimidazo[1,5-a]pyrazin-3(2H)-one hydrochloride

### (1) tert-butyl 4-[(2-chlorophenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 2-chlorophenyl isocyanate (0.692 ml, 5.54 mmol) under ice-cooling, and the mixture was stirred at room temperature for 2 hr. The solvent was evaporated under reduced pressure. The residue was purified by silica gel column chromatography (hexane:ethyl acetate= 1:1) to give the object product (1.67 g, 87.4%) as an oil.
¹H-NMR (CDCl₃) δ; 1.45 (9H, s), 2.97 - 3.14 (3H, m), 3.59 - 4.03 (6H, m), 4.39 (1H, br s), 6.88 - 6.94 (1H, m), 7.15 - 7.21 (1H, m), 7.29 (1H, d, J = 9.3 Hz), 7.88 (1H, d, J = 8.1 Hz), 8:09 (1H, br s).

### (2) tert-butyl 2-(2-chlorophenyl)-3-oxohexahydroimidazo[1,5-a]pyrazine-7(1H)-carboxylate

To a solution of tert-butyl 4-[(2-chlorophenyl)carbamoyl]-3-(hydroxymethyl)piperazine-1-carboxylate (450 mg, 1.22 mmol) and triphenylphosphine (639 mg, 2.44 mmol) in N,N-dimethylformamide (7 ml) was added 40% diethyl azodicarboxylate-toluene solution (1.06 g, 2.44 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (380 mg, 88.6%) as a solid.
¹H-NMR (CDCl₃) δ; 1.49 (9H, s), 2.87 - 3.03 (3H, m), 3.46 - 3.49 (1H, m), 3.74 - 3.93 (3H, m), 4.09 - 4.27 (2H, m), 7.20 - 7.45 (4H, m).

### (3) 2-(2-chlorophenyl)hexahydroimidazo[1,5-a]pyrazin-3(2H)-one hydrochloride

A mixture of tert-butyl 2-(2-chlorophenyl)-3-oxohexahydroimidazo[1,5-a]pyrazine-7(1H)-carboxylate (350 mg, 0.996 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (200 mg, 69.7%) as a solid.
¹H-NMR (DMSO-d₆) δ; 2.50 - 3.00 (2H, m), 3. 17 - 3.51 (4H, m), 3.80 - 3.87 (2H, m), 4.10 - 4.17 (1H, m), 7.32 - 7.56 (4H, m), 9.49 (2H, br s).

### Example 5

### 2-(4-chlorophenyl)hexahydroimidazo[1,5-a]pyrazin-3(2H)-one hydrochloride

### (1) tert-butyl 4-[(4-chlorophenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 4-chlorophenyl isocyanate (0.267 ml, 2.09 mmol) under ice-cooling, and the mixture was stirred at room temperature for 2 hr. The solvent was evaporated under reduced pressure. The residue was recrystallized from a mixed solvent of hexane and ethyl acetate to give the object product (490 mg, 95.3%) as a solid.
¹H-NMR (CDCl₃) δ; 1.41 (9H, s), 2.94 - 3.02 (3H, m), 3.44 (2H, br s), 3.85 - 3.97 (3H, m), 4.15 (1H, br s), 4.94 (1H, br s), 7.26 (2H, d, J = 8.7 Hz), 7.45 (2H, d, J = 8.7 Hz), 8.60 (1H, s).

### (2) tert-butyl 2-(4-chlorophenyl)-3-oxohexahydroimidazo[1,5-a]pyrazine-7(1H)-carboxylate

To a solution of tert-butyl 4-[(4-chlorophenyl)carbamoyl]-3-(hydroxymethyl)piperazine-1-carboxylate (450 mg, 1.22 mmol) and triphenylphosphine (639 mg, 2.44 mmol) in N,N-dimethylformamide (7 ml) was added 40% diethyl azodicarboxylate-toluene solution (1.06 g, 2.44 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product 400 mg, 93.2%) as an oil.
¹H-NMR (CDCl₃) δ; 1.49 (9H, s), 2.62 - 3.01 (3H, m), 3.38 - 3.43 (1H, m), 3.69 - 3.99 (1H, m), 3.90 - 3.94 (2H, m), 4.17 - 4.33 (2H, m), 7.28 (2H, d, J = 9.0 Hz), 7.48 (2H, d, J = 9.0 Hz).

### (3) 2-(4-chlorophenyl)hexahydroimidazo[1,5-a]pyrazin-3(2H)-one hydrochloride

A mixture of tert-butyl 2-(4-chlorophenyl)-3-oxohexahydroimidazo[1,5-a]pyrazine-7(1H)-carboxylate (390 mg, 1.11 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (149 mg, 46.7%) as a solid.
¹H-NMR (DMSO-d₆) δ; 2.91 - 2.98 (2H, m), 3.16 - 3.41 (3H, m), 3.56 - 3.61 (1H, m), 3.85 - 3.99 (2H, m), 4.10 (1H, br s), 7.39 (2H, d, J = 8.7 Hz), 7.58 (2H, d, J = 8.7 Hz), 9.44 (2H, br s).

### Example 6

### 2-(3,4-dichlorophenyl)hexahydroimidazo[1,5-a]pyrazin-3(2H)-one hydrochloride

### (1) tert-butyl 4-[(3,4-dichlorophenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 3,4-dichlorophenyl isocyanate (392 mg, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (370 mg, 65.8%) as a solid.
¹H-NMR (CDCl₃) δ; 1.47 (9H, s), 3.04 - 3.26 (5H, m), 3.71 - 3.89 (3H, m), 4.03 - 4.13 (2H, m), 7.10 (1H, dd, J = 8.7, 2.7 Hz), 7.29 (1H, d, J = 8.7 Hz), 7.48 (1H, d, J = 2.7 Hz), 7.83 (1H, br s).

### (2) tert-butyl 2-(3,4-dichlorophenyl)-3-oxohexahydroimidazo[1,5-a]pyrazine-7(1H)-carboxylate

To a solution of tert-butyl 4-[(3,4-dichlorophenyl)carbamoyl]-3-(hydroxymethyl)piperazine-1-carboxylate (350 mg, 0.866 mmol) and triphenylphosphine (454 mg, 1.73 mmol) in N,N-dimethylformamide (7 ml) was added 40% diethyl azodicarboxylate-toluene solution (753 mg, 1.73 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (320 mg, 95.5%) as an oil.
¹H-NMR (CDCl₃) δ; 1.49 (9H, s), 2.65 - 3.01 (3H, m), 3.37 - 3.42 (1H, m), 3.69 - 3.79 (1H, m), 3.86 - 4.00 (2H, m), 4.08 - 4.52 (2H, m), 7.36 (1H, d, J = 8.7 Hz), 7.46 (1H, dd, J = 8.7, 2.7 Hz), 7.65 (1H, d, J = 2.7 Hz).

### (3) 2-(3,4-dichlorophenyl)hexahydroimidazo[1,5-a]pyrazin-3(2H)-one hydrochloride

A mixture of tert-butyl 2-(3,4-dichlorophenyl)-3-oxohexahydroimidazo[1,5-a]pyrazine-7(1H)-carboxylate (310 mg, 0.803 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (139 mg, 53.7%) as a solid.
¹H-NMR (DMSO-d₆) δ; 2.86 - 2.99 (2H, m), 3.16 - 3.40 (3H, m), 3.58 - 3.63 (1H, m), 3.85 - 4.02 (2H, m), 4.07 - 4.15 (1H, m), 7.50 (1H, dd, J = 9.0, 2.4 Hz), 7.59 (1H, d, J = 9.0 Hz), 7.89 (1H, d, J = 2.4 Hz), 9.47 (2H, br s).

### Example 7

### 2-(3-fluorophenyl)hexahydroimidazo[1,5-a]pyrazin-3(2H)-one hydrochloride

### (1) tert-butyl 4-[(3-fluorophenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 3-fluorophenyl isocyanate (287 mg, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (410 mg, 83.5%) as a solid.
¹H-NMR (CDCl₃) δ; 1.46 (9H, s), 3.02 - 3.23 (3H, m), 3.40 (1H, br s), 3.66 - 3.85 (4H, m), 3.99 - 4.13 (2H, m), 6.66 - 6.72 (1H, m), 6.91 - 6.95 (1H, m), 7.15 - 7.22 (2H, m), 7.86 (1H, br s).

### (2) tert-butyl 2-(3-fluorophenyl)-3-oxohexahydroimidazo[1,5-a]pyrazine-7(1H)-carboxylate

To a solution of tert-butyl 4-[(3-fluorophenyl)carbamoyl]-3-(hydroxymethyl)piperazine-1-carboxylate (380 mg, 10.8 mmol) and triphenylphosphine (567 mg, 2.16 mmol) in N,N-dimethylformamide (8 ml) was added 40% diethyl azodicarboxylate-toluene solution (940 mg, 2.16 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (350 mg, 96.7%) as a solid.
¹H-NMR (CDCl₃) δ; 1.49 (9H, s), 2.62 - 3.01 (3H, m), 3.39 - 3.44 (1H, m), 3.72 - 3.76 (1H, m), 3.88 - 3.94 (2H, m), 4.10 - 4.36 (2H, m), 6.71- 6.77 (1H, m), 7.20 - 7.29 (2H, m), 7.44 - 7.48 (1H, m).

### (3) 2-(3-fluorophenyl)hexahydroimidazo[1,5-a]pyrazin-3(2H)-one hydrochloride

A mixture of tert-butyl 2-(3-fluorophenyl)-3-oxohexahydroimidazo[1,5-a]pyrazine-7(1H)-carboxylate (355 mg, 1.06 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (232 mg, 80.6%) as a solid.
¹H-NMR (DMSO-d₆) δ; 2.92 (2H, br s), 3.16 - 3.62 (4H, m), 3.85 - 4.01 (2H, m), 4.13 (1H, br s), 6.82 - 6.87 (1H, m), 7.24 - 7.41 (2H, m), 7.53 - 7.58 (1H, m), 9.51 (2H, br s).

### Example 8

### 2-[3-(trifluoromethyl)phenyl]hexahydroimidazo[1,5-a]pyrazin-3(2H)-one hydrochloride

### (1) tert-butyl 3-(hydroxymethyl)-4-{[3-(trifluoromethyl)phenyl]carbamoyl}piperazine-1-carboxylate

To a solution of tert-butyl 3-(hydroxymethyl)piperazine-1-carboxylate (300 mg, 1.39 mmol) in tetrahydrofuran (6 ml) was added 3-(trifluoromethyl)phenyl isocyanate (287 mg, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (390 mg, 69.6%) as a solid.
¹H-NMR (CDCl₃) δ; 1.46 (9H, s), 3.09 - 3.23 (4H, m), 3.69 - 3.88 (4H, m), 4.01 - 4.15 (2H, m), 7.24 (1H, d, J = 8.1 Hz), 7.33 - 7.39 (1H, m), 7.46 (1H, d, J = 8.7 Hz), 7.58 (1H, s), 7.97 (1H, br s).

### (2) tert-butyl 3-oxo-2-[3-(trifluoromethyl)phenyl]hexahydroimidazo[1,5-a]pyrazine-7(1H)-carboxylate

To a solution of tert-butyl 3-(hydroxymethyl)-4-{[3-(trifluoromethyl)phenyl]carbamoyl}piperazine-1-carboxylate (370 mg, 0.917 mmol) and triphenylphosphine (481 mg, 1.83 mmol) in N,N-dimethylformamide (8 ml) was added 40% diethyl azodicarboxylate-toluene solution (940 mg, 2.16 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (350 mg, 99.2%) as a solid.
¹H-NMR (CDCl₃) δ; 1.49 (9H, s), 2.68 - 3.02 (3H, m), 3.44 - 3.49 (1H, m), 3.72 - 3.81 (1H, m), 3.91 - 4.00 (2H, m), 4.11 - 4.36 (2H, m), 7.28 (1H, d, J = 8.4 Hz), 7.41 - 7.46 (1H, m), 7.56 (1H, s), 7.82 (1H, d, J = 8.7 Hz).

### (3) 2-[3-(trifluoromethyl)phenyl]hexahydroimidazo[1,5-a]pyrazin-3(2H)-one hydrochloride

A mixture of tert-butyl 3-oxo-2-[3-(trifluoromethyl)phenyl]hexahydroimidazo[1,5-a]pyrazine-7(1H)-carboxylate (330 mg, 0.856 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (216 mg, 78.5%) as a solid.
¹H-NMR (DMSO-d₆) δ; 2.96 (2H, br s), 3.24 - 3.46 (3H, m), 3.64 - 3.69 (1H, m), 3.88 - 3.92 (1H, m), 4.01 - 4.15 (2H, m), 7.36 (1H, d, J = 7.2 Hz), 7.55 - 7.60 (1H, m), 7.70 (1H, d, J = 8.7 Hz), 8.08 (1H, s), 9.50 (2H, br s).

### Example 9

### 2-(3-methylphenyl)hexahydroimidazo[1,5-a]pyrazin-3(2H)-one hydrochloride

### (1) tert-butyl 3-(hydroxymethyl)-4-[(3-methylphenyl)carbamoyl]piperazine-1-carboxylate

To a solution of tert-butyl 3-(hydroxymethyl)piperazine-1-carboxylate (300 mg, 1.39 mmol) in tetrahydrofuran (6 ml) was added 3-methylphenyl isocyanate (0.269 ml, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (400 mg, 82.3%) as a solid.
¹H-NMR (CDCl₃) δ; 1.46 (9H, s), 2.31 (3H, s), 3.05 - 3.19 (3H, m), 3.39 (1H, br s), 3.65 - 4.08 (6H, m), 6.82 (1H, d, J = 7.2 Hz), 7.05 - 7.17 (3H, m), 7.49 (1H, br s).

### (2) tert-butyl 2-(3-methylphenyl)-3-oxohexahydroimidazo[1,5-a]pyrazine-7(1H)-carboxylate

To a solution of t-butyl 3-(hydroxymethyl)-4-[(3-methylphenyl)carbamoyl]piperazine-1-carboxylate (380 mg, 1.09 mmol) and triphenylphosphine (570 mg, 2.18 mmol) in N,N-dimethylformamide (8 ml) was added 40% diethyl azodicarboxylate-toluene solution (949 mg, 2.18 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (350 mg, 97.0%) as a solid.
¹H-NMR (CDCl₃) δ; 1.49 (9H, s), 2.35 (3H, s), 2.67 - 2.99 (3H, m), 3.40 - 3.45 (1H, m), 3.67 - 3.76 (1H, m), 3.89 - 3.94 (2H, m), 4.08 - 4.31 (2H, m), 6.86 (1H, d, J = 6.3 Hz), 7.18 - 7.24 (1H, m), 7.31 (1H, d, J = 8.4 Hz) 7.40 (1H, s).

### (3) 2-(3-methylphenyl)hexahydroimidazo[1,5-a]pyrazin-3(2H)-one hydrochloride

A mixture of tert-butyl 2-(3-methylphenyl)-3-oxohexahydroimidazo[1,5-a]pyrazine-7(1H)-carboxylate (330 mg, 0.997 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (183 mg, 68.5%) as a solid.
¹H-NMR (DMSO-d₆) δ; 2.29 (3H, s), 2.91 (2H, br s), 3.19 - 3.28 (2H, m), 3.34 - 3.43 (1H, m), 3.55 - 3.60 (1H, m), 3.84 - 3.98 (2H, m), 4.05 - 4.12 (1H, m), 6.84 (1H, d, J = 7.5 Hz), 7.18 - 7.23 (1H, m), 7.36 - 7.39 (2H, m), 9.45 (2H, br s).

### Example 10

### 2-(3-methoxyphenyl)hexahydroimidazo[1,5-a]pyrazin-3(2H)-one hydrochloride

### (1) tert-butyl 3-(hydroxymethyl)-4-[(3-methoxyphenyl)carbamoyl]piperazine-1-carboxylate

To a solution of tert-butyl 3-(hydroxymethyl)piperazine-1-carboxylate (300 mg, 1.39 mmol) in tetrahydrofuran (6 ml) was added 3-methoxyphenyl isocyanate (0.274 ml, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (460 mg, 93.7%) as a solid.
¹H-NMR (CDCl₃) δ; 1.46 (9H, s), 3.05 - 3.20 (3H, m), 3.45 (1H, br s), 3.66 - 4.15 (9H, m), 6.55 - 6.58 (1H, m), 6.78 (1H, d, J = 7.8 Hz), 7.00 (1H, s), 7.12 - 7.18 (1H, m), 7.64 (1H, br s).

### (2) tert-butyl 2-(3-methoxyphenyl)-3-oxohexahydroimidazo[1,5-a]pyrazine-7(1H)-carboxylate

To a solution of tert-butyl 3-(hydroxymethyl)-4-[(3-methoxyphenyl)carbamoyl]piperazine-1-carboxylate (440 mg, 1.25 mmol) and triphenylphosphine (653 mg, 2.49 mmol) in N,N-dimethylformamide (8 ml) was added 40% diethyl azodicarboxylate-toluene solution (1.08 g, 2.49 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (430 mg, 99.1%) as a solid.
¹H-NMR (CDCl₃) δ; 1.49 (9H, s), 2.67 - 3.04 (3H, m), 3.39 - 3.44 (1H, m), 3.67 - 3.73 (1H, m), 3.81 (3H, s), 3.88 - 3.96 (2H, m), 4.05 - 4.30 (2H, m), 6.57 - 6.64 (1H, m), 6.98 - 7.01 (1H, m), 7.19 - 7.26 (1H, m), 7.34 (1H, br s).

### (3) 2-(3-methoxyphenyl)hexahydroimidazo[1,5-a]pyrazin-3(2H)-one hydrochloride

A mixture of tert-butyl 2-(3-methoxyphenyl)-3-oxohexahydroimidazo[1,5-a]pyrazine-7(1H)-carboxylate (410 mg, 1.18 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (216 mg, 64.5%) as a solid.
¹H-NMR (DMSO-d₆) δ; 2.92 (2H, br s), 3.19 - 3.27 (2H, m), 3.39 (1H, br s), 3.56 - 3.61 (1H, m), 3.73 (3H, s), 3.85 - 3.98 (2H, m), 4.06 (1H, br s), 6.60 - 6.64 (1H, m), 7.06 (1H, d, J = 9.0 Hz), 7.21 - 7.26 (2H, m), 9.43 (2H, br s).

### Example 11

### 3-(3-oxohexahydroimidazo[1,5-a]pyrazin-2(3H)-yl)benzonitrile hydrochloride

### (1) tert-butyl 4-[(3-cyanophenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 3-cyanophenyl isocyanate (301 mg, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (363 mg, 72.6%) as a solid.
¹H-NMR (CDCl₃) δ; 1.47 (9H, s), 3.09 - 3.29 (5H, m), 3.76 - 3.91 (3H, m), 4.09 (2H, br s), 7.26 - 7.37 (2H, m), 7.48 - 7.52 (1H, m), 7.63 (1H, s), 8.02 (1H, br s).

### (2) tert-butyl 2-(3-cyanophenyl)-3-oxohexahydroimidazo[1,5-a]pyrazine-7(1H)-carboxylate

To a solution of tert-butyl 4-[(3-cyanophenyl)carbamoyl]-3-(hydroxymethyl)piperazine-1-carboxylate (330 mg, 0.916 mmol) and triphenylphosphine (480 mg, 1.83 mmol) in N,N-dimethylformamide (6 ml) was added 40% diethyl azodicarboxylate-toluene solution (797 mg, 1.83 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (290 mg, 92.4%) as a solid.
¹H-NMR (CDCl₃) δ; 1.49 (9H, s), 2.67 - 3.02 (3H, m), 3.41 - 3.46 (1H, m), 3.74 - 3.82 (1H, m), 3.90 - 3.96 (2H, m), 4.08 - 4.27 (2H, m), 7.27 - 7.32 (1H, m), 7.39 - 7.44 (1H, m), 7.83 (2H, br s).

### (3) 3-(3-oxohexahydroimidazo[1,5-a]pyrazin-2(3H)-yl)benzonitrile hydrochloride

A mixture of tert-butyl 2-(3-cyanophenyl)-3-oxohexahydroimidazo[1,5-a]pyrazine-7(1H)-carboxylate (290 mg, 0.847 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (130 mg, 55.1%) as a solid.
¹H-NMR (DMSO-d₆) δ; 2. 87 - 3.00 (2H, m), 3.22 - 3.42 (3H, m), 3.61 - 3.66 (1H, m), 3.86 - 3.92 (1H, m), 3.99 - 4.05 (1H, m), 4.10 - 4.18 (1H, m), 7.46 - 7.58 (2H, m), 7.86 - 7.90 (1H, m), 7.99 - 8.00 (1H, m), 9.52 (2H, br s).

### Example 12

### 2-(3-ethylphenyl)hexahydroimidazo[1,5-a]pyrazin-3(2H)-one hydrochloride

### (1) tert-butyl 4-[(3-ethylphenyl)carbamoyl]-3-(hydroxymethyl)piperazine-1-carboxylate

To a solution of tert-butyl 3-(hydroxymethyl)piperazine-1-carboxylate (300 mg, 1.39 mmol) in tetrahydrofuran (6 ml) was added 3-ethylphenyl isocyanate (0.298 ml, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (410 mg, 81.1%) as a solid.
¹H-NMR (CDCl₃) δ; 1.22 (3H, t, J = 7.5 Hz), 1.46 (9H, s), 2.60 (2H, q, J = 7.5 Hz), 3.03 (2H, br s), 3.13 - 3.19 (1H, m), 3.40 (1H, br s), 3.63- 4.13 (6H, m), 6.85 (1H, d, J = 7.2 Hz), 7.08 (1H, d, J = 8.4 Hz), 7. 15 - 7.25 (2H, m), 7.50 (1H, br s).

### (2) tert-butyl 2-(3-ethylphenyl)-3-oxohexahydroimidazo[1,5-a]pyrazine-7(1H)-carboxylate

To a solution of tert-butyl 4-[(3-ethylphenyl)carbamoyl]-3-(hydroxymethyl)piperazine-1-carboxylate (363 mg, 1.00 mmol) and triphenylphosphine (525 mg, 2.00 mmol) in N,N-dimethylformamide (6 ml) was added 40% diethyl azodicarboxylate-toluene solution (870 mg, 2.00 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (hexane:ethyl acetate=1:1) to give the object product (190 mg, 55.1%) as a solid.
¹H-NMR (CDCl₃) δ; 1.24 (3H, t, J = 7.8 Hz), 1.44 (9H, s), 2.61 - 3.00 (5H, m), 3.42 - 3.46 (1H, m), 3.67 - 3.76 (1H, m), 3.90 - 3.96 (2H, m), 4.08 - 4.23 (2H, m), 6.99 (1H, d, J = 7.2 Hz), 7.21 - 7.32 (2H, m), 7.45 (1H, s).

### (3) 2-(3-ethylphenyl)hexahydroimidazo[1,5-a]pyrazin-3(2H)-one hydrochloride

A mixture of tert-butyl 2-(3-ethylphenyl)-3-oxohexahydroimidazo[1,5-a]pyrazine-7(1H)-carboxylate (180 mg, 0.521 mmol) and 4N hydrogen chloride-ethyl acetate (5 ml) solution was stirred at room temperature for 1 hr, and the solvent was evaporated under reduced pressure. The residue was recrystallized from a mixed solvent of methanol and ether to give the object product (136 mg, 92.5%) as a solid.
¹H-NMR (DMSO-d₆) δ; 1.17 (3H, t, J = 7.2 Hz), 2.58 (2H, q, J = 7.2 Hz), 2.85 - 2.96 (2H, m), 3.18 - 3.40 (3H, m), 3.57 - 3.61 (1H, m), 3.84 - 4.11 (3H, m), 6.88 (1H, d, J = 7.2 Hz), 7.20 - 7.25 (1H, m), 7.36 - 7.41 (2H, m), 9.36 (2H, br s).

### Example 13

### 2-(pyridin-3-yl)hexahydroimidazo[1,5-a]pyrazin-3(2H)-one dihydrochloride

### (1) tert-butyl 3-(hydroxymethyl)-4-[(pyridin-3-yl)carbamoyl]piperazine-1-carboxylate

To a solution of tert-butyl 3-(hydroxymethyl)piperazine-1-carboxylate (300 mg, 1.39 mmol) in tetrahydrofuran (6 ml) was added pyridine-3-isocyanate (251 mg, 2.09 mmol) under ice-cooling, and the mixture was stirred at room temperature for 1 hr. The solvent was evaporated under reduced pressure. The residue was recrystallized from a mixed solvent of hexane and ethyl acetate to give the object product (162 mg, 34.6%) as a solid.
¹H-NMR (CDCl₃) δ; 1.41 (9H, s), 2.90 - 3.05 (3H, m), 3.44 - 3.48 (2H, m), 3.87 - 4.02 (3H, m), 4.18 (1H, br s), 4.91 - 4.95 (1H, m), 7.24 - 7.28 (1H, m), 7.83 - 7.86 (1H, m), 8.13 - 8.15 (1H, m), 8.61 (1H, s), 8.66 (1H, s).

### (2) tert-butyl 3-oxo-2-(pyridin-3-yl)hexahydroimidazo[1,5-a]pyrazine-7(1H)-carboxylate

To a solution of tert-butyl 3-(hydroxymethyl)-4-[(pyridin-3-yl)carbamoyl]piperazine-1-carboxylate (155 mg, 0.461 mmol) and triphenylphosphine (242 mg, 0.922 mmol) in N,N-dimethylformamide (3 ml) was added 40% diethyl azodicarboxylate-toluene solution (401 mg, 0.922 mmol) at room temperature, and the mixture was stirred under a nitrogen atmosphere at room temperature for 12 hr. The reaction mixture was poured into water, and the mixture was extracted with ethyl acetate. The extract was washed with water, and dried over anhydrous magnesium sulfate. The residue was purified by silica gel column chromatography (ethyl acetate) to give the object product (80.0 mg, 54.4%) as a solid.
¹H-NMR (CDCl₃) δ; 1.49 (9H, s), 2.69 - 3.06 (3H, m), 3.44 - 3.49 (1H, m), 3.73 - 4.00 (3H, m), 4.10 (1H, br s), 4.29 (1H, br s), 7.24 - 7.28 (1H, m), 8.17 - 8.19 (1H, m), 8.29 - 8.30 (1H, m), 8.58 (1H, s).

### (3) 2-(pyridin-3-yl)hexahydroimidazo[1,5-a]pyrazin-3(2H)-one dihydrochloride

A mixture of tert-butyl 3-oxo-2-(pyridin-3-yl)hexahydroimidazo[1,5-a]pyrazine-7(1H)-carboxylate (80 mg, 0.251 mmol) and 4N hydrogen chloride-ethyl acetate (10 ml) solution was stirred at room temperature for 1 hr, and the solvent was evaporated under reduced pressure. The residue was recrystallized from a mixed solvent of methanol and ether to give the object product (65.7 mg, 90.0%) as a solid.
¹H-NMR (DMSO-d₆) δ; 2.29 - 3.00 (2H, m), 3.27 - 3.46 (4H, m), 3.68 - 3.72 (1H, m), 3.89 - 3.95 (1H, m), 4.06 - 4.12 (1H, m), 4.24 (1H, br s), 7.87 (1H, dd, J = 8.7, 5.1 Hz), 8.40 (1H, dd, J = 8.7, 2.4 Hz), 8.47 (1H, d, J = 5.1 Hz), 9.04 (1H, d, J = 2.4 Hz), 9.67 (2H, br s).

### Example 14

### 2-(3-acetylphenyl)hexahydroimidazo[1,5-a]pyrazin-3(2H)-one hydrochloride

The object product was synthesized in the same manner as in Example 1 and from 3-acetylphenyl isocyanate and tert-butyl 3-(hydroxymethyl)piperazine-1-carboxylate.

### (1) tert-butyl 4-[(3-acetylphenyl)carbamoyl]-3-(hydroxymethyl)piperazine-1-carboxylate

¹H-NMR (CDCl₃) δ; 1.47 (9H, s), 2.58 (3H, s), 3.17 - 3.07 (2H, m), 3.21 (1H, dd, J = 14.0, 4.4 Hz), 3.62 - 3.98 (4H, m), 4.02 - 4.18 (2H, m), 7.36 (1H, t, J = 8.0 Hz), 7.57 - 7.63 (2H, m), 7.78 (1H, s), 7.81 - 7.90 (1H, m).

### (2) tert-butyl 2-(3-acetylphenyl)-3-oxohexahydroimidazo[1,5-a]pyrazine-7(1H)-carboxylate

¹H-NMR (CDCl₃) δ; 1.49 (9H, s), 2.61 (3H, s), 2.62 - 2.77 (1H, m), 2.78 - 2.90 (1H, m), 2.91 - 3.02 (1H, m), 3.50 (1H, dd, J = 9.2, 5.2 Hz), 3.71 - 3.80 (1H, m), 3.91 - 4.02 (2H, m), 4.03 - 4.19 (1H, m), 4.21 - 4.40 (1H, m), 7.43 (1H, t, J = 8.0 Hz), 7.62 (1H, d, J = 7.6 Hz), 7.92 - 8.13 (2H, m).

### (3) 2-(3-acetylphenyl)hexahydroimidazo[1,5-a]pyrazin-3(2H)-one hydrochloride

¹H-NMR (DMSO-d₆) δ; 2.56 (3H, s), 2.82 - 3.00 (2H, m), 3.21 - 3.49 (3H, m), 3.65 (1H, dd, J = 9.6, 4.5 Hz), 3.84 - 3.92 (1H, m), 4.02 (1H, t, J = 9.0 Hz), 4.10 - 4.21 (1H, m), 7.44 - 7.51 (1H, m), 7.63 (1H, dd, J = 6.6, 1.2 Hz), 7.81 - 7.86 (1H, m), 8.06 (1H, t, J = 1.8 Hz), 9.66 (1H, br s),9.80 (1H, br s).

### Example 15

### 2-(3-benzylphenyl)hexahydroimidazo[1,5-a]pyrazin-3(2H)-one hydrochloride

The object product was synthesized in the same manner as in Example 1 and from 3-benzylphenyl isocyanate and tert-butyl 3-(hydroxymethyl)piperazine-1-carboxylate.

### (1) tert-butyl 4-[(3-benzylphenyl)carbamoyl]-3-(hydroxymethyl)piperazine-1-carboxylate

¹H-NMR (CDCl₃) δ; 1.46 (9H, s), 2.80 - 3.15 (3H, m), 3.51 - 3.77 (4H, m), 3.83 - 3.92 (3H, m), 3.96 - 4.08 (1H, m), 6.85 (1H, d, J = 7.2 Hz), 7.10 - 7.21 (6H, m), 7.21 - 7.30 (2H, m), 7.51 - 7.82 (1H, m).

### (2) tert-butyl 2-(3-benzylphenyl)-3-oxohexahydroimidazo[1,5-a]pyrazine-7(1H)-carboxylate

¹H-NMR (CDCl₃) δ; 1.48 (9H, s), 2.58 - 3.00 (3H, m), 3.37 - 3.43 (1H, m), 3.65 - 3.76 (1H, m), 3.85 - 3.95 (2H, m), 3.97 (2H, s), 4.02 - 4.32 (2H, m), 6.88 (1H, d, J = 7.5 Hz), 7.15 - 7.30 (6H, m), 7.34 (1H, d, J = 1.5 Hz),7.45 (1H, s).

### (3) 2-(3-benzylphenyl)hexahydroimidazo[1,5-a]pyrazin-3(2H)-one hydrochloride

¹H-NMR (DMSO-d₆) δ; 2.78 - 2.92 (2H, m), 3.18 - 3.37 (3H, m), 3.55 (1H, dd, J = 10.0, 4.4 Hz), 3.79 - 3.92 (4H, m), 4.08 - 4.18 (1H, m), 6.88 (1H, d, J = 7.2 Hz), 7.12 - 7.29 (6H, m), 7.30 - 7.36 (1H, m), 7.48 (1H, s), 9.65 - 9.70 (1H, m), 9.72 - 9.95 (1H, m).

### Example 16

### 2-{3-[(trifluoromethyl)thio]phenyl}hexahydroimidazo[1,5-a]pyrazin-3(2H)-one hydrochloride

The object product was synthesized in the same manner as in Example 1 and from 3-[(trifluoromethyl)thio]phenyl isocyanate and tert-butyl 3-(hydroxymethyl)piperazine-1-carboxylate.

### (1) tert-butyl 3-(hydroxymethyl)-4-({3-[(trifluoromethyl)thio]phenyl}carbamoyl)piperazine-1-carboxylate

¹H-NMR (CDCl₃) δ; 1.46 (9H, s), 2.96-3.18 (2H, m), 3.20 (1H, dd, J = 14.0, 4.4 Hz), 3.67 - 3.91 (4H, m), 3.96 - 4.14 (2H, m), 7.23 - 7.36 (2H, m), 7.43 (1H, d, J = 7.2 Hz), 7.60 (1H, s), 7.62 - 7.99 (1H, m).

### (2) tert-butyl 3-oxo-2-{3-[(trifluoromethyl)thio]phenyl}hexahydroimidazo[1,5-a]pyrazine-7(1H)-carboxylate

¹H-NMR (CDCl₃) δ; 1.49 (9H, s), 2.60 - 3.01 (3H, m), 3.45 (1H, dd, J = 9.2, 5.6 Hz), 3.61-3.79 (1H, m), 3.88 - 3.98 (2H, m), 4.00 - 4.40 (2H, m), 7.28 - 7.39 (2H, m),7.71 - 7.82 (2H, m).

### (3) 2-{3-[(trifluoromethyl)thio]phenyl}hexahydroimidazo[1,5-a]pyrazin-3(2H)-one hydrochloride

¹H-NMR (DMSO-d₆) δ; 2.81 - 2.96 (2H, m), 3.17 - 3.26 (3H, m), 3.56 -3.62 (1H, m), 3.80 - 3.87 (1H, m), 3.96 (1H, t, J = 9.2 Hz), 4.06 -4.15 (1H, m), 7.30 (1H, d, J = 7.6 Hz), 7.46 (1H, t, J = 8.0 Hz), 7.57 (1H, dd, J = 8.4, 1.2 Hz), 8.06 (1H, s),9.55 (2H, br s).

### Example 17

### ethyl 3-(3-oxohexahydroimidazo[1,5-a]pyrazin-2(3H)-yl)benzoate hydrochloride

The object product was synthesized in the same manner as in Example 1 and from 3-(ethoxycarbonyl)phenyl isocyanate and tert-butyl 3-(hydroxymethyl)piperazine-1-carboxylate.

### (1) tert-butyl 4-{[3-(ethoxycarbonyl)phenyl]carbamoyl}-3-(hydroxymethyl)piperazine-1-carboxylate

¹H-NMR (CDCl₃) δ; 1.37 (3H, t, J = 7.0 Hz),1.46 (9H, s), 3.02 - 3.13 (2H, m), 3.18 (1H, dd, J = 14.0, 4.4 Hz), 3.68 - 3.91 (4H, m), 3.97 - 4.15 (2H, m), 4.34 (2H, q, J = 7.2 Hz), 7.33 (1H, t, J = 8.0 Hz), 7.62 (1H, dd, J = 8.0, 1.2 Hz), 7.66 (1H, dd, J = 6.8, 1.2 Hz), 7.80 (1H, s), 7.83 - 7.92 (1H, m).

### (2) tert-butyl 2-[3-(ethoxycarbonyl)phenyl]-3-oxohexahydroimidazo[1,5-a]pyrazine-7(1H)-carboxylate

¹H-NMR (CDCl₃) δ; 1.39 (3H, t, J = 7.2 Hz), 1.48 (9H, s), 2.62 - 2.90 (2H, m), 2.91 - 3.01 (1H, m), 3.49 (1H, dd, J = 9.2, 5.2 Hz), 3.71 - 3.78 (1H, m), 3.89 - 4.00 (2H, m), 4.37 (2H, q, J = 7.2 Hz), 4.01 - 4.32 (2H, m), 7.40 (1H, t, J = 8.0 Hz), 7.72 (1H, d, J = 7.6 Hz), 7.90 (1H, s), 8.07 (1H, d, J = 7.6 Hz).

### (3) ethyl 3-(3-oxohexahydroimidazo[1,5-a]pyrazin-2(3H)-yl)benzoate hydrochloride

¹H-NMR (DMSO-d₆) δ; 1.30 (3H, t, J = 7.0 Hz), 2. 85 - 2.98 (2H, m), 3.20 - 3.30 (3H, m), 3.64 (1H, dd, J = 9.6, 4.4 Hz), 3.83 - 3.90 (1H, m), 4.02 (1H, t, J = 9.2 Hz), 4.10 - 4.18 (1H, m), 4.30 (2H, q, J = 6.8 Hz), 7.48 (1H, t, J = 8.0 Hz), 7.60 (1H, d, J = 7.6 Hz), 7.74 - 7.78 (1H, m), 8.19 (1H, s), 9.56 - 9.85 (2H, m).

### Example 18

### 2-(2,3-dihydro-1-benzofuran-5-yl)hexahydroimidazo[1,5-a]pyrazin-3(2H)-one trifluoroacetate

The object product was synthesized in the same manner as in Example 1 and from 5-isocyanato-2,3-dihydro-1-benzofuran and tert-butyl 3-(hydroxymethyl)piperazine-1-carboxylate, and the final object product was purified by preparative high performance liquid chromatography (HPLC; column: Fuji C18 (300x25); wavelength 220 nm; mobile phase: A acetonitrile (containing 0.1% trifluoroacetic acid); B water (containing 0.1% trifluoroacetic acid); flow rate: 25 mL/min).

### (1) tert-butyl 4-[(2,3-dihydro-1-benzofuran-5-yl)carbamoyl]-3-(hydroxymethyl)piperazine-1-carboxylate

¹H-NMR (CDCl₃) δ; 1.38 (9H, s), 2.82 - 3.12 (5H, m), 3.54 - 3.61 (1H, m), 3.62 - 3.94 (4H, m), 3.95 - 4.09 (1H, m), 4.46 (2H, t, J = 17.2 Hz), 6.60 (1H, d, J = 8.4 Hz), 6.79 (1H, d, J = 8.0 Hz), 7.16 (1H, s), 7.20 - 7.31 (1H, m).

### (2) tert-butyl 2-(2,3-dihydro-1-benzofuran-5-yl)-3-oxohexahydroimidazo[1,5-a]pyrazine-7(1H)-carboxylate

¹H-NMR (CDCl₃) δ; 1.48 (9H, s), 2.60 - 2.99 (3H, m), 3.14 - 3.24 (2H, m), 3.38 (1H, dd, J = 9.2, 5.2 Hz), 3.62 - 3.75 (1H, m), 3.83 - 3.92 (2H, m), 4.00 - 4.30 (2H, m), 4.51 - 4.58 (2H, m), 6.73 (1H, d, J = 8.4 Hz), 6.96 - 7.04 (1H, m),7.53 (1H, s).

### (3) 2-(2,3-dihydro-1-benzofuran-5-yl)hexahydroimidazo[1,5-a]pyrazin-3(2H)-one trifluoroacetate

¹H-NMR (DMSO-d₆) δ; 2.86 - 2.95 (2H, m), 3.13 - 3.20 (3H, m), 3.21 -3.29 (1H, m), 3.49 - 3.55 (1H, m), 3.82 - 3.91 (2H, m), 3.97 - 4.06 (2H, m), 4.49 (2H, t, J = 8.6 Hz), 6.79 (1H, d, J = 8.8 Hz), 7.16 (1H, d, J = 8.8 Hz), 7.44 (1H, s),9.37 (2H, br s).

### Example 19

### 2-(2,3-dihydro-1H-inden-5-yl)hexahydroimidazo[1,5-a]pyrazin-3(2H)-one trifluoroacetate

The object product was synthesized in the same manner as in Example 1 and from 5-isocyanato-2,3-dihydro-1H-indene and tert-butyl 3-(hydroxymethyl)piperazine-1-carboxylate, and the final object product was purified by preparative high performance liquid chromatography (HPLC; column: Fuji C18 (300x25); wavelength 220 nm; mobile phase: A acetonitrile (containing 0.1% trifluoroacetic acid); B water (containing 0.1% trifluoroacetic acid); flow rate: 25 mL/min).

### (1) tert-butyl 4-[(2,3-dihydro-1H-inden-5-yl)carbamoyl]-3-(hydroxymethyl)piperazine-1-carboxylate

¹H-NMR (CDCl₃) δ; 1.47 (9H, s), 2.00 - 2.10 (2H, m), 2.82 - 2.91 (4H, m), 2.91 - 3.12 (2H, m), 3.15 (1H, dd, J = 14.0, 4.4 Hz), 3.62 - 4.12 (6H, m), 6.98 (1H, dd, J = 8.0, 1.2 Hz), 7.10 (1H, d, J = 8.0 Hz), 7.21 (1H, s), 7.31 - 7.56 (1H, m).

### (2) tert-butyl 2-(2,3-dihydro-1H-inden-5-yl)-3-oxohexahydroimidazo[1,5-a]pyrazine-7(1H)-carboxylate

¹H-NMR (CDCl₃) δ; 1.49 (9H, s), 2.02 - 2.10 (2H, m), 2.60 - 2.76 (1H, m), 2.79 - 3.00 (6H, m), 3.38 - 3.45 (1H, m), 3.66 - 3.75 (1H, m), 3.91 (2H, t, J = 9.2 Hz), 4.00 - 4.30 (2H, m), 7.16 - 7.25 (2H, m),7.47 (1H, s).

### (3) 2-(2,3-dihydro-1H-inden-5-yl)hexahydroimidazo[1,5-a]pyrazin-3(2H)-one trifluoroacetate

¹H-NMR (DMSO-d₆) δ; 1.96 - 2.02 (2H, m), 2.77 - 2.85 (4H, m), 2.86 -2.96 (2H, m), 3.17 - 3.28 (2H, m), 3.55 (1H, dd, J = 9.6, 4.0 Hz), 3.83 - 3.95 (2H, m), 4.01 - 4.08 (2H, m), 7.15 (1H, d, J = 8.4 Hz), 7.28 (1H, dd, J = 8.0, 1.6 Hz), 7.41 (1H, s),9.41 (2H, br s).

### Example 20

### 2-[3-(1H-pyrrol-1-yl)phenyl]hexahydroimidazo[1,5-a]pyrazin-3(2H)-one trifluoroacetate

The object product was synthesized in the same manner as in Example 1 and from 1-(3-isocyanatophenyl)-1H-pyrrole and tert-butyl 3-(hydroxymethyl)piperazine-1-carboxylate, and the final object product was purified by preparative high performance liquid chromatography (HPLC; column: Fuji C18 (300x25); wavelength 220 nm; mobile phase: A acetonitrile (containing 0.1% trifluoroacetic acid); B water (containing 0.1% trifluoroacetic acid); flow rate: 25 mL/min).

### (1) tert-butyl 3-(hydroxymethyl)-4-{[3-(1H-pyrrol-1-yl)phenyl]carbamoyl}piperazine-1-carboxylate

¹H-NMR (CDCl₃: δ; 1.46 (9H, s), 3.00 - 3.12 (2H, m), 3.19 (1H, dd, J = 13.8, 4.5 Hz), 3.63 - 3.86 (4H, m), 3.97 - 4.13 (2H, m), 6.32 (2H, t, J = 2.1 Hz), 6.98 - 7.09 (4H, m), 7.22 - 7.29 (1H, m), 7.44 (1H, s), 7.82 - 8.01 (1H, m).

### (2) tert-butyl 3-oxo-2-[3-(1H-pyrrol-1-yl)phenyl]hexahydroimidazo[1,5-a]pyrazine-7(1H)-carboxylate

¹H-NMR (CDCl₃) δ; 1.49 (9H, s), 2.61 - 2.89 (2H, m), 2.91 - 2.99 (1H, m), 3.46 (1H, dd, J = 9.2, 5.2 Hz), 3.70 - 3.79 (1H, m), 3.89 - 3.98 (2H, m), 4.02 - 4.34 (2H, m), 6.33 (2H, t, J = 2.2 Hz), 7.06 - 7.12 (3H, m), 7.25 - 7.30 (1H, m), 7.36 (1H, t, J = 8.2 Hz),7.80 (1H, s).

### (3) 2-[3-(1H-pyrrol-1-yl)phenyl]hexahydroimidazo[1,5-a]pyrazin-3(2H)-one trifluoroacetate

¹H-NMR (DMSO-d₆) δ; 2.88 - 3.02 (2H, m), 3.17 - 3.32 (2H, m), 3.38 - 3.44 (1H, m), 3.63 - 3.72 (1H, m), 3.90 (1H, dd, J = 13.6, 3.6 Hz), 4.01 - 4.11 (2H, m), 6.26 (2H, t, J = 2.2 Hz), 7.19 - 7.22 (1H, m), 7.30 (2H, t, J = 2.2 Hz), 7.73 (1H, t, J = 3.6 Hz), 7.37 - 7.45 (2H, m), 9.32 (2H, br s,).

### Example 21

### 2-(1-benzothien-5-yl)hexahydroimidazo[1,5-a]pyrazin-3(2H)-one trifluoroacetate

The object product was synthesized in the same manner as in Example 1 and from 5-isocyanato-1-benzothiophene and tert-butyl 3-(hydroxymethyl)piperazine-1-carboxylate, and the final object product was purified by preparative high performance liquid chromatography (HPLC; column: Fuji C18 (300x25); wavelength 220 nm; mobile phase: A acetonitrile (containing 0.1% trifluoroacetic acid); B water (containing 0.1% trifluoroacetic acid); flow rate: 25 mL/min).

### (1) tert-butyl 4-[(1-benzothien-5-yl)carbamoyl]-3-(hydroxymethyl)piperazine-1-carboxylate

¹H-NMR (CDCl₃) δ; 1.43 (9H, s), 2.91 - 3.08 (2H, m), 3.13 (1H, dd, J = 14.0, 4.4 Hz), 3.54 - 3.80 (4H, m), 3.92 - 4.11 (2H, m), 7.12 - 7.21 (2H, m), 7.40 (1H, d, J = 5.2 Hz), 7.70 (1H, d, J = 8.4 Hz), 7.79 (1H, s),7.79 (1H, br s,).

### (2) tert-butyl 2-(1-benzothien-5-yl)-3-oxohexahydroimidazo[1,5-a]pyrazine-7(1H)-carboxylate

¹H-NMR (CDCl₃) δ; 1.49 (9H, s), 2.65 - 3.02 (3H, m), 3.51 (1H, dd, J = 9.2, 5.2 Hz), 3.71 - 3.80 (1H, m), 3. 91 - 4.02 (2H, m), 4.03 - 4.35 (2H, m), 7.28 (1H, d, J = 5.6 Hz), 7.44 (1H, d, J = 5.2 Hz), 7.69 (1H, dd, J = 8.8, 1.6 Hz),7.87 (1H, d, J = 2.0 Hz), 7.81 (1H, d, J = 8.8 Hz).

### (3) 2-(1-benzothien-5-yl)hexahydroimidazo[1,5-a]pyrazin-3(2H)-one trifluoroacetate

¹H-NMR (DMSO-d₆) δ; 2.90 - 3.02 (2H, m), 3.18 - 3.30 (2H, m), 3.37 - 3.43 (1H, m), 3.64 - 3.70 (1H, m), 3.91 (1H, dd, J = 14.0, 3.6 Hz), 4.00 - 4.12 (2H, m), 7.40 (1H, d, J = 5.6 Hz), 7.72 - 7.77 (2H, m), 7.88 - 7.95 (2H, m),9.35 (2H, br s,).

### Example 22

### 2-(biphenyl-3-yl)hexahydroimidazo[1,5-a]pyrazin-3(2H)-one trifluoroacetate

### (1) tert-butyl 2-(biphenyl-3-yl)-3-oxohexahydroimidazo[1,5-a]pyrazine-7(1H)-carboxylate

A mixture of tert-butyl 2-(3-bromophenyl)-3-oxohexahydroimidazo[1,5-a]pyrazine-7(1H)-carboxylate (400 mg, 1.00 mmol), phenylboronic acid (148 mg, 1.20 mmol), potassium carbonate (415 mg, 3.00 mmol), bis(triphenylphosphine)palladium(II) dichloride (35.1 mg, 0.05 mmol), 1,4-dioxane (4 ml) and water (4 ml) was stirred at 110°C for 5 hr under a nitrogen atmosphere, and the reaction mixture was poured into water. The mixture was extracted with ethyl acetate. The extract was washed with water, and dried over anhydrous magnesium sulfate. The solvent was evaporated under reduced pressure, and the residue was purified by silica gel column chromatography (hexane:ethyl acetate=1:1) to give the object product (316 mg, 79.0%) as an oil.
¹H-NMR (CDCl₃) δ; 1.43 (9H, s), 2.55 - 2.97 (3H, m), 3.44 (1H, dd, J = 9.2, 5.2 Hz), 3.66 - 3.74 (1H, m), 3.89 - 3.97 (2H, m), 3.98 - 4.31 (2H, m), 7.21 - 7.41 (4H, m), 7.47 - 7.51 (1H, m), 7.53 - 7.57 (2H, m), 7.58 - 7.65 (1H, m),7.75 (1H, s).

### (2) 2-(biphenyl-3-yl)hexahydroimidazo[1,5-a]pyrazin-3(2H)-one trifluoroacetate

The object product was synthesized in the same manner as in Example 3 (3) and from tert-butyl 2-(biphenyl-3-yl)-3-oxohexahydroimidazo[1,5-a]pyrazine-7(1H)-carboxylate, and the final object product was purified by preparative high performance liquid chromatography (HPLC; column: Fuji C18 (300x25); wavelength 220 nm; mobile phase: A acetonitrile (containing 0.1% trifluoroacetic acid); B water (containing 0.1% trifluoroacetic acid); flow rate: 25 mL/min).
¹H-NMR (DMSO-d₆) δ; 2.98 - 3.10 (2H, m), 3.26 - 3.38 (2H, m), 3.46 -3.52 (1H, m), 3.76 - 3.81 (1H, m), 3.99 (1H, dd, J = 13.6, 3.6 Hz), 4.11 - 4.20 (2H, m), 7.37 - 7.56 (5H, m), 7.66 (1H, dd, J = 8.4, 1.6 Hz), 7.69 - 7.72 (2H, m), 7.91.(1H, d, J = 2.0 Hz),9.38 (2H, br s).

### Example 23

### 2-(4-fluoro-3-methylphenyl)hexahydroimidazo[1,5-a]pyrazin-3(2H)-one hydrochloride

The object product was synthesized in the same manner as in Example 1 and from 4-fluoro-3-methylphenyl isocyanate and tert-butyl 3-(hydroxymethyl)piperazine-1-carboxylate.

### (1) tert-butyl 4-[(4-fluoro-3-methylphenyl)carbamoyl]-3-(hydroxymethyl)piperazine-1-carboxylate

¹H-NMR (CDCl₃) δ; 1.39 (9H, s), 2.14 (3H, s), 2.85 - 3.14 (2H, m), 3.07 (1H, dd, J = 14.0, 4.8 Hz), 3.51 - 3.60 (1H, m), 3.61 - 3.69 (1H, m), 3.70 - 3.78 (1H, m), 3.79 - 3.92 (2H, m), 3.93 - 4.00 (1H, m), 6.81 - 6.86 (1H, m), 6.94 - 7.00 (1H, m), 7.02 - 7.07 (1H, m), 7.39 - 7.65 (1H, m).

### (2) tert-butyl 2-(4-fluoro-3-methylphenyl)-3-oxohexahydroimidazo[1,5-a]pyrazine-7(1H)-carboxylate

¹H-NMR (CDCl₃) δ; 1.48 (9H, s), 2.27 (3H, s), 2.62 - 2.87 (2H, m), 2.88 - 2.98 (1H, m), 3.36 - 3.43 (1H, m), 3.65 - 3.74 (1H, m), 3.84 - 3.94 (2H, m), 3.95 - 4.31 (2H, m), 6.88 - 6.99 (1H, m), 7.22 - 7.38 (1H, m), 7.35 - 7.42 (1H, m).

### (3) 2-(4-fluoro-3-methylphenyl)hexahydroimidazo[1,5-a]pyrazin-3(2H)-one hydrochloride

¹H-NMR (DMSO-d₆) δ; 2.20 (3H, s), 2.79 - 2.94 (2H, m), 3.18 - 3.28 (2H, m), 3.55 (1H, dd, J = 9.2, 4.0 Hz), 3.78 - 3.87 (1H, m), 3.95 (1H, t, J = 9.8 Hz), 4.06 - 4.14 (2H, m), 7.09 (1H, t, J = 8.8 Hz), 7.34 - 7.43 (2H, m),9.60 (2H, br s,).

### Example 24

### 2-[3-(methylthio)phenyl]hexahydroimidazo[1,5-a]pyrazin-3(2H)-one hydrochloride

The object product was synthesized in the same manner as in Example 1 and from 3-(methylthio)phenyl isocyanate and tert-butyl 3-(hydroxymethyl)piperazine-1-carboxylate.

### (1) tert-butyl 3-(hydroxymethyl)-4-{[3-(methylthio)phenyl]carbamoyl}piperazine-1-carboxylate

¹H-NMR (CDCl₃) δ; 1.47 (9H, s), 2.46 (3H, s), 2. 90 - 3.12 (2H, m), 3.16 (1H, dd, J = 14.0, 4.4 Hz), 3.59 - 3.81 (3H, m), 3.92 - 4.14 (3H, m), 6.89 (1H, d, J = 8.0 Hz), 7.00 (1H, d, J = 8.0 Hz), 7.16 (1H, t, J = 8.0 Hz), 7.25 (1H, s), 7.62 - 7.95 (1H, m).

### (2) tert-butyl 2-[3-(methylthio)phenyl]-3-oxohexahydroimidazo[1,5-a]pyrazine-7(1H)-carboxylate

¹H-NMR (CDCl₃) δ; 1.48 (9H, s), 2.49 (3H, s), 2.54 - 2.87 (2H, m), 2.88 - 3.00 (1H, m), 3.37 - 3.44 (1H, m), 3.66 - 3.76 (1H, m), 3.84 -3.94 (2H, m), 3.94 - 4.35 (2H, m), 6.48 - 6.95 (1H, m), 7.17 - 7.28 (2H, m), 7.58 (1H, s).

### (3) 2-[3-(methylthio)phenyl]hexahydroimidazo[1,5-a]pyrazin-3(2H)-one hydrochloride

¹H-NMR (DMSO-d₆) δ; 2.44 (3H, s), 2.81 - 2.94 (2H, m), 3.18 - 3.29 (2H, m), 3.55 - 3.62 (1H, m), 3.78 - 3.88 (1H, m), 3.96 (1H, t, J = 9.2 Hz), 4.06 -4.15 (2H, m), 6.86 - 6.94 (1H, m), 7.21 - 7.28 (2H, m), 7.51 (1H, s),9.58 (2H, br s).

### Example 25

### 2-(3-phenoxyphenyl)hexahydroimidazo[1,5-a]pyrazin-3(2H)-one hydrochloride

The object product was synthesized in the same manner as in Example 1 and from 3-phenoxyphenyl isocyanate and tert-butyl 3-(hydroxymethyl)piperazine-1-carboxylate.

### (1) tert-butyl 3-(hydroxymethyl)-4-[(3-phenoxyphenyl)carbamoyl]piperazine-1-carboxylate

¹H-NMR (CDCl₃) δ; 1.45 (9H, s), 2.91 - 3.10 (2H, m), 3.11-3.19 (1H, m), 3.58 -3.85 (4H, m), 3.91 - 4.00 (1H, m), 4.02 -4.10 (1H, m), 6.64 (1H, d, J = 8.0 Hz), 6.92 - 7.03 (4H, m), 7.08 (1H, t, J = 7.2 Hz), 7.20 (1H, t, J = 8.0 Hz), 7.28 - 7.35 (2H, m), 7.65 - 7.90 (1H, m).

### (2) tert-butyl 3-oxo-2-(3-phenoxyphenyl)hexahydroimidazo[1,5-a]pyrazine-7(1H)-carboxylate

¹H-NMR (CDCl₃) δ; 1.48 (9H, s), 2.57 - 2.84 (2H, m), 2.85 - 2.97 (1H, m), 3.35 - 3.41 (1H, m), 3.65 - 3.74 (1H, m), 3.83 - 3.94 (2H, m), 3.96 - 4.32 (2H, m), 6.67 (1H, d, J = 7.6 Hz), 7.04 (2H, d, J = 8.0 Hz), 7.09 (1H, t, J = 7.4 Hz), 7.20 - 7.38 (5H, m).

### (3) 2-(3-phenoxyphenyl)hexahydroimidazo[1,5-a]pyrazin-3(2H)-one hydrochloride

¹H-NMR (DMSO-d₆) δ; 2.82- 2.95 (2H, m), 3.19- 3.29 (3H, m), 3.54 -3.60 (1H, m), 3.81- 3.88 (1H, m), 3.95 (1H, t, J = 9.2 Hz), 4.07 - 4.16 (1H, m), 6.65 (1H, dd, J = 8.0, 2.0 Hz), 6.99 (2H, d, J = 7.6 Hz), 7.13 (1H, t, J = 7.4 Hz), 7.18 (1H, dd, J = 8.0, 1.2 Hz), 7.30-7.45 (4H, m),9.59 (2H, br s).

### Example 26

### 2-(3,4-difluorophenyl)hexahydroimidazo[1,5-a]pyrazin-3(2H)-one hydrochloride

The object product was synthesized in the same manner as in Example 1 and from 3,4-difluorophenyl isocyanate and tert-butyl 3-(hydroxymethyl)piperazine-1-carboxylate.

### (1) tert-butyl 4-[(3,4-difluorophenyl)carbamoyl]-3-(hydroxymethyl)piperazine-1-carboxylate

¹H-NMR (CDCl₃) δ; 1.34 (9H, s), 2.82 - 3.10 (3H, m), 3.44 - 3.65 (2H, m), 3.69 - 3.84 (2H, m), 3.89 - 4.13 (2H, m), 6.82 - 6.95 (2H, m), 7.26 - 7.38 (1H, m),8.34 (1H, br s,).

### (2) tert-butyl 2-(3,4-difluorophenyl)-3-oxohexahydroimidazo[1,5-a]pyrazine-7(1H)-carboxylate

¹H-NMR (CDCl₃) δ; 1.48 (9H, s), 2.55- 2.87 (2H, m), 2.88-2.98 (1H, m), 3.33 -3.41 (1H, m), 3.66 - 3.77 (1H, m), 3.81 - 3.95 (2H, m), 3.98 - 4.40 (2H, m), 7.04 - 7.12 (2H, m), 7.60 (1H, dd, J = 11.6, 6.8 Hz).

### (3) 2-(3,4-difluorophenyl)hexahydroimidazo[1,5-a]pyrazin-3(2H)-one hydrochloride

¹H-NMR (DMSO-d₆) δ; 2. 82 - 2.96 (2H, m), 3.20 - 3.30 (3H, m), 3.58 (1H, dd, J = 9.6, 4.4 Hz), 3.81- 3.90 (1H, m), 3.96 (1H, t, J = 9.2 Hz), 4.09 - 4.18 (1H, m), 7.20 - 7.28 (1H, m), 7.36 - 7.45 (1H, m), 7.69 - 7.78 (1H, m),9.66 (2H, br s).

### Example 27

### 2-[3-(diethylamino)phenyl]hexahydroimidazo[1,5-a]pyrazin-3(2H)-one hydrochloride

### (1) tert-butyl 2-[3-(diethylamino)phenyl]-3-oxohexahydroimidazo[1,5-a]pyrazine-7(1H)-carboxylate

A mixture of tert-butyl 2-(3-bromophenyl)-3-oxohexahydroimidazo[1,5-a]pyrazine-7(1H)-carboxylate (200 mg, 0.510 mmol), diethylamine (44.0 mg, 0.610 mmol), sodium tert-butoxide (59.0 mg, 0.610 mmol), 2-dicyclohexylphosphino-2',4',6'-triisopropylbiphenyl (X-phos) (7.00 mg, 0.0200 mmol), bis(dibenzylideneacetone)palladium (0) (6.00 mg, 0.0100 mmol) and toluene (6 ml) was stirred at 90°C for 12 hr under a nitrogen atmosphere, and the solvent was evaporated under reduced pressure. The residue was purified by silica gel column chromatography (hexane:ethyl acetate=1:1) to give the object product (160 mg, 82.0%) as an oil.
¹H-NMR (CDCl₃) δ; 1.10 (6H, t, J = 7.0 Hz), 1.41 (9H, s), 2.51 - 2.92 (3H, m), 3.29 (4H, q, J = 7.0 Hz), 3.32 - 3.39 (1H, m), 3.57 - 3.67 (1H, m), 3.80 - 3.89 (2H, m), 3.91 - 4.29 (2H, m), 6.33 (1H, dd, J = 8.4, 2.6 Hz), 6.42 (1H, dd, J = 8.0, 1.2 Hz), 7.07 (1H, t, J = 8.2 Hz),7.24 (1H, s).

### (2) 2-[3-(diethylamino)phenyl]hexahydroimidazo[1,5-a]pyrazin-3(2H)-one hydrochloride

The object product was synthesized in the same manner as in Example 3 (3) and from tert-butyl 2-[3-(diethylamino)phenyl]-3-oxohexahydroimidazo[1,5-a]pyrazine-7(1H)-carboxylate.
¹H-NMR (DMSO-d₆) δ; 1.02 (6H, t, J = 7.1 Hz), 2.78 -3.03 (2H, m), 3.18 -3.53 (7H, m), 3.80 -3.90 (1H, m), 3.91-4.05 (1H, m), 4.11-4.26 (2H, m), 7.32 -7.58 (2H, m), 7.60 - 7.80 (1H, m), 7.85 - 8.02 (1H, m), 9.72 - 10.07 (1H, m), 12.70 - 13.10 (1H, m).

### Example 28

### 2-benzylhexahydroimidazo[1,5-a]pyrazin-3(2H)-one hydrochloride

The object product was synthesized in the same manner as in Example 1 and from benzyl isocyanate and tert-butyl 3-(hydroxymethyl)piperazine-1-carboxylate.
¹H-NMR (D₂O) δ; 3.12 - 3.21 (4H, m), 3.51 - 3.71 (3H, m), 4.02 - 4.11 (1H, m), 4.45 - 4.53 (2H, m), 4.67 (2H, br s), 4.95 - 4.99 (1H, m), 7.28 - 7.37 (5H, m).

### Example 29

### 2-(2,3-dichlorophenyl)hexahydroimidazo[1,5-a]pyrazin-3(2H)-one hydrochloride

The object product was synthesized in the same manner as in Example 1 and from 2,3-dichlorophenyl isocyanate and tert-butyl 3-(hydroxymethyl)piperazine-1-carboxylate.
¹H-NMR (D₂O) δ; 2.99 - 3.60 (5H, m), 3.75 - 3.99 (2H, m), 4.18 (2H, br s), 4.67 (2H, br s), 7.25 - 7.27 (2H, m), 7.45 - 7.55 (1H, m).

### Example 30

### 2-(2,3,4-trifluorophenyl)hexahydroimidazo[1,5-a]pyrazin-3(2H)-one hydrochloride

The object product was synthesized in the same manner as in Example 1 and from 2,3,4-trifluorophenyl isocyanate and tert-butyl 3-(hydroxymethyl)piperazine-1-carboxylate.
¹H-NMR (D₂O) δ; 3.00 - 3.60 (5H, m), 3.85 - 3.99 (2H, m), 4.20 (2H, br s), 4.67 (2H, br s), 7.00 - 7.11 (2H, m).

### Example 31

### 2-(2,3-dimethylphenyl)hexahydroimidazo[1,5-a]pyrazin-3(2H)-one hydrochloride

The object product was synthesized in the same manner as in Example 1 and from 2,3-dimethylphenyl isocyanate and tert-butyl 3-(hydroxymethyl)piperazine-1-carboxylate.
¹H-NMR (D₂O) δ; 2.03 (3H, s), 2.20 (3H, s), 3.01 - 3.11 (2H, m), 3.16 - 3.52 (4H, m), 3.84 - 3.93 (2H, m), 4.18 (1H, br s), 4.69 (2H, br s), 6.99 - 7.02 (1H, m), 7.10 - 7.17 (2H, m).

### Example 32

### 2-(4-methyl-2-thienyl)hexahydroimidazo[1,5-a]pyrazin-3(2H)-one hydrochloride

The object product was synthesized in the same manner as in Example 1 and from 2-isocyanato-4-methylthiophene and tert-butyl 3-(hydroxymethyl)piperazine-1-carboxylate.
¹H-NMR (DMSO-d₆) δ; 2.11 (3H, s), 2.88 - 2. 95 (2H, m), 3.21 - 3.38 (3H, m), 3.51 - 3.55 (1H, m), 3.80 - 3.95 (2H, m), 4.20 (1H, br s), 6.19 (1H, s), 6.50 (1H, s), 9.70 (2H, br s).

### Example 33

### 2-[5-methyl-2-(trifluoromethyl)-3-furyl]hexahydroimidazo[1,5-a]pyrazin-3(2H)-one hydrochloride

The object product was synthesized in the same manner as in Example 1 and from 3-isocyanato-5-methyl-2-(trifluoromethyl)furan and tert-butyl 3-(hydroxymethyl)piperazine-1-carboxylate.
(1) tert-butyl 3-(hydroxymethyl)-4-{[5-methyl-2-(trifluoromethyl)furan-3-yl]carbamoyl}piperazine-1-carboxylate ¹H NMR (CDCl₃)δ; 1.39 (9H, s), 2.21 (3H, s), 2.72-3.17 (4H, m), 3.41-4.02 (6H, m), 6.58 (1H, s), 7.65-7.81 (1H, m).
(2) tert-butyl 2-[5-methyl-2-(trifluoromethyl)furan-3-yl]-3-oxohexahydroimidazo[1,5-a]pyrazine-7(1H)-carboxylate ¹H NMR (CDCl₃)δ; 1.41 (9H, s), 2.23 (3H, s), 2.51-2.94 (3H, m), 3.24-3.32 (1H, m), 3.60-3.70 (1H, m), 3.72-3.86 (2H, m), 3.90-4.25 (2H, m), 6.46 (1H, s).
(3) 2-[5-methyl-2-(trifluoromethyl)-3-furyl]hexahydroimidazo[1,5-a]pyrazin-3(2H)-one hydrochloride ¹H-NMR (D₂O) δ; 2.03 (3H, s), 3.01 - 3.08 (2H, m), 3.11 - 3.56 (4H, m), 3.94 - 3.98 (2H, m), 4.20 (1H, br s), 4.74 (2H, br s), 6.26 (1H, s).

### Example 34

### 2-(2-thienyl)hexahydroimidazo[1,5-a]pyrazin-3(2H)-one trifluoroacetate

The object product was synthesized in the same manner as in Example 1 and from 2-isocyanatothiophene and tert-butyl 3-(hydroxymethyl)piperazine-1-carboxylate, and the final object product was purified by preparative high performance liquid chromatography (HPLC; column: Fuji C18 (300x25); wavelength 220 nm; mobile phase: A acetonitrile (containing 0.1% trifluoroacetic acid); B water (containing 0.1% trifluoroacetic acid); flow rate: 25 mL/min).
¹H-NMR (D₂O) δ; 2.32 - 2.59 (2H, m), 2.76 - 3.00 (3H, m), 3.39 - 3.42 (1H, m), 3.61 - 3.90 (3H, m), 4.77 (2H, br s), 6.36 - 6.37 (1H, m), 6.80 - 6.90 (1H, m), 7.10 - 7.15 (1H, m).

### Example 35

### 2-(3-thienyl)hexahydroimidazo[1,5-a]pyrazin-3(2H)-one hydrochloride

The object product was synthesized in the same manner as in Example 1 and from 3-isocyanatothiophene and tert-butyl 3-(hydroxymethyl)piperazine-1-carboxylate.
¹H-NMR (D₂O) δ; 2.89 - 2.96 (2H, m), 3.16 - 3.50 (4H, m), 3.86 - 4.00 (2H, m), 4.03 (1H, br s), 4.69 (2H, br s), 6.91(1H, s), 7.22 (1H, d, J = 0.4 Hz), 7.33 (1H, d,J = 0.4 Hz).

### Example 36

### 2-(3,5-dimethylisoxazol-4-yl)hexahydroimidazo[1,5-a]pyrazin-3(2H)-one hydrochloride

The object product was synthesized in the same manner as in Example 1 and from 4-isocyanato-3,5-dimethylisoxazole and tert-butyl 3-(hydroxymethyl)piperazine-1-carboxylate.
¹H-NMR (D₂O) δ; 2.06 (3H, s), 2.21 (3H, s), 2.98 - 3.50 (6H, m), 3.81 - 3.92 (2H, m), 4.16 (1H, br s), 4.69 (2H, br s).

### Example 37

### 2-(2-ethylphenyl)hexahydroimidazo[1,5-a]pyrazin-3(2H)-one hydrochloride

### (1) tert-butyl 4-[(2-ethylphenyl)carbamoyl]-3-(hydroxymethyl)piperazine-1-carboxylate

To a solution (10 ml) of tert-butyl 3-(hydroxymethyl)piperazine-1-carboxylate (357 mg, 1.65 mmol) in tetrahydrofuran was added 2-ethylphenyl isocyanate (0.212 ml, 1.5 mmol) under ice-cooling, and the mixture was stirred for 1.5 hr. The solvent was evaporated under reduced pressure, and the residue was purified by silica gel column chromatography (hexane:ethyl acetate=4:1 - 1:2) to give the object product (545 mg, 100%) as an oil.
¹H-NMR (CDCl₃) δ; 1.20 (3H, t, J = 7.5 Hz), 1.47 (9H, s), 2.55 (2H, q, J = 7.5 Hz), 2.90 - 3.30 (4H, m), 3.57 - 4.18 (5H, m), 6.99 - 7.08 (1H, m), 7.10 - 7.23 (3H, m), 7.52 - 7.61 (1H, m).

### (2) tert-butyl 2-(2-ethylphenyl)-3-oxohexahydroimidazo[1,5-a]pyrazine-7(1H)-carboxylate

To a solution (10 ml) of tert-butyl 4-[(2-ethylphenyl)carbamoyl]-3-(hydroxymethyl)piperazine-1-carboxylate (490 mg, 1.4 mmol) and 1,8-diazabicyclo[5.4.0]-7-undecene (1.6 ml, 10.8 mmol) in dichloromethane was added methanesulfonyl chloride (0.262 ml, 3.4 mmol) under ice-cooling, and the mixture was stirred at room temperature for 15 hr. Water was added to the reaction mixture, and the mixture was extracted with dichloromethane. The extract was washed with saturated sodium hydrogen carbonate solution, saturated ammonium chloride solution and saturated brine, and dried over anhydrous magnesium sulfate. The solvent was evaporated under reduced pressure, and the residue was purified by silica gel column chromatography (hexane:ethyl acetate=9:1 - 2:1) to give the object product (421 mg, 90%) as an oil.
¹H-NMR (CDCl₃) δ; 1.23 (3H, t, J = 7.5 Hz), 1.49 (9H, s), 2.64 (2H, q, J = 7.5 Hz), 2.71 - 3.03 (3H, m), 3.25 - 3.38 (1H, m), 3.66 - 4.31 (5H, m), 7.10 - 7.34 (4H, m).

### (3) 2-(2-ethylphenyl)hexahydroimidazo[1,5-a]pyrazin-3(2H)-one hydrochloride

To tert-butyl 2-(2-ethylphenyl)-3-oxohexahydroimidazo[1,5-a]pyrazine-7(1H)-carboxylate (337 mg, 0.98 mmol) was added 4N hydrogen chloride-ethyl acetate solution (10 ml), and the mixture was stirred at room temperature for 1 hr. The solvent was evaporated under reduced pressure, and the residue was washed with ethyl acetate to give the object product (250 mg, 91%) as a solid.
¹H-NMR (DMSO-d₆) δ; 1.15 (3H, t, J = 7.5 Hz), 2.56 (2H, q, J = 7.5 Hz), 2.78 - 3.02 (2H, m), 3.17 - 3.47 (4H, m), 3.73 - 3.87 (2H, m), 4.06 - 4.21 (1H, m), 7.15 - 7.36 (4H, m) 9.67 (2H, br s).

### Example 38

### 2-(4-ethylphenyl)hexahydroimidazo[1,5-a]pyrazin-3(2H)-one hydrochloride

### (1) tert-butyl 4-[(4-ethylphenyl)carbamoyl]-3-(hydroxymethyl)piperazine-1-carboxylate

To a solution (10 ml) of tert-butyl 3-(hydroxymethyl)piperazine-1-carboxylate (324 mg, 1.50 mmol) in tetrahydrofuran was added 4-ethylphenyl isocyanate (0.216 ml, 1.5 mmol) under ice-cooling, and the mixture was stirred for 3 hr. The solvent was evaporated under reduced pressure, and the residue was recrystallized from a mixed solvent of hexane and ethyl acetate to give the object product (487 mg, 87%) as a solid.
¹H-NMR (CDCl₃) δ; 1.20 (3H, t, J = 7.6 Hz), 1.46 (9H, s), 2.59 (2H, q, J = 7.6 Hz), 2.96 - 3.38 (4H, m), 3.61 - 4.16 (6H, m), 7.05 - 7.23 (4H, m), 7.40 (1H, br s).

### (2) tert-butyl 2-(4-ethylphenyl)-3-oxohexahydroimidazo[1,5-a]pyrazine-7(1H)-carboxylate

To a solution (10 ml) of tert-butyl 4-[(4-ethylphenyl)carbamoyl]-3-(hydroxymethyl)piperazine-1-carboxylate (430 mg, 1.2 mmol) and 1,8-diazabicyclo[5.4.0]-7-undecene (1.4 ml, 9.5 mmol) in dichloromethane was added methanesulfonyl chloride (0.229 ml, 3.0 mmol) under ice-cooling, and the mixture was stirred at room temperature for 15 hr. Water was added to the reaction mixture, and the mixture was extracted with ethyl acetate. The extract was washed with saturated sodium hydrogen carbonate solution, saturated ammonium chloride solution and saturated brine, and dried over anhydrous magnesium sulfate. The solvent was evaporated under reduced pressure, and the residue was purified by silica gel column chromatography (hexane:ethyl acetate=9:1 - 2:1) to give the object product (339 mg, 83%) as an oil, which was recrystallized from a mixed solvent of hexane and ethyl acetate to give the object product as a solid.
¹H-NMR (CDCl₃) δ; 1.21 (3H, t, J = 7.6 Hz), 1.49 (9H, s), 2.53 - 3.03 (5H, m), 3.42 (1H, dd, J = 9.2, 5.1 Hz), 3.64 - 3.78 (1H, m), 3.84 - 4.35 (4H, m), 7.12 - 7.20 (2H, m), 7.41 - 7.48 (2H, m).

### (3) 2-(4-ethylphenyl)hexahydroimidazo[1,5-a]pyrazin-3(2H)-one hydrochloride

To tert-butyl 2-(4-ethylphenyl)-3-oxohexahydroimidazo[1,5-a]pyrazine-7(1H)-carboxylate (280 mg, 0.81 mmol) was added 4N hydrogen chloride-ethyl acetate solution (10 ml), and the mixture was stirred at room temperature for 1 hr. The solvent was evaporated under reduced pressure, and the residue was washed with ethyl acetate to give the object product (200 mg, 88%) as a solid.
¹H-NMR (DMSO-d₆) δ; 1.15 (3H, t, J = 7.6 Hz), 2.46 - 2.62 (2H, m), 2.80 - 3.00 (2H, m), 3.17 - 3.43 (3H, m), 3.57 (1H, dd, J = 9.7, 4.4 Hz), 3.80 - 4.01 (2H, m), 4.06 - 4.20 (1H, m), 7.13 - 7.21 (2H, m), 7.41 - 7.50 (2H, m), 9.47 - 9.97 (2H, m).

### Example 39

### 2-[3-(methylsulfonyl)phenyl]hexahydroimidazo[1,5-a]pyrazin-3(2H)-one hydrochloride

### (1) tert-butyl 2-[3-(methylsulfonyl)phenyl]-3-oxohexahydroimidazo[1,5-a]pyrazine-7(1H)-carboxylate

To a solution (10 ml) of tert-butyl 2-[3-(methylthio)phenyl]-3-oxohexahydroimidazo[1,5-a]pyrazine-7(1H)-carboxylate (350 mg, 0.96 mmol) in ethyl acetate was added m-chloroperbenzoic acid (65%, 512 mg, 1.92 mmol) at room temperature, and the mixture was stirred for 2 hr. The reaction mixture was poured into water, and the mixture was extracted with ethyl acetate. The extract was washed with saturated sodium hydrogen carbonate solution, sodium thiosulfate solution and saturated brine, and dried over anhydrous magnesium sulfate. The solvent was evaporated under reduced pressure, and the residue was recrystallized from a mixed solvent of hexane and ethyl acetate to give the object product (329 mg, 86%) as a solid.
¹H-NMR (CDCl₃) δ; 1.49 (9H, s), 2.59 - 3.04 (3H, m), 3.06 (3H, s), 3.50 (1H, dd, J = 9.1, 5.3 Hz), 3.72 - 3.85 (1H, m), 3.88 - 4.43 (4H, m), 7.49 - 7.63 (2H, m), 7.87 - 7.93 (1H, m), 8.04 - 8.13 (1H, m).

### (2) 2-[3-(methylsulfonyl)phenyl]hexahydroimidazo[1,5-a]pyrazin-3(2H)-one hydrochloride

To tert-butyl 2-[3-(methylsulfonyl)phenyl]-3-oxohexahydroimidazo[1,5-a]pyrazine-7(1H)-carboxylate (280 mg, 0.71 mmol) was added 4N hydrogen chloride-ethyl acetate solution (10 ml), and the mixture was stirred at room temperature for 1 hr, and the precipitated crystals were washed with ethyl acetate to give the object product (206 mg, 88%) as a solid.
¹H-NMR (DMSO-d₆) δ; 2.83 - 3.03 (2H, m), 3.20 (3H, s), 3.22 - 3.46 (3H, m), 3.68 (1H, dd, J = 9.5, 4.2 Hz), 3.84 - 3.96 (1H, m), 4.06 (1H, t, J = 9.1 Hz), 4.12 - 4.25 (1H, m), 7.53 - 7.67 (2H, m), 7.79 - 7.87 (1H, m), 8.14 - 8.20 (1H, m), 9.50 - 9.88 (2H, m).

### Example 40

### 2-[4-(methylthio)phenyl]hexahydroimidazo[1,5-a]pyrazin-3(2H)-one hydrochloride

### (1) tert-butyl 3-(hydroxymethyl)-4-{[4-(methylthio)phenyl]carbamoyl}piperazine-1-carboxylate

To a solution (20 ml) of tert-butyl 3-(hydroxymethyl)piperazine-1-carboxylate (540 mg, 2.50 mmol) in tetrahydrofuran was added 4-(methylthio)phenyl isocyanate (0.351 ml, 2.50 mmol) under ice-cooling, and the mixture was stirred for 30 min. The solvent was evaporated under reduced pressure, and the residue was recrystallized from diethyl ether to give the object product (801 mg, 84%) as a solid.
¹H-NMR (CDCl₃) δ; 1.46 (9H, s), 2.45 (3H, s), 2.96 - 3.47 (4H, m), 3.60 - 4.16 (6H, m), 7.21 (4H, s), 7.60 (1H, br s).

### (2) tert-butyl 2-[4-(methylthio)phenyl]-3-oxohexahydroimidazo[1,5-a]pyrazine-7(1H)-carboxylate

To a solution (10 ml) of tert-butyl 3-(hydroxymethyl)-4-{[4-(methylthio)phenyl]carbamoyl}piperazine-1-carboxylate (750 mg, 1.96 mmol) and 1,8-diazabicyclo[5.4.0]-7-undecene (2.4 ml, 15.7 mmol) in dichloromethane was added methanesulfonyl chloride (0.380 ml, 4.91 mmol) under ice-cooling, and the mixture was stirred at room temperature for 15 hr. Water was added to the reaction mixture, and the mixture was extracted with ethyl acetate. The extract was washed with saturated sodium hydrogen carbonate solution, saturated ammonium chloride solution and saturated brine, and dried over anhydrous magnesium sulfate. The solvent was evaporated under reduced pressure, and the residue was recrystallized from a mixed solvent of hexane and ethyl acetate to give the object product (613 mg, 86%) as a solid.
¹H-NMR (CDCl₃) δ; 1.49 (9H, s), 2.46 (3H, s), 2.58 - 3.03 (3H, m), 3.41 (1H, dd, J = 9.1, 5.3 Hz), 3.65 - 3.79 (1H, m), 3.83 - 3.97 (2H, m), 3.99 - 4.37 (2H, m), 7.23 - 7.31 (2H, m), 7.44 - 7.53 (2H, m).

### (3) 2-[4-(methylthio)phenyl]hexahydroimidazo[1,5-a]pyrazin-3(2H)-one hydrochloride

To tert-butyl 2-[4-(methylthio)phenyl]-3-oxohexahydroimidazo[1,5-a]pyrazine-7(1H)-carboxylate (200 mg, 0.55 mmol) was added 4N hydrogen chloride-ethyl acetate solution (10 ml), and the mixture was stirred at room temperature for 1 hr, and the precipitated crystals were washed with ethyl acetate to give the object product (153 mg, 93%) as a solid.
¹H-NMR (DMSO-d₆) δ; 2.44 (3H, s), 2.81 - 3.01 (1H, m), 3.17 - 3.47 (4H, m), 3.57 (1H, dd, J = 9.8, 4.5 Hz), 3.80 - 4.01 (2H, m), 4.06 - 4.20 (1H, m), 7.22 - 7.33 (2H, m), 7.46 - 7.57 (2H, m), 9.62 (2H, br s).

### Example 41

### 2-[4-(methylsulfonyl)phenyl]hexahydroimidazo[1,5-a]pyrazin-3(2H)-one hydrochloride

### (1) tert-butyl 2-[4-(methylsulfonyl)phenyl]-3-oxohexahydroimidazo[1,5-a]pyrazine-7(1H)-carboxylate

To a solution (10 ml) of tert-butyl 2-[4-(methylthio)phenyl]-3-oxohexahydroimidazo[1,5-a]pyrazine-7(1H)-carboxylate (350 mg, 0.96 mmol) in ethyl acetate was added m-chloroperbenzoic acid (65%, 512 mg, 1.92 mmol) at room temperature, and the mixture was stirred for 2 hr. The precipitated crystals were washed with ethyl acetate to give the object product (336 mg, 88%) as a solid.
¹H-NMR (DMSO-d₆) δ; 1.43 (9H, s), 2.63 - 2.96 (3H, m), 3.15 (3H, s), 3.57 (1H, dd, J = 9.8, 5.3 Hz), 3.67 - 3.82 (2H, m), 3.87 - 4.20 (3H, m), 7.75 - 7.89 (4H, m).

### (2) 2-[4-(methylsulfonyl)phenyl]hexahydroimidazo[1,5-a]pyrazin-3(2H)-one hydrochloride

To tert-butyl 2-[4-(methylsulfonyl)phenyl]-3-oxohexahydroimidazo[1,5-a]pyrazine-7(1H)-carboxylate (250 mg, 0.63 mmol) was added 4N hydrogen chloride-ethyl acetate solution (10 ml), and the mixture was stirred at room temperature for 1 hr, and the precipitated crystals were washed with ethyl acetate to give the object product (196 mg, 93%) as a solid.
¹H-NMR (DMSO-d₆) δ; 2.86 - 3.04 (2H, m), 3.16 (3H, s), 3.19 - 3.48 (3H, m), 3.66 (1H, dd, J = 9.5, 4.2 Hz), 3.84 - 4.22 (3H, m), 7.75 - 7.93 (4H, m), 9.38 (2H, br s).

### Example 42

### ethyl 4-(3-oxohexahydroimidazo[1,5-a]pyrazin-2(3H)-yl)benzoate hydrochloride

### (1) t-butyl 4-{[4-(ethoxycarbonyl)phenyl]carbamoyl}-3-(hydroxymethyl)piperazine-1-carboxylate

To a solution (10 ml) of tert-butyl 3-(hydroxymethyl)piperazine-1-carboxylate (324 mg, 1.50 mmol) in tetrahydrofuran was added ethyl 4-isocyanatobenzoate (287 mg, 1.5 mmol) under ice-cooling, and the mixture was stirred for 1 hr. The solvent was evaporated under reduced pressure, and the residue was recrystallized from a mixed solvent of hexane and ethyl acetate to give the object product (491 mg, 80%) as a solid.
¹H-NMR (CDCl₃) δ; 1.38 (3H, t, J = 7.2 Hz), 1. 45 (9H, s), 2.93 - 3.28 (3H, m), 3.58 - 3.91 (5H, m), 3.96 - 4.17 (2H, m), 4.34 (2H, q, J = 7.2 Hz), 7.28 - 7.36 (2H, m), 7.90 - 7.98 (2H, m), 8.20 (1H, br s).

### (2) tert-butyl 2-[4-(ethoxycarbonyl)phenyl]-3-oxohexahydroimidazo[1,5-a]pyrazine-7(1H)-carboxylate

To a solution (8 ml) of tert-butyl 4-{[4-(ethoxycarbonyl)phenyl]carbamoyl}-3-(hydroxymethyl)piperazine-1-carboxylate (430 mg, 1.05 mmol) and 1,8-diazabicyclo[5.4.0]-7-undecene (1.3 ml, 8.4 mmol) in dichloromethane was added methanesulfonyl chloride (0.204 ml, 2.6 mmol) under ice-cooling, and the mixture was stirred at room temperature for 15 hr. Water was added to the reaction mixture, and the mixture was extracted with ethyl acetate. The extract was washed with saturated sodium hydrogen carbonate solution, saturated ammonium chloride solution and saturated brine, and dried over anhydrous magnesium sulfate. The solvent was evaporated under reduced pressure, and the residue was recrystallized from a mixed solvent of hexane and ethyl acetate to give the object product (322 mg, 78%) as a solid.
¹H-NMR (CDCl₃) δ; 1.39 (3H, t, J = 7.2 Hz), 1.49 (9H, s), 2.56 - 3.05 (3H, m), 3.47 (1H, dd, J = 9.5, 5.3 Hz), 3.69 - 3.83 (1H, m), 3.89 - 4.42 (6H, m), 7.58 - 7.67 (2H, m), 7.97 - 8.06 (2H, m).

### (3) ethyl 4-(3-oxohexahydroimidazo[1,5-a]pyrazin-2(3H)-yl)benzoate hydrochloride

To tert-butyl 2-[4-(ethoxycarbonyl)phenyl]-3-oxohexahydroimidazo[1,5-a]pyrazine-7(1H)-carboxylate (250 mg, 0.64 mmol) was added 4N hydrogen chloride-ethyl acetate solution (10 ml), and the mixture was stirred at room temperature for 1 hr, and the precipitated crystals were washed with ethyl acetate to give the object product (186 mg, 89%) as a solid.
¹H-NMR (DMSO-d₆) δ; 1.31 (3H, t, J = 7.2 Hz), 2.84 - 3.04 (2H, m), 3.20 - 3.46 (3H, m), 3.65 (1H, dd, J = 9.8, 4.5 Hz), 3.84 - 3.96 (1H, m), 4.04 (1H, t, J = 9.3 Hz), 4.11 - 4.24 (1H, m), 4.28 (2H, q, J = 7.2 Hz), 7.63 - 7.76 (2H, m), 7.86 - 7.98 (2H, m), 9.70 (2H, br s).

### Example 43

### ethyl 2-(3-oxohexahydroimidazo[1,5-a]pyrazin-2(3H)-yl)benzoate hydrobromide

### (1) tert-butyl 4-{[2-(ethoxycarbonyl)phenyl]carbamoyl}- 3-(hydroxymethyl)piperazine-1-carboxylate

To a solution (10 ml) of tert-butyl 3-(hydroxymethyl)piperazine-1-carboxylate (324 mg, 1.50 mmol) in tetrahydrofuran was added ethyl 2-isocyanatobenzoate (487 mg, 1.50 mmol) under ice-cooling, and the mixture was stirred for 30 min. The solvent was evaporated under reduced pressure, and the residue was purified by silica gel column chromatography (hexane:ethyl acetate=9:1 - 1:1 to give the object product (469 mg, 77%) as an oil.
¹H-NMR (CDCl₃) δ; 1.41 (3H, t, J = 7.1 Hz), 1.49 (9H, s), 3.02 - 3.35 (3H, m), 3.65 - 3.84 (2H, m), 3.89 - 4.46 (6H, m), 6.93 - 7.04 (1H, m), 7.44 - 7.57 (1H, m), 8.02 (1H, dd, J = 8.0, 1.6 Hz), 8.49 (1H, dd, J = 8.6, 0.9 Hz), 10.86 (1H, s).

### (2) tert-butyl 2-[2-(ethoxycarbonyl)phenyl]-3-oxohexahydroimidazo[1,5-a]pyrazine-7(1H)-carboxylate

To a solution (8 ml) of tert-butyl 4-{[2-(ethoxycarbonyl)phenyl]carbamoyl}-3-(hydroxymethyl)piperazine-1-carboxylate (408 mg, 1.0 mmol) and 1,8-diazabicyclo[5.4.0]-7-undecene (1.2 ml, 8.0 mmol) in dichloromethane was added methanesulfonyl chloride (0.194 ml, 2.5 mmol) under ice-cooling, and the mixture was stirred at room temperature for 15 hr. Water was added to the reaction mixture, and the mixture was extracted with ethyl acetate. The extract was washed with saturated sodium hydrogen carbonate solution, saturated ammonium chloride solution and saturated brine, and dried over anhydrous magnesium sulfate. The solvent was evaporated under reduced pressure, and the residue was purified by silica gel column chromatography (hexane:ethyl acetate=9:1 - 1:1 to give the object product (355 mg, 91%) as an oil.
¹H-NMR (CDCl₃) δ; 1.36 (3H, t, J = 7.2 Hz), 1.49 (9H, s), 2.77 - 3.06 (3H, m), 3.48 (1H, dd, J = 8.7, 4.5 Hz), 3.70 - 4.40 (7H, m), 7.16 - 7.33 (2H, m), 7.44 - 7.55 (1H, m), 7.86 (1H, dd, J = 7.8, 1.7 Hz).

### (3) ethyl 2-(3-oxohexahydroimidazo[1,5-a]pyrazin-2(3H)-yl)benzoate hydrobromide

To tert-butyl 2-[2-(ethoxycarbonyl)phenyl]-3-oxohexahydroimidazo[1,5-a]pyrazine-7(1H)-carboxylate (290 mg, 0.74 mmol) was added 20% hydrobromic acid-ethanol reagent (10 ml), and the mixture was stirred at room temperature for 1 hr, and the precipitated crystals were washed with ethyl acetate to give the object product (239 mg, 87%) as a solid.
¹H-NMR (DMSO-d₆) δ; 1.26 (3H, t, J = 7.2 Hz), 2.70 - 2.89 (1H, m), 2.91 - 3.09 (1H, m), 3.15 - 3.37 (2H, m), 3.38 - 3.52 (1H, m), 3.64 (1H, dd, J = 9.2, 3.6 Hz), 3.82 (1H, dd, J = 13.7, 3.7 Hz), 3.93 - 4.29 (4H, m), 7.27 - 7.43 (2H, m), 7.54 - 7.66 (1H, m), 7.72 (1H, dd, J = 7.7, 1.3 Hz), 9.15 (2H, br s).

### Example 44

### 2-[2-(methylthio)phenyl]hexahydroimidazo[1,5-a]pyrazin-3(2H)-one hydrobromide

### (1) tert-butyl 3-(hydroxymethyl)-4-{[2-(methylthio)phenyl]carbamoyl}piperazine-1-carboxylate

To a solution (15 ml) of tert-butyl 3-(hydroxymethyl)piperazine-1-carboxylate (540 mg, 2.5 mmol) in tetrahydrofuran was added 2-(methylthio)phenyl isocyanate (0.341 ml, 2.5 mmol) under ice-cooling, and the mixture was stirred for 2 hr. The solvent was evaporated under reduced pressure, and the residue was purified by silica gel column chromatography (hexane:ethyl acetate=9:1 - 1:1 to give the object product (839 mg, 88%) as an oil.
¹H-NMR (CDCl₃) δ; 1.48 (9H, s), 2.36 (3H, s), 2.98 - 3.28 (3H, m), 3.64 - 4.30 (6H, m), 6.95 - 7.04 (1H, m), 7.21 - 7.31 (1H, m), 7.44 (1H, dd, J = 7.9, 1.5 Hz), 7.97 - 8.07 (2H, m).

### (2) tert-butyl 2-[2-(methylthio)phenyl]-3-oxohexahydroimidazo[1,5-a]pyrazine-7(1H)-carboxylate

To a solution (15 ml) of tert-butyl 3-(hydroxymethyl)-4-{[2-(methylthio)phenyl]carbamoyl}piperazine-1-carboxylate (780 mg, 2.00 mmol) and 1,8-diazabicyclo[5.4.0]-7-undecene (2.40 ml, 16.0 mmol) in dichloromethane was added methanesulfonyl chloride (0.464 ml, 6.00 mmol) under ice-cooling, and the mixture was stirred at room temperature for 15 hr. Water was added to the reaction mixture, and the mixture was extracted with ethyl acetate. The extract was washed with saturated sodium hydrogen carbonate solution, saturated ammonium chloride solution and saturated brine, and dried over anhydrous magnesium sulfate. The solvent was evaporated under reduced pressure, and the residue was purified by silica gel column chromatography (hexane:ethyl acetate=9:1 - 1:1 to give the object product (700 mg, 94%) as an oil.
¹H-NMR (CDCl₃) δ; 1.49 (9H, s), 2.44 (3H, s), 2.78 - 3.04 (3H, m), 3.39 - 3.49 (1H, m), 3.69 - 4.28 (5H, m), 7.13 - 7.34 (4H, m).

### (3) 2-[2-(methylthio)phenyl]hexahydroimidazo[1,5-a]pyrazin-3(2H)-one hydrobromide

To tert-butyl 2-[2-(methylthio)phenyl]-3-oxohexahydroimidazo[1,5-a]pyrazine-7(1H)-carboxylate (300 mg, 0.83 mmol) was added 4N hydrogen chloride-ethyl acetate solution (10 ml), and the mixture was stirred at room temperature for 30 min. The solvent was evaporated under reduced pressure, and a solution (10 ml) of the residue in ethyl acetate was neutralized with triethylamine (0.127 ml, 0.91 mmol). The solvent was evaporated under reduced pressure, 20% hydrobromic acid-ethanol solution (10 ml) was added to the residue, and the mixture was stirred at room temperature for 1 hr. The precipitated crystals were washed with ethyl acetate to give the object product (207 mg, 73%) as a solid.
¹H-NMR (DMSO-d₆) δ; 2.44 (3H, s), 2.80 - 3.06 (2H, m), 3.12 - 3.50 (4H, m), 3.74 - 3.90 (2H, m), 3.99 - 4.13 (1H, m), 7.16 - 7.28 (2H, m), 7.30 - 7.40 (2H, m), 8.99 (2H, br s).

### Example 45

### 2-[2-(methylsulfonyl)phenyl]hexahydroimidazo[1,5-a]pyrazin-3(2H)-one hydrobromide

### (1) tert-butyl 2-[2-(methylsulfonyl)phenyl]-3-oxohexahydroimidazo[1,5-a]pyrazine-7(1H)-carboxylate

To a solution (10 ml) of tert-butyl 2-[2-(methylthio)phenyl]-3-oxohexahydroimidazo[1,5-a]pyrazine-7(1H)-carboxylate (338 mg, 0.93 mmol) in ethyl acetate was added m-chloroperbenzoic acid (65%, 494 mg, 1.86 mmol) at room temperature, and the mixture was stirred for 1 hr. The reaction mixture was poured into water, and the mixture was extracted with ethyl acetate. The extract was washed with saturated sodium hydrogen carbonate solution, sodium thiosulfate solution and saturated brine, and dried over anhydrous magnesium sulfate. The solvent was evaporated under reduced pressure, and the residue was purified by silica gel column chromatography (hexane:ethyl acetate=85:15 - 1:1) to give the object product (277 mg, 75%) as an oil.
¹H-NMR (CDCl₃) δ; 1.49 (9H, s), 2.72 - 3.10 (3H, m), 3.20 (3H, s), 3.72 - 4.30 (6H, m), 7.35 (1H, d, J = 8.0 Hz), 7.55 (1H, t, J = 7.6 Hz), 7.65 - 7.78 (1H, m), 8.11 (1H, dd, J = 8.0, 1.5 Hz).

### (2) 2-[2-(methylsulfonyl)phenyl]hexahydroimidazo[1,5-a]pyrazin-3(2H)-one hydrobromide

To tert-butyl 2-[2-(methylsulfonyl)phenyl]-3-oxohexahydroimidazo[1,5-a]pyrazine-7(1H)-carboxylate (243 mg, 0.61 mmol) was added 4N hydrogen chloride-ethyl acetate solution (10 ml), and the mixture was stirred at room temperature for 1 hr. The solvent was evaporated under reduced pressure, and a solution (10 ml) of the residue in ethyl acetate was neutralized with triethylamine (0.094 ml, 0.67 mmol). The solvent was evaporated under reduced pressure, 20% hydrobromic acid-ethanol solution (10 ml) was added to the residue, and the mixture was stirred at room temperature for 1 hr. The precipitated crystals were washed with ethyl acetate to give the object product (37 mg, 16%) as a solid.

### ¹H-NMR (DMSO-d₆) δ; 2.77 - 3.11 (2H, m), 3.15 - 3.53 (7H, s), 3.79 - 3.97 (2H, m), 4.02 - 4.16 (1H, m), 7.50 - 7.72 (2H, m), 7.76 - 7.78 (1H, m), 8.00 (1H, dd, J = 7.9, 1.5 Hz), 8.97 (2H, br s).

### Example 46

### 2-(3-aminophenyl)hexahydroimidazo[1,5-a]pyrazin-3(2H)-one dihydrochloride

### (1) tert-butyl 2-{3-[(diphenylmethylene)amino]phenyl}-3-oxohexahydroimidazo[1,5-a]pyrazine-7(1H)-carboxylate

A solution (10 ml) of tert-butyl 4-[(3-bromophenyl)carbamoyl]-3-(hydroxymethyl)piperazine-1-carboxylate (310 mg, 0.78 mmol), benzophenonimine (0.158 ml, 0.94 mmol), bis(dibenzylideneacetone)palladium (45 mg, 0.078 mmol), dicyclohexyl(2',4',6'-triisopropylbiphenyl-2-yl)phosphine (75 mg, 0.156 mmol) and sodium tert-butoxide (90 mg, 0.94 mmol) in toluene was stirred under an argon stream at 90°C for 2 hr. Water was added to the reaction product, and the mixture was filtered through celite. The filtrate was extracted with ethyl acetate. The extract was washed with saturated brine, and dried over anhydrous magnesium sulfate. The solvent was evaporated under reduced pressure, and the residue was purified by silica gel column chromatography (hexane:ethyl acetate=9:1 - 2:1) to give the object product (353 mg, 91%) as an oil, which was recrystallized from a mixed solvent of hexane and diethyl ether to give the object product as a solid.
¹H-NMR (CDCl₃) δ; 1.48 (9H, s), 2.52 - 2.99 (3H, m), 3.25 (1H, dd, J = 8.8, 4.6 Hz), 3.58 - 3.79 (2H, m), 3.83 - 3.95 (1H, m), 3.97 - 4.32 (2H, m), 6.37 (1H, d, J = 7.7 Hz), 6.75 (1H, br s), 7.02 - 7.19 (3H, m), 7.20 - 7.32 (3H, m), 7.33 - 7.51 (4H, m), 7.68 - 7.78 (2H, m).

### (2) tert-butyl 2-(3-aminophenyl)-3-oxohexahydroimidazo[1,5-a]pyrazine-7(1H)-carboxylate

A solution (6 ml) of tert-butyl 2-{3-[(diphenylmethylene)amino]phenyl}-3-oxohexahydroimidazo[1,5-a]pyrazine-7(1H)-carboxylate (300 mg, 0.6 mmol), hydroxylamine hydrochloride (84 mg, 1.2 mmol) and sodium acetate (124 mg, 1.5 mmol) in methanol was stirred at room temperature for 30 min. The solvent was evaporated under reduced pressure, saturated aqueous sodium hydrogen carbonate solution was added to the residue, and the mixture was extracted with ethyl acetate. The extract was washed with saturated brine, and dried over anhydrous magnesium sulfate. The solvent was evaporated under reduced pressure, and the residue was recrystallized from diethyl ether to give the object product (180 mg, 90%) as a solid.
¹H-NMR (CDCl₃) δ; 1.48 (9H, s), 2.57 - 3.02 (3H, m), 3.40 (1H, dd, J = 9.2, 5.1 Hz), 3. 61 - 3.77 (3H, m), 3. 83 - 3.97 (2H, m), 4.00 - 4.32 (2H, m), 6.34 - 6.43 (1H, m), 6.65 - 6.74 (1H, m), 7.10 (1H, t, J = 8.1 Hz), 7.17 - 7.23 (1H, m).

### (3) 2-(3-aminophenyl)hexahydroimidazo[1,5-a]pyrazin-3(2H)-one dihydrochloride

To tert-butyl 2-(3-aminophenyl)-3-oxohexahydroimidazo[1,5-a]pyrazine-7(1H)-carboxylate (125 mg, 0.38 mmol) was added 4N hydrogen chloride-ethyl acetate solution (10 ml), and the mixture was stirred at room temperature for 30 min. The precipitated crystals were washed with ethyl acetate to give the object product (101 mg, 88%) as a solid.
¹H-NMR (DMSO-d₆) δ; 2.79 - 3.04 (2H, m), 3.17 - 4.23 (7H, m), 6.90 - 7.00 (1H, m), 7.23 - 7.34 (1H, m),7.34 - 7.45 (1H, m), 7.76 (1H, s), 9.42 - 10.32 (4H, m).

### Example 47

### 2-(3-hydroxyphenyl)hexahydroimidazo[1,5-a]pyrazin-3(2H)-one hydrochloride

### (1) tert-butyl 2-(3-hydroxyphenyl)-3-oxohexahydroimidazo[1,5-a]pyrazine-7(1H)-carboxylate

A solution of tert-butyl 4-[(3-bromophenyl)carbamoyl]-3-(hydroxymethyl)piperazine-1-carboxylate (396 mg, 1.00 mmol), potassium hydroxide (94 mg, 1.70 mmol), tris(dibenzylideneacetone)dipalladium (18.3 mg, 0.02 mmol) and di-tert-butyl(2',4',6'-triisopropylbiphenyl-2-yl)phosphine (34 mg, 0.08 mmol) in 1,4-dioxane-water (2:1, 1.5 ml) was stirred under an argon stream at 100°C for 15 hr. The reaction product was acidified with 1N hydrochloric acid, water was added, and the mixture was extracted with ethyl acetate. The extract was washed with saturated brine, and dried over anhydrous magnesium sulfate. The solvent was evaporated under reduced pressure, and the residue was recrystallized from a mixed solvent of diethyl ether and ethyl acetate to give the object product (260 mg, 78%) as a solid.
¹H-NMR (CDCl₃) δ; 1.48 (9H, s), 2.56 - 3.05 (3H, m), 3.43 (1H, dd, J = 9.3, 5.4 Hz), 3. 64 - 3.79 (1H, m), 3. 85 - 4.36 (4H, m), 6.52 - 6.65 (2H, m), 7.17 (1H, t, J = 8.2 Hz), 7.41 (1H, br s), 7.74 - 7.82 (1H, m).

### (2) 2-(3-hydroxyphenyl)hexahydroimidazo[1,5-a]pyrazin-3(2H)-one hydrochloride

To tert-butyl 2-(3-hydroxyphenyl)-3-oxohexahydroimidazo[1,5-a]pyrazine-7(1H)-carboxylate (200 mg, 0.60 mmol) was added 4N hydrogen chloride-ethyl acetate solution (10 ml), and the mixture was stirred at room temperature for 1 hr, and the precipitated crystals were washed with ethyl acetate to give the object product (158 mg, 98%) as a solid.
¹H-NMR (DMSO-d₆) δ; 2.80 - 2.99 (2H, m), 3.15 - 3.43 (3H, m), 3.53 (1H, dd, J = 9.7, 4.4 Hz), 3.79 - 3.98 (2H, m), 4.01 - 4.15 (1H, m), 6.37 - 6.51 (1H, m), 6.82 - 6.96 (1H, m), 7.02 - 7.18 (2H, m), 9.44 (1H, s), 9.52 (2H, br s).

The compounds synthesized in Examples are shown in Table 1 - Table 2.

**Table 1**

| Ex. | R¹ | salt | Ex. | R¹ | salt |
|---|---|---|---|---|---|
| 1 | 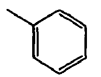 | HCl | 15 | 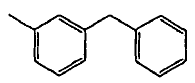 | HCl |
| 2 | 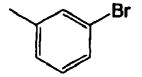 | HCl | 16 | 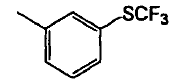 | HCl |
| 3 | 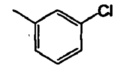 | HCl | 17 | 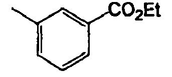 | HCl |
| 4 | 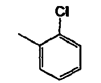 | HCl | 18 | 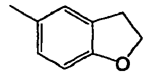 | TFA |
| 5 | 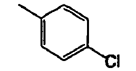 | HCl | 19 | 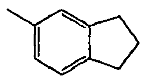 | TFA |
| 6 | 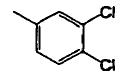 | HCl | 20 | 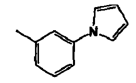 | TFA |
| 7 | 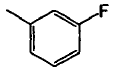 | HCl | 21 | 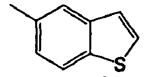 | TFA |
| 8 | 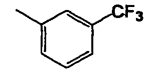 | HCl | 22 | 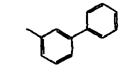 | TFA |
| 9 | 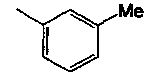 | HCl | 23 | 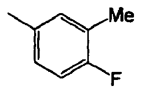 | HCl |
| 10 | 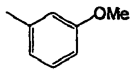 | HCl | 24 | 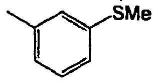 | HCl |
| 11 | 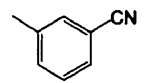 | HCl | 25 | 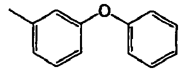 | HCl |
| 12 | 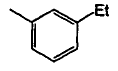 | HCl | 26 | 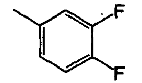 | HCl |
| 13 | 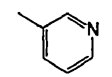 | 2HCl | 27 | 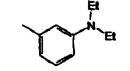 | HCl |
| 14 | 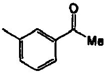 | HCl | 28 | 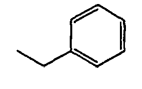 | HCl |

**Table 2**

| | | | | | |
|---|---|---|---|---|---|
| 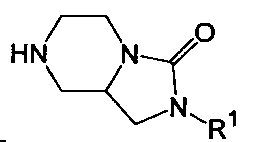 | | | | | |

| Ex. | R¹ | salt | Ex. | R¹ | salt |
|---|---|---|---|---|---|
| 29 | 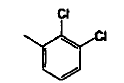 | HCl | 39 | 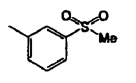 | HCl |
| 30 | 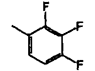 | HCl | 40 | 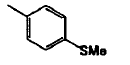 | HCl |
| 31 | 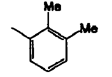 | HCl | 41 | 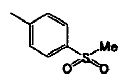 | HCl |
| 32 | 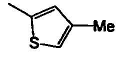 | HCl | 42 | 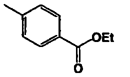 | HCl |
| 33 | 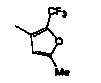 | HCl | 43 | 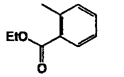 | HBr |
| 34 | 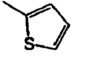 | TFA | 44 | 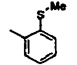 | HBr |
| 35 | 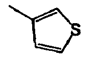 | HCl | 45 | 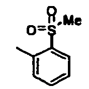 | HBr |
| 36 | 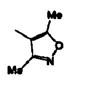 | HCl | 46 | 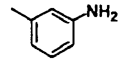 | 2HCl |
| 37 | 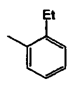 | HCl | 47 | 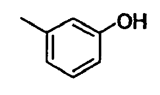 | HCl |
| 38 | 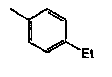 | HCl | | | |

### Formulation Example 1

| | |
|---|---|
| (1) compound of Example 1 | 10 mg |
| (2) Lactose | 60 mg |
| (3) Cornstarch | 35 mg |
| (4) hydroxypropylmethylcellulose | 3 mg |
| (5) Magnesium stearate | 2 mg |

A mixture of 10 mg of the compound obtained in Example 1, 60 mg of lactose and 35 mg of corn starch is granulated using 0.03 mL of an aqueous solution of 10 wt% hydroxypropylmethylcellulose (3 mg as hydroxypropylmethylcellulose), and then dried at 40°C and sieved. The obtained granules are mixed with 2 mg of magnesium stearate and compressed. The obtained uncoated tablets are sugar-coated with an aqueous suspension of sucrose, titanium dioxide, talc and gum arabic. The thus-coated tablets are glazed with beeswax to give finally-coated tablets.

### Formulation Example 2

| | |
|---|---|
| (1) compound of Example 1 | 10 mg |
| (2) Lactose | 70 mg |
| (3) Cornstarch | 50 mg |
| (4) Soluble starch | 7 mg |
| (5) Magnesium stearate | 3 mg |

The compound (10 mg) obtained in Example 1 and 3 mg of magnesium stearate are granulated with 0.07 mL of an aqueous solution of soluble starch (7 mg as soluble starch), dried, and mixed with 70 mg of lactose and 50 mg of corn starch. The mixture is compressed to give tablets.

### Reference Formulation Example 1

| | |
|---|---|
| (1) Rofecoxib | 5.0 mg |
| (2) Sodium chloride | 20.0 mg |
| (3) Distilled water amount to make total volume | 2.0 mL |

Rofecoxib (5.0 mg) and 20.0 mg of Sodium chloride are dissolved in distilled water, and water is added to make the total volume 2.0 mL. The solution is filtered, and filled into 2 mL of ampoule under sterile condition. The ampoule is sterilized, and then sealed to give a solution for injection.

### Reference Formulation Example 2

| | |
|---|---|
| (1) Rofecoxib | 50 mg |
| (2) Lactose | 34 mg |
| (3) Cornstarch | 10.6 mg |
| (4) Cornstarch (paste) | 5 mg |
| (5) Magnesium stearate | 0.4 mg |
| (6) Calcium carboxymethylcellulose | 20 mg |
| total | 120 mg |

The above-mentioned (1) to (6) are mixed according to a conventional method and the mixture is tableted by a tableting machine to give a tablet.

### Formulation Example 3

The formulation prepared in Formulation Example 1 or 2, and the formulation prepared in Reference Formulation Example 1 or 2 are combined.

### Experimental Example 1

The serotonin 5-HT_{2c} receptor agonist activity of the Example compounds was evaluated based on the changes in the intracellular calcium concentration by the following method. After transcription, 5-HT_{2c} undergoes RNA editing of the second intracellular loop, which results in the change of three amino acids and 14 receptor isoforms. 5-HT_{2c} stably expressing CHO cell that expresses isoform type VSV stably was purchased from Euroscreen S.A., and cultured in an UltraCHO (BioWhittaker) medium containing 1% dialyzed bovine serum and 400 µg/mL G418. The cells were plated in a 384-well black clear bottom plate (PE Biosystems) at 5000 cells/well, cultured for 24 hr in a CO₂ incubator, and changes in the intracellular calcium concentration mediated by the 5-HT_{2c} receptor were evaluated using Calcium Kit-Fluo 3 (Dojindo Laboratories). A calcium kit buffer containing 2.5 mM probenecid, 0.04% Pluronic F-127 and 2.5 µg Fluo-3 AM (calcium indicator fluorescent dye) was prepared and used as a Fluo-3 loading solution (contained in Dojindo Laboratories Calcium Kit). The loading solution was incubated at 37°C, the medium in the wells of the cell culture plate was removed, and the loading solution was added to each well by 40 µL. The cells were reacted at 37°C for 1 hr to allow uptake of Fluo-3 AM into the cells and washed. The Example compound was diluted with a calcium kit buffer, and dispensed to each well of the 384-well plate (REMP) by 40 µL to give a Example compound plate. The cell culture plate and test compound plate were set on a Fluometric Imaging Plate Reader (FLIPR, Molecular Devices), and changes in the intracellular calcium concentration were measured. An increase in the fluorescence intensity of Fluo-3 matches with an increase in the intracellular calcium concentration mediated by a receptor. The changes in the intracellular fluorescence intensity were measured every second with a CCD camera of FLIPR and, after measurement for 5 seconds before addition of the compound, a diluted solution of the Example compound was added by 20 µL to each well of the cell culture plate using an automatic dispenser in FLIPR.
The agonist activity was evaluated based on the difference in the fluorescence level obtained by subtracting the fluorescence intensity before addition of the compound from the maximum fluorescence intensity after the addition thereof. The activity of the test compound is shown by the ratio relative to the maximum response by 5-HT (Table 3).

**Table 3**

| Ex. No. | ratio to maximum response by 5-HT (1 µL) | Ex. No. | ratio to maximum response by 5-HT (1 µL) |
|---|---|---|---|
| 1 | 112.9 | 9 | 91.4 |
| 2 | 109.2 | 10 | 90.0 |
| 3 | 97.7 | 12 | 95.7 |
| 4 | 73.9 | 34 | 100.6 |
| 7 | 99.5 | 35 | 99.5 |
| 8 | 90.7 | 36 | 96.5 |

From Table 3, it was found that the compound of the present invention has a superior serotonin 5-HT_{2c} receptor agonist activity.

### Industrial Applicability

Since compound (I) of the present invention or a prodrug thereof has a superior serotonin 5-HT_{2c} receptor activating action, it is useful as safe drugs for the prophylaxis or treatment of all serotonin 5-HT_{2C}-related diseases, for example, stress urinary incontinence and the like.

This application is based on a patent application No. 2006-190924 filed in Japan, the contents of which are incorporated in full herein by this reference.

## Claims

1. A compound represented by the formula: 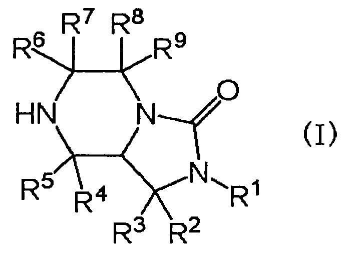 wherein
R¹ is a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s) or a heterocyclic group optionally having substituent(s);
R², R³, R⁴, R⁵, R⁶, R⁷, R⁸ and R⁹ are the same or different and each is a hydrogen atom, a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s), a hydroxyl group optionally having a substituent, mercapto optionally having a substituent, amino optionally having substituent(s) or a heterocyclic group optionally having substituent(s), or each of R⁴ and R⁵, R⁶ and
R⁷, and R⁸ and R⁹ optionally form, together with the adjacent carbon atom, carbonyl, thiocarbonyl or imino optionally having a substituent,
or a salt thereof.

2. The compound of claim 1, wherein R¹ is aryl optionally having substituent(s), aralkyl optionally having substituent(s) or a heterocyclic group optionally having substituent(s).

3. The compound of claim 1, wherein R¹ is
(1) C₆₋₁₄ aryl optionally having 1 to 3 substituents selected from
(a) a halogen atom,
(b) cyano,
(c) a hydroxyl group,
(d) amino,
(e) C₁₋₆ alkyl optionally having 1 to 3 halogen atoms,
(f) C₆₋₁₄ aryl,
(g) C₇₋₁₆ aralkyl,
(h) di-C₁₋₆ alkylamino,
(i) C₁₋₆ alkoxy,
(j) C₆₋₁₄ aryloxy,
(k) C₁₋₆ alkylthio optionally having 1 to 3 halogen atoms,
(l) C₁₋₆ alkyl-carbonyl,
(m) C₁₋₆ alkoxy-carbonyl,
(n) C₁₋₆ alkylsulfonyl, and
(o) a 5- to 8-membered aromatic heterocyclic group containing, besides carbon atom, 1 to 4 hetero atoms
selected from nitrogen atom, sulfur atom and oxygen atom, and optionally condensed with C₃₋₈ cycloalkane,
(2) C₇₋₁₆ aralkyl,
(3) a 5- to 8-membered non-aromatic heterocyclic group containing, besides carbon atom, 1 to 4 hetero atoms selected from nitrogen atom, sulfur atom and oxygen atom, and optionally condensed with C₆₋₁₄ arene, or
(4) a 5- to 8-membered aromatic heterocyclic group containing, besides carbon atom, 1 to 4 hetero atoms selected from nitrogen atom, sulfur atom and oxygen atom, and optionally having 1 to 3 C₁₋₆ alkyl optionally having 1 to 3 halogen atoms, and optionally condensed with C₆₋₁₄ arene.

4. The compound of claim 1, wherein R², R³, R⁴, R⁵, R⁶, R⁷, R⁸ and R⁹ are each a hydrogen atom.

5. 2-(2-Thienyl)hexahydroimidazo[1,5-a]pyrazin-3(2H)-one, 2-(3-thienyl)hexahydroimidazo[1,5-a]pyrazin-3(2H)-one, 2-[5-methyl-2-(trifluoromethyl)-3-furyl]hexahydroimidazo[1,5-a]pyrazin-3(2H)-one, or
2-(3-ethylphenyl)hexahydroimidazo[1,5-a]pyrazin-3(2H)-one, or a salt thereof.

6. A prodrug of the compound of claim 1.

7. A pharmaceutical agent comprising a compound represented by the formula: 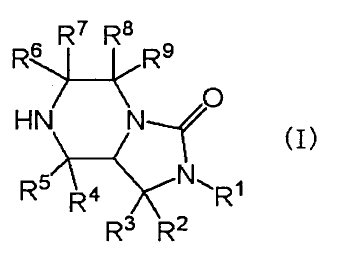 wherein
R¹ is a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s) or a heterocyclic group optionally having substituent(s);
R², R³, R⁴, R⁵, R⁶, R⁷, R⁸ and R⁹ are the same or different and each is a hydrogen atom, a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s), a hydroxyl group optionally having a substituent, mercapto optionally having a substituent, amino optionally having substituent(s) or a heterocyclic group optionally having substituent(s), or each of R⁴ and R⁵, R⁶ and
R⁷, and R⁸ and R⁹ optionally form, together with the adjacent carbon atom, carbonyl, thiocarbonyl or imino optionally having a substituent,
or a salt thereof, or a prodrug thereof.

8. The pharmaceutical agent of claim 7, which is a serotonin 5-HT_{2c} receptor activator.

9. The pharmaceutical agent of claim 7, which is an agent for the prophylaxis or treatment of stress urinary incontinence, obesity and/or pelvic organ prolapse.

10. A method for the prophylaxis or treatment of stress urinary incontinence, obesity and/or pelvic organ prolapse in a mammal, comprising administering an effective amount of a compound represented by the formula: 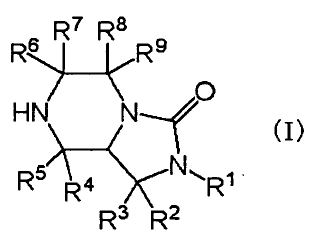 wherein
R¹ is a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s) or a heterocyclic group optionally having substituent(s);
R², R³, R⁴, R⁵, R⁶, R⁷, R⁸ and R⁹ are the same or different and each is a hydrogen atom, a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s), a hydroxyl group optionally having a substituent, mercapto optionally having a substituent, amino optionally having substituent(s) or a heterocyclic group optionally having substituent(s), or each of R⁴ and R⁵, R⁶ and
R⁷, and R⁸ and R⁹ optionally form, together with the adjacent carbon atom, carbonyl, thiocarbonyl or imino optionally having a substituent,
or a salt thereof, or a prodrug thereof, to the mammal.

11. Use of a compound represented by the formula: 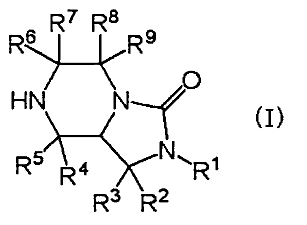 wherein
R¹ is a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s) or a heterocyclic group optionally having substituent(s);
R², R³, R⁴, R⁵, R⁶, R⁷, R⁸ and R⁹ are the same or different and each is a hydrogen atom, a hydrocarbon group optionally having substituent(s), alkylcarbonyl optionally having substituent(s), alkenylcarbonyl optionally having substituent(s), alkynylcarbonyl optionally having substituent(s), aralkylcarbonyl optionally having substituent(s), arylcarbonyl optionally having substituent(s), cycloalkylcarbonyl optionally having substituent(s), a hydroxyl group optionally having a substituent, mercapto optionally having a substituent, amino optionally having substituent(s) or a heterocyclic group optionally having substituent(s), or each of R⁴ and R⁵, R⁶ and
R⁷, and R⁸ and R⁹ optionally form, together with the adjacent carbon atom, carbonyl, thiocarbonyl or imino optionally having a substituent,
or a salt thereof, or a prodrug thereof, for the prophylaxis or treatment of stress urinary incontinence, obesity and/or pelvic organ prolapse.
